# EUROPEAN PATENT SPECIFICATION

(11) **EP 4 558 507 B1**
(45) Date of publication and mention of the grant of the patent: **29.04.2026**
(21) Application number: 23748009.0
(22) Date of filing: 18.07.2023
(51) Int. Cl.: C07D 513/22, A61P 35/00, A61K 31/504

(54) **MACROCYCLE COMPOUNDS FOR THE TREATMENT OF CANCER**
MAKROCYCLISCHE VERBINDUNGEN ZUR BEHANDLUNG VON KREBS
COMPOSÉS MACROCYCLES POUR LE TRAITEMENT DU CANCER

(30) Priority: 20.07.2022 WO PCT/CN2022/106792; 01.11.2022 WO PCT/CN2022/128979; 05.01.2023 WO PCT/CN2023/070766; 11.04.2023 WO PCT/CN2023/087631
(43) Date of publication of application: 28.05.2025
(73) Proprietor: F. Hoffmann-La Roche AG, 4070 Basel (CH)
(72) Inventor: CHEN, Jianguo, Shanghai 201203 (CN); GUO, Lei, Shanghai 201203 (CN); LIU, Haixia, Shanghai 201203 (CN); SHEN, Hong, Shanghai 201203 (CN); ZHANG, Weixing, Shanghai 201203 (CN); ZHAO, Dan, Shanghai 201203 (CN); ZHU, Wei, Shanghai 201203 (CN)
(74) Representative: Jochnowitz, Evan
(86) International application number: PCT/EP2023/069851
(87) International publication number: WO 2024/017859

(56) References cited:
- WO-A1-2021/091956
- WO-A1-2022/060836

## Description

The present invention relates to organic compounds useful for therapy and/or prophylaxis in a mammal, and in particular to inhibition of KRAS mutant useful for treating cancers.

### FIELD OF THE INVENTION

RAS is one of the most well-known proto-oncogenes. Approximately 30% of human cancers contain mutations in three most notable members, KRAS, HRAS, and NRAS, making them the most prevalent oncogenic drivers. KRAS mutations are generally associated with poor prognosis especially in colorectal cancer, pancreatic cancer, lung cancers. As the most frequently mutated RAS isoform, KRAS has been intensively studied in the past years. Among the most commonly occurring KRAS alleles (including G12D, G12V, G12C, G13D, G12R, G12A, G12S, Q61H, etc), G12C, G12D, G12V represent more than half of all K-RAS-driven cancers across colorectal cancer (CRC), pancreatic ductal adenocarcinoma (PDAC), lung adenocarcinoma (LUAD). Of note, KRAS wild-type amplifications are also found in around 7% of all KRAS-altered cancers (ovarian, esophagogastric, uterine), ranking among the top alterations.

All RAS proteins belong to a protein family of small GTPases that hydrolyze GTP to GDP. KRAS is structurally divided into an effector binding lobe followed by the allosteric lobe and a carboxy-terminal region that is responsible for membrane anchoring. The effector lobe comprises the P-loop, switch I, and switch II regions. The switch I/II loops play a critical role in KRAS downstream signaling through mediating protein-protein interactions with effector proteins that include RAF in the mitogen-activated protein kinase (MAPK) pathway or PI3K in the phosphatidylinositol 3-kinase (PI3K)/protein kinase B (AKT) pathway.

KRAS protein switches between an inactive to an active form via binding to GTP and GDP, respectively. Under physiological conditions, the transition between these two states is regulated by guanine nucleotide exchange factors (GEFs), such as Son Of Sevenless Homolog 1 (SOS1), or GTPase-activating proteins (GAPs) that involve catalyzing the exchange of GDP for GTP, potentiating intrinsic GTPase activity or accelerating RAS-mediated GTP hydrolysis. In response to extracellular stimuli, the inactive RAS-GDP is converted to active RAS-GTP which directly binds to RAF RAS binding domains (RAF^{RBD}), recruiting RAF kinase family from cytoplasm to membranes, where they dimerize and become active. The activated RAF subsequently carries out a chain of phosphorylation reactions to its downstream Mitogen-activated protein kinase (MEK) and extracellular signal-regulated kinase (ERK), and propagates the growth signal. Of the RAF family of protein kinases (three known isoforms ARAF, BRAF, CRAF/RAF1), BRAF is most frequently mutated and remains the most potent activator of MEK. Despite that individual RAS and RAF family members revealed distinct binding preferences, all RAFs possess the conserved RBD for forward transmission of MAPK singnaling, frequently used for characterize KRAS inhibition (e.g. KRAS-BRAF^{RBD} herein). For KRAS, mutations at positions 12, 13, 61, and 146 lead to a shift toward the active KRAS form through impairing nucleotide hydrolysis or activating nucleotide exchange, leading to hyper-activation of the MAPK pathway that results in tumorigenesis.

Despite its well-recognized importance in cancer malignancy, continuous efforts in the past failed to develop approved therapies for KRAS mutant cancer until recently, the first selective drug AMG510 has fast approval as second line treatment in KRAS G12C driven non-small cell lung cancer (NSCLC). Nevertheless, the clinical acquired resistance to KRAS G12C inhibitors emerge rigorously with disease progresses after around 6 month of treatment. All of the mutations converge to reactivate RAS-MAPK signaling, with secondary RAS mutants at oncogenic hotspots (e.g. G12/G13/Q61) and within the switch II pocket (e.g. H95, R68, and Y96) have been observed; moreover, over 85% of all KRAS-mutated or wild-type amplified driven cancers still lack novel agents. Altogether, both the myriad of escape mechanism and various oncogenic alleles, highlight the urgent medical need for additional KRAS therapies. As such, we invented oral compounds that target and inhibit KRAS alleles for the treatment of KRAS mutant driven cancers.

First generation KRAS G12C inhibitors like Sotorosib, Adagrasib targeting on 'GDP bound off' form (RASOFF) of KRAS G12C mutation have demonstrated promising efficacy. While this treatment has benefited many patients with activating KRAS mutations, almost all who initially benefited will eventually acquire resistance via various mechanism. Increasing cases of *KRAS G12C* second mutations have been identified either from patients' samples such as Y96D, R68S, H95D, H95Q, H95R, V8L (Tanaka et al., Cancer Discovery (2021), Awad et al., NEJM (2021), Ho et al., EJC (2021), Zhao et al., Nature (2021), Tsai et al., JCI (2022)), or discovered from saturation mutagenesis (Siyu et al, PNAS (2022)) and ENU mutagenesis (Takamasa et al, J Thorac Oncol (2021)) that demonstrated resistance to KRAS(OFF) G12C inhibitors. Therefore, there are unmet needs to prevent the acquisition of one or more mutations in RAS that confer resistance to the RAS(OFF) inhibitor.

### SUMMARY OF THE INVENTION

The present invention relates to novel compounds of formula (I), 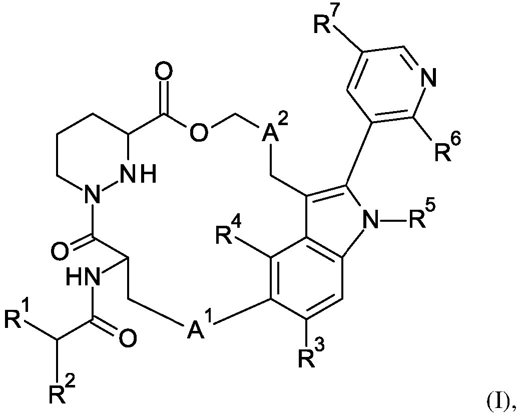 wherein
R¹ is 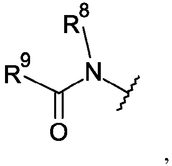 3-oxo-5,6,8,8a-tetrahydro-1H-imidazo[5,1-c][1,4]oxazinyl or (C₁₋₆alkyl)oxoimidazolidinyl;
   wherein
   R⁸ is C₁₋₆alkyl;
   R⁹ is ((C₁₋₆alkyl)zamino)azetidinyl, C₁₋₆alkylpiperazinyl, haloazetidinyl, haloC₁₋₆alkylamino, haloC₁₋₆alkylaminoazetidinyl, haloC₁₋₆alkylpiperazinyl, hydroxy(C₁₋₆alkyl)piperidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

The invention also relates to their manufacture, medicaments based on a compound in accordance with the invention and their production as well as the use of compounds of formula (I) or (Ia) thereof as inhibitor of KRAS.

The compounds of formula (I) or (Ia) show good KRAS inhibition for G12C, G12D and G12V. In another embodiment, the compounds of this invention showed superior cancer cell inhibition and human hepatocyte stability. In addition, the compounds of formula (I) or (Ia) also show good or improved cytotoxicity and solubility profiles. Furthermore, the compound of current invention had excellent pharmacokinetic properties comparing with the reference compounds.

### BRIEF DESCRIPTION OF THE FIGURE

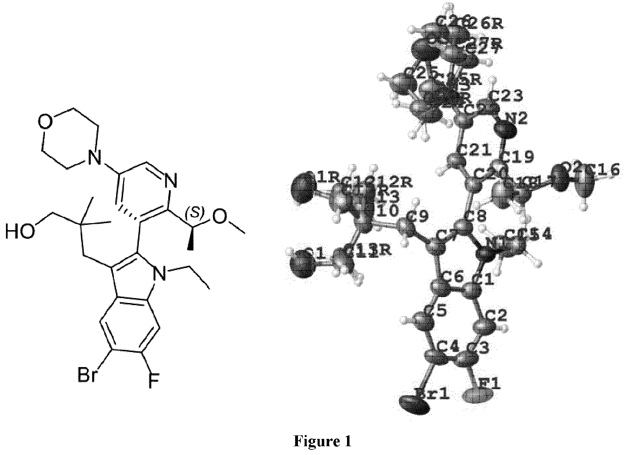

Figure 1. X-ray crystallographic analysis of compound G5.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *tert-*butyl and the like. Particular "C₁₋₆alkyl" groups are methyl, ethyl and *n*-propyl.

The term "C₁₋₆alkoxy" denotes C₁₋₆alkyl-O-.

The term "C₁₋₆alkylene" denotes a linear or branched saturated divalent hydrocarbon group of 1 to 6 carbon atoms or a divalent branched saturated divalent hydrocarbon group of 3 to 6 carbon atoms. Examples of C₁₋₆alkylene groups include methylene, ethylene, propylene, 2-methylpropylene, butylene, 2-ethylbutylene, pentylene, hexylene.

The term "halogen" and "halo" are used interchangeably herein and denote fluoro, chloro, bromo, or iodo.

The term "haloC₁₋₆alkyl" denotes a C₁₋₆alkyl group wherein at least one of the hydrogen atoms of the C₁₋₆alkyl group has been replaced by same or different halogen atoms, particularly fluoro atoms. Examples of haloalkyl include monofluoro-, difluoro- or trifluoro-methyl, -ethyl or -propyl, for example 3,3,3-trifluoropropyl, 2-fluoroethyl, 2,2,2-trifluoroethyl, fluoromethyl, or trifluoromethyl.

The term "C₃₋₇cycloalkyl" denotes a monovalent saturated monocyclic or bicyclic hydrocarbon group of 3 to 7 ring carbon atoms. Bicyclic means consisting of two saturated carbocycles having one or more carbon atoms in common. Examples for monocyclic cycloalkyl are cyclopropyl, cyclobutyl, cyclopentyl, cyclohexyl or cycloheptyl. Examples for bicyclic cycloalkyl are bicyclo[1.1.0]butyl, bicyclo[2.2.1]heptanyl, bicyclo[1.1.1]pentanyl, or bicyclo[2.2.2]octanyl.

The term "thiazolylene" denotes a divalent thiazolyl group.

The term "oxo" denotes a divalent oxygen atom =O.

The term "oxoimidazolidinyl" denotes

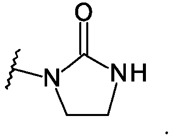

The term "haloazetidinyl" denotes an azetidinyl group wherein at least one of the hydrogen atoms of the azetidinyl group has been replaced by same or different halogen atoms, particularly fluoro atoms. Examples of haloazetidinyl include fluoroazetidinyl and difluoroazetidinyl.

The term "dimethylmethylene" denotes

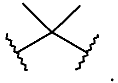

The term "protecting group" denotes the group which selectively blocks a reactive site in a multifunctional compound such that a chemical reaction can be carried out selectively at another unprotected reactive site in the meaning conventionally associated with it in synthetic chemistry. Protecting groups can be removed at the appropriate point. Exemplary protecting groups are amino-protecting groups, carboxy-protecting groups or hydroxy-protecting groups.

The skilled of the art would understand that the following structures of compounds of formula (Ia) and (Ia') are equal especially for the chiral centers:

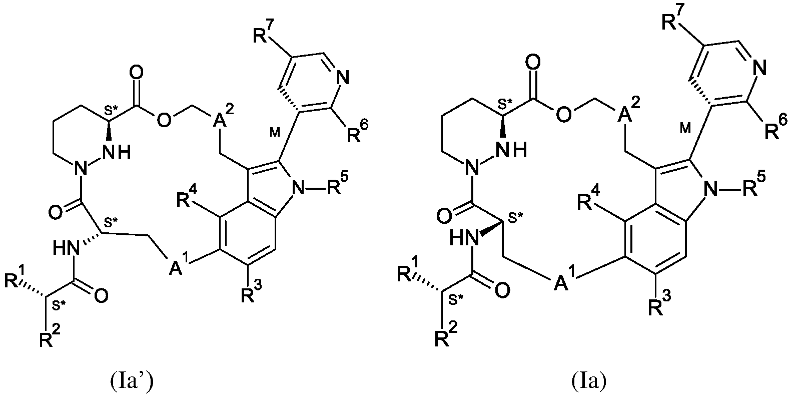

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and *tert*iary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

The terms "pharmaceutically acceptable excipient", "pharmaceutically acceptable carrier" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents or lubricants used in formulating pharmaceutical products.

### INHIBITOR OF KRAS

The present invention relates to (i) a compound of formula (I), 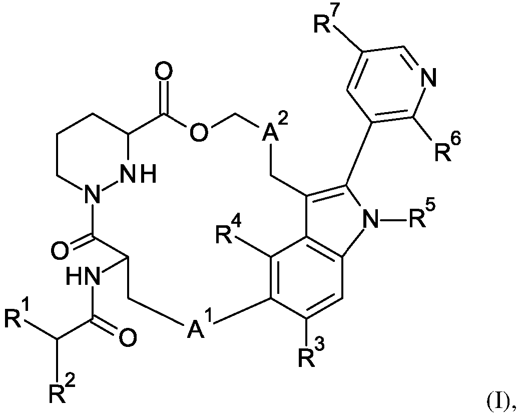 wherein
R¹ is 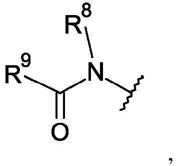 3-oxo-5,6,8,8a-tetrahydro-1H-imidazo[5,1-c][1,4]oxazinyl or (C₁₋₆alkyl)oxoimidazolidinyl;
   wherein
   R⁸ is C₁₋₆alkyl;
   R⁹ is ((C₁₋₆alkyl)zamino)azetidinyl, C₁₋₆alkylpiperazinyl, haloazetidinyl, haloC₁₋₆alkylamino, haloC₁₋₆alkylaminoazetidinyl, haloC₁₋₆alkylpiperazinyl, hydroxy(C₁₋₆alkyl)piperidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

Another embodiment of present invention is (ii) a compound of formula (Ia), 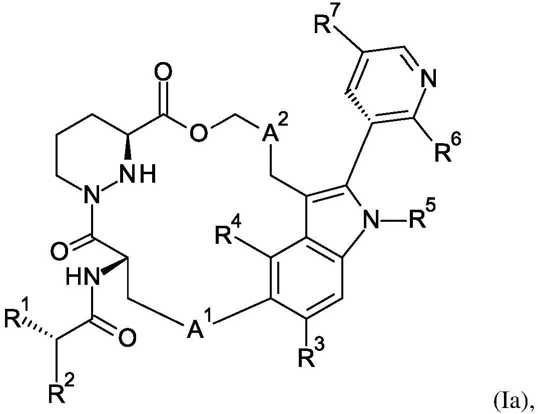 wherein
R¹ is 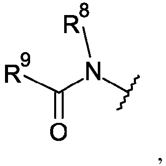 3-oxo-5,6,8,8a-tetrahydro-1H-imidazo[5,1-c][1,4]oxazinyl or (C₁₋₆alkyl)oxoimidazolidinyl;
   wherein
   R⁸ is C₁₋₆alkyl;
   R⁹ is ((C₁₋₆alkyl)zamino)azetidinyl, C₁₋₆alkylpiperazinyl, haloazetidinyl, haloC₁₋₆alkylamino, haloC₁₋₆alkylaminoazetidinyl, haloC₁₋₆alkylpiperazinyl, hydroxy(C₁₋₆alkyl)piperidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

A further embodiment of present invention is (iii) a compound of formula (I) or (Ia) according to (i) or (ii), or a pharmaceutically acceptable salt thereof, wherein R¹ is 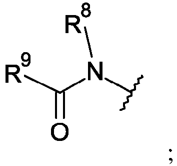 wherein R⁸ is C₁₋₆alkyl; R⁹ is C₁₋₆alkylpiperazinyl, haloC₁₋₆alkylpiperazinyl or morpholinyl.

A further embodiment of present invention is (iv) a compound of formula (I) or (Ia), according to any one of (i) to (iii), or a pharmaceutically acceptable salt thereof, wherein R¹ is 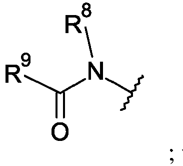 wherein R⁸ is methyl; R⁹ is 4-methylpiperazin-1-yl, 4-(2,2,2-trifluoroethyl)piperazin-1-yl or morpholinyl.

A further embodiment of present invention is (v) a compound of formula (I) or (Ia) according to any one of (i) to (iv), wherein R¹ is methyl-(4-methylpiperazine-1-carbonyl)amino, methyl-[4-(2,2,2-trifluoroethyl)piperazine-1-carbonyl]amino or methyl(morpholine-4-carbonyl)amino.

A further embodiment of present invention is (vi) a compound of formula (I) or (Ia), or a pharmaceutically acceptable salt thereof, according to any one of (i) to (v), wherein R² is isopropyl.

A further embodiment of present invention is (vii) a compound of formula (I) or (Ia), or a pharmaceutically acceptable salt thereof, according to any one of (i) to (vi), wherein R³ is H or fluoro.

A further embodiment of present invention is (viii) a compound of formula (I) or (Ia), or a pharmaceutically acceptable salt thereof, according to any one of (i) to (vii), wherein R³ is fluoro.

A further embodiment of present invention is (ix) a compound of formula (I) or (Ia), or a pharmaceutically acceptable salt thereof, according to any one of (i) to (viii), wherein R⁴ is H or fluoro.

A further embodiment of present invention is (x) a compound of formula (I) or (Ia), or a pharmaceutically acceptable salt thereof, according to any one of (i) to (ix), wherein R⁴ is H.

A further embodiment of present invention is (xi) a compound of formula (I) or (Ia), or a pharmaceutically acceptable salt thereof, according to any one of (i) to (x), wherein R⁵ is haloC₁₋₆alkyl.

A further embodiment of present invention is (xii) a compound of formula (I) or (Ia), or a pharmaceutically acceptable salt thereof, according to any one of (i) to (xi), wherein R⁵ is 2,2,2-trifluoroethyl.

A further embodiment of present invention is (xiii) a compound of formula (I) or (Ia), or a pharmaceutically acceptable salt thereof, according to any one of (i) to (xii), wherein R⁶ is 1-methoxyethyl.

A further embodiment of present invention is (xiv) a compound of formula (I) or (Ia), or a pharmaceutically acceptable salt thereof, according to any one of (i) to (xiii), wherein R⁷ is (haloC₁₋₆alkyl)piperazinyl.

A further embodiment of present invention is (xv) a compound of formula (I) or (Ia), or a pharmaceutically acceptable salt thereof, according to any one of (i) to (xiv), wherein R⁷ is 4-(2,2,2-trifluoroethyl)piperazin-1-yl.

A further embodiment of present invention is (xvi) a compound of formula (I) or (Ia), or a pharmaceutically acceptable salt thereof, according to any one of (i) to (xv), wherein A¹ is 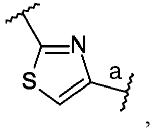 wherein bond "a" connects to indole ring.

A further embodiment of present invention is (xvii) a compound of formula (I) or (Ia), or a pharmaceutically acceptable salt thereof, according to any one of (i) to (xvi), wherein A² is dimethylmethylene.

Another embodiment of present invention is (xviii) a compound of formula (I) or (Ia), according to (i) or (ii), wherein
R¹ is 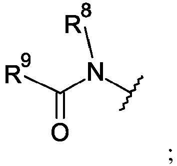 wherein R⁸ is C₁₋₆alkyl; R⁹ is C₁₋₆alkylpiperazinyl, haloC₁₋₆alkylpiperazinyl or morpholinyl;
R² is C₁₋₆alkyl;
R³ is halogen;
R⁴ is H;
R⁵ is haloC₁₋₆alkyl;
R⁶ is C₁₋₆alkoxyC₁₋₆alkyl;
R⁷ is (haloC₁₋₆alkyl)piperazinyl;
A¹ is 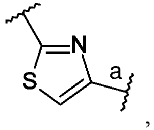 wherein bond "a" connects to indole ring;
A² is C₁₋₆alkylene;
or a pharmaceutically acceptable salt thereof.

Another embodiment of present invention is (xix) a compound of formula (I) or (Ia), according to (xviii), wherein
R¹ is methyl-(4-methylpiperazine-1-carbonyl)amino, methyl-[4-(2,2,2-trifluoroethyl)piperazine-1-carbonyl]amino or methyl(morpholine-4-carbonyl)amino;
R² is isopropyl;
R³ is fluoro;
R⁴ is H;
R⁵ is 2,2,2-trifluoroethyl;
R⁶ is (1S)-1-methoxyethyl;
R⁷ is 4-(2,2,2-trifluoroethyl)piperazin-1-yl;
A¹ is 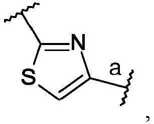 wherein bond "a" connects to indole ring;
A² is dimethylmethylene;
or a pharmaceutically acceptable salt thereof.

The present invention relates to (i') a compound of formula (I), 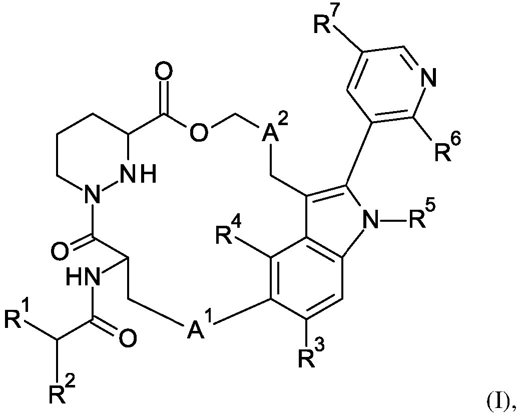 wherein
R¹ is 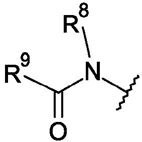 or (C₁₋₆alkyl)oxoimidazolidinyl;
   wherein
   R⁸ is C₁₋₆alkyl;
   R⁹ is ((C₁₋₆alkyl)zamino)azetidinyl, haloazetidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

Another embodiment of present invention is (ii') a compound of formula (Ia), 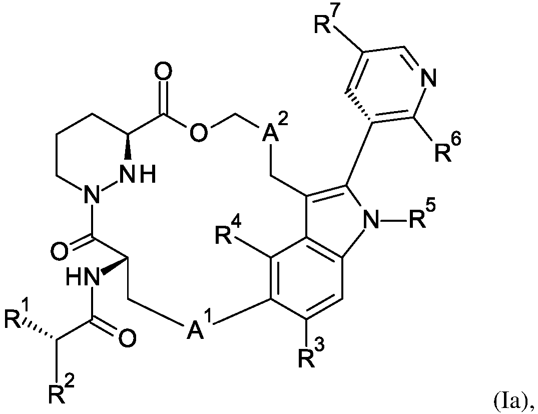 wherein
R¹ is 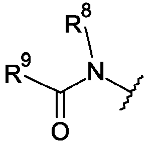 or (C₁₋₆alkyl)oxoimidazolidinyl;
   wherein
   R⁸ is C₁₋₆alkyl;
   R⁹ is ((C₁₋₆alkyl)zamino)azetidinyl, haloazetidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

A further embodiment of present invention is (iii') a compound of formula (I) or (Ia) according to (i') or (ii'), or a pharmaceutically acceptable salt thereof, wherein R¹ is 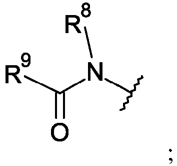 wherein R⁸ is C₁₋₆alkyl; R⁹ is haloazetidinyl or morpholinyl.

A further embodiment of present invention is (iv') a compound of formula (I) or (Ia), according to any one of (i') to (iii'), or a pharmaceutically acceptable salt thereof, wherein R¹ is 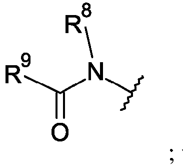 wherein R⁸ is methyl; R⁹ is 3,3-difluoroazetidin-1-yl or morpholinyl.

A further embodiment of present invention is (v') a compound of formula (I) or (Ia) according to any one of (i') to (iv'), wherein R¹ is (3,3-difluoroazetidine-1-carbonyl)-methyl-amino or methyl(morpholine-4-carbonyl)amino.

A further embodiment of present invention is (vi') a compound of formula (I) or (Ia), or a pharmaceutically acceptable salt thereof, according to any one of (i') to (v'), wherein R² is isopropyl.

A further embodiment of present invention is (vii') a compound of formula (I) or (Ia), or a pharmaceutically acceptable salt thereof, according to any one of (i') to (vi'), wherein R³ is H or fluoro.

A further embodiment of present invention is (viii') a compound of formula (I) or (Ia), or a pharmaceutically acceptable salt thereof, according to any one of (i') to (vii'), wherein R³ is fluoro.

A further embodiment of present invention is (ix') a compound of formula (I) or (Ia), or a pharmaceutically acceptable salt thereof, according to any one of (i') to (viii'), wherein R⁴ is H or fluoro.

A further embodiment of present invention is (x') a compound of formula (I) or (Ia), or a pharmaceutically acceptable salt thereof, according to any one of (i') to (ix'), wherein R⁴ is H.

A further embodiment of present invention is (xi') a compound of formula (I) or (Ia), or a pharmaceutically acceptable salt thereof, according to any one of (i') to (x'), wherein R⁵ is ethyl or 2,2,2-trifluoroethyl.

A further embodiment of present invention is (xii') a compound of formula (I) or (Ia), or a pharmaceutically acceptable salt thereof, according to any one of (i') to (xi'), wherein R⁶ is 1-methoxyethyl.

A further embodiment of present invention is (xiii') a compound of formula (I) or (Ia), or a pharmaceutically acceptable salt thereof, according to any one of (i') to (xii'), wherein R⁷ is 4-(2,2,2-trifluoroethyl)piperazin-1-yl or morpholinyl.

A further embodiment of present invention is (xiv') a compound of formula (I) or (Ia), or a pharmaceutically acceptable salt thereof, according to any one of (i') to (xiii'), wherein A¹ is 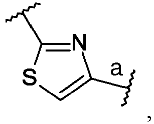 wherein bond "a" connects to indole ring.

A further embodiment of present invention is (xv') a compound of formula (I) or (Ia), or a pharmaceutically acceptable salt thereof, according to any one of (i') to (xiv'), wherein A² is dimethylmethylene.

Another embodiment of present invention is (xvi') a compound of formula (I) or (Ia), according to (i') or (ii'), wherein
R¹ is 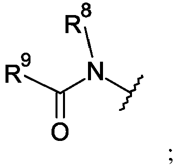 wherein R⁸ is C₁₋₆alkyl; R⁹ is haloazetidinyl or morpholinyl;
R² is C₁₋₆alkyl;
R³ is halogen;
R⁴ is H;
R⁵ is C₁₋₆alkyl or haloC₁₋₆alkyl;
R⁶ is C₁₋₆alkoxyC₁₋₆alkyl;
R⁷ is morpholinyl or (haloC₁₋₆alkyl)piperazinyl;
A¹ is 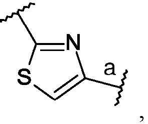 wherein bond "a" connects to indole ring;
A² is C₁₋₆alkylene;
or a pharmaceutically acceptable salt thereof.

Another embodiment of present invention is (xvii') a compound of formula (I) or (Ia), according to (xvi'), wherein
R¹ is (3,3-difluoroazetidine-1-carbonyl)-methyl-amino or methyl(morpholine-4-carbonyl)amino;
R² is isopropyl;
R³ is fluoro;
R⁴ is H;
R⁵ is ethyl or 2,2,2-trifluoroethyl;
R⁶ is (1S)-1-methoxyethyl;
R⁷ is 4-(2,2,2-trifluoroethyl)piperazin-1-yl or morpholinyl;
A¹ is 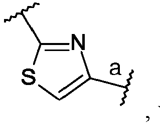 wherein bond "a" connects to indole ring;
A² is dimethylmethylene;
or a pharmaceutically acceptable salt thereof.

Another embodiment of present invention is (xx) a compound of formula (I) or (Ia) selected from the following:
3-(dimethylamino)-*N*-[(1S)-1-[[(7S,13S)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
3-(dimethylamino)-*N*-[(1*S*)-1-[[(7*S*,13*S*)-25-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
*N*-[(1*S*)-1-[[(7*S*,13*S*)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
3-(dimethylamino)-*N*-[(1*S*)-1-[[(7*S*,13*S*)-24-fluoro-(20*M*)-20-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo [17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3,3-difluoro-*N*-methyl-azetidine-1-carboxamide;
(2*S*)*-N-*[(7*S,*13*S*)*-*21-ethyl-24-fluoro-(20*M)*-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-methyl-2-oxo-imidazolidin-1-yl)butanamide;
*N*-[(1*S*)-1-[[(7*S*,13*S*)-21-ethyl-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-21-ethyl-24-fluoro-(20*M*)-20-[2-[(1*S*)-1-methoxyethyl]-5-(4-methylpiperazin-1-yl)-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-24-fluoro-20-(20*M*)-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
3-(dimethylamino)-*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1 .0^{22,26}]octacosa-1(25),2,5(28),19,22(26),23-hexaen-7-yl]carbamoyl]-2-methyl-propyl]-*N*-methyl-azetidine-1-carboxamide;
(2*S*)-*N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-[methyl(2,2,2-trifluoroethylcarbamoyl)amino]butanamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-3-(trifluoromethyl)azetidine-1-carboxamide;
(2*S*)-*N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-oxo-5,6,8,8a-tetrahydro-1*H*-imidazo[5,1-c][1,4]oxazin-2-yl)butanamide;
(3*R*)-*N*-[(1*S*)-1-[[(7*S*,13*S*)-24-fluoro-(20*M*)-20-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3-hydroxy-N,3-dimethyl-piperidine-1-carboxamide;
*N*-[(1*S*)-1-[[(7*S*,13*S*)-25-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*,4-dimethyl-piperazine-1-carboxamide; and
*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-4-(2,2,2-trifluoroethyl)piperazine-1-carboxamide;
or a pharmaceutically acceptable salt thereof.

Another embodiment of present invention is related to (xxi) a process for the preparation of a compound according to any one of (i) to (xix) or (i') to (xvii') comprising the following step:
a) coupling reaction between compound of formula (II), 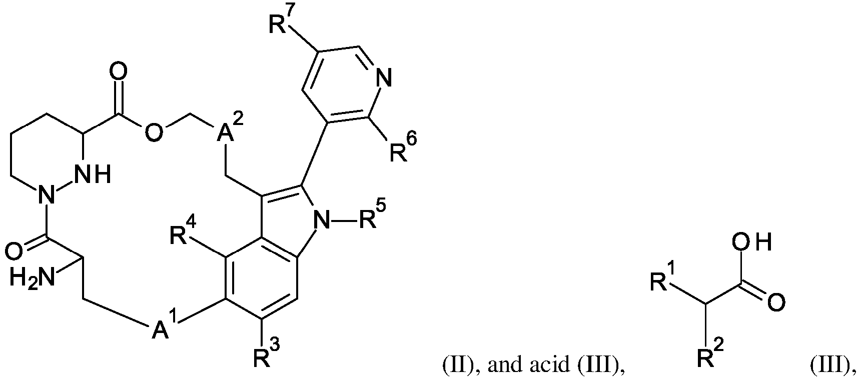 in the presence of a coupling reagent and a base to form the compound of formula (I);
wherein R¹, R², R³, R⁴, R⁵, R⁶, R⁷, A¹ and A² are defined as in any one of (i) to (xix); the coupling reagent is T₃P, HATU, PyBOP or EDCI/HOBt; the base is TEA, DIEPA or DMAP.

Another embodiment of present invention is (xxii) a compound or pharmaceutically acceptable salt according to any one of (i) to (xx) or (i') to (xvii') for use as therapeutically active substance.

Another embodiment of present invention is (xxiii) a pharmaceutical composition comprising a compound in accordance with any one of (i) to (xx) or (i') to (xvii') and a pharmaceutically acceptable excipient.

Another embodiment of present invention is (xxiv) the use of a compound according to any one of (i) to (xx) or (i') to (xvii') for treating a KRAS G12C protein-related disease.

Another embodiment of present invention is (xxv) the use of a compound according to any one of (i) to (xx) or (i') to (xvii') for treating a KRAS G12C, G12D and G12V protein-related disease.

Another embodiment of present invention is (xxvi) the use of a compound according to any one of (i) to (xx) or (i') to (xvii') for inhibiting RAS interaction with downstream effectors, wherein the downstream effectors are RAF and PI3K.

Another embodiment of present invention is (xxvii) the use of a compound according to any one of (i) to (xx) or (i') to (xvii') for inhibiting the propagating oncogenic MAPK and PI3K signaling.

Another embodiment of present invention is (xxviii) the use of a compound according to any one of (i) to (xx) or (i') to (xvii') for the treatment or prophylaxis of KRAS mutation driven cancers, wherein the cancer is selected from pancreatic cancer, colorectal cancer, lung cancer, esophageal cancer, gallbladder cancer, melanoma ovarian cancer and endometrial cancer.

Another embodiment of present invention is (xxix) the use of a compound according to any one of (i) to (xx) or (i') to (xvii') for the treatment or prophylaxis of KRAS mutation driven cancers, wherein the cancer is selected from pancreatic adenocarcinoma, colorectal cancer and non-small cell lung cancer.

Another embodiment of present invention is (xxx) a compound or pharmaceutically acceptable salt according to any one of (i) to (xx) or (i') to (xvii') for the treatment or prophylaxis of KRAS mutation driven cancers, wherein the cancer is selected from pancreatic adenocarcinoma, colorectal cancer and non-small cell lung cancer.

Another embodiment of present invention is (xxxi) a compound or pharmaceutically acceptable salt according to any one of (i) to (xx) or (i') to (xvii') for the treatment or prophylaxis of KRAS mutation driven cancers, wherein the cancer comprises a first mutation that is G12C, and a second mutation at a position selected from V8A, V9Y, S17E, T58I, A59T, S65W, R68S, D69P, M72I, D92R, H95N, Y96D, Q99F, Q99W, Y96H, and F156L.

Another embodiment of present invention is (xxxii) the use of a compound according to any one of (i) to (xx) or (i') to (xvii') for the preparation of a medicament for the treatment or prophylaxis of KRAS mutation driven cancers, wherein the cancer is selected from pancreatic adenocarcinoma, colorectal cancer and non-small cell lung cancer.

Another embodiment of present invention is (xxxiii) the use of a compound according to any one of (i) to (xx) or (i') to (xvii') for the preparation of a medicament for the treatment or prophylaxis of KRAS mutation driven cancers, wherein the cancer comprises a first mutation that is G12C, and a second mutation at a position selected from V8A, V9Y, S17E, T58I, A59T, S65W, R68S, D69P, M72I, D92R, H95N, Y96D, Q99F, Q99W, Y96H, and F156L.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit mutant RAS (e.g. KRAS G12C) interaction with RAF, blocking the oncogenic MAPK signaling. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 0.1 to 1000 mg/kg of patient body weight per day, with the typical initial range of compound used being 0.3 to 15 mg/kg/day. In another embodiment, oral unit dosage forms, such as tablets and capsules, preferably contain from about 1 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 1 to 1000 mg of the compound of the invention compounded with about 1 to 1000 mg anhydrous lactose, about 1 to 1000 mg sodium croscarmellose, about 1 to 1000 mg polyvinylpyrrolidone (PVP) K30, and about 1 to 1000 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of formula (I), or a stereoisomer or pharmaceutically acceptable salt thereof. In a further embodiment includes a pharmaceutical composition comprising a compound of formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment of mutant KRAS-driven cancers. Another embodiment includes a pharmaceutical composition comprising a compound of Formula (I) for use in the treatment of mutant KRAS-driven cancers.

The following composition A and B illustrate typical compositions of the present invention, but serve merely as representative thereof.

### Composition A

A compound of the present invention can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Composition B

A compound of the present invention can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### INDICATIONS AND METHODS OF TREATMENT

The compounds of the invention induce a new binding pocket in KRAS by driving formation of a high affinity tri-complex between KRAS protein and the widely expressed cyclophilin A (CYPA), which inhibit KRAS interaction with downstream effectors, such as RAF and PI3K. Accordingly, the compounds of the invention are useful for inhibiting the propagating oncogenic MAPK and PI3K signaling, reducing cell proliferation, in particular cancer cells. Compounds of the invention are useful for termination of RAS signaling in cells that express RAS mutant, e.g. KRAS mutation driven pancreatic cancer, colorectal cancer, lung cancer, esophageal cancer, gallbladder cancer, melanoma ovarian cancer, endometrial cancer, etc. Alternatively, compounds of the invention are useful for termination of RAS signaling in malignant solid tumor where the oncogenic role of KRAS mutation is reinforced by dysregulation or mutation of effector pathways as MAPK, PI3K-AKT-mTOR (Mammalian target of rapamycin) driven signaling, for targeted therapy in pancreatic adenocarcinoma, colorectal cancer, non-small cell lung cancer, etc.

### SYNTHESIS

The compounds of the present invention can be prepared by any conventional means. Suitable processes for synthesizing these compounds as well as their starting materials are provided in the schemes below and in the examples. All substituents, in particular, R¹ to R⁷, A¹ and A² are as defined above unless otherwise indicated. Furthermore, and unless explicitly otherwise stated, all reactions, reaction conditions, abbreviations and symbols have the meanings well known to a person of ordinary skill in organic chemistry.

General synthetic routes for preparing the compound of formula (I) are shown below.

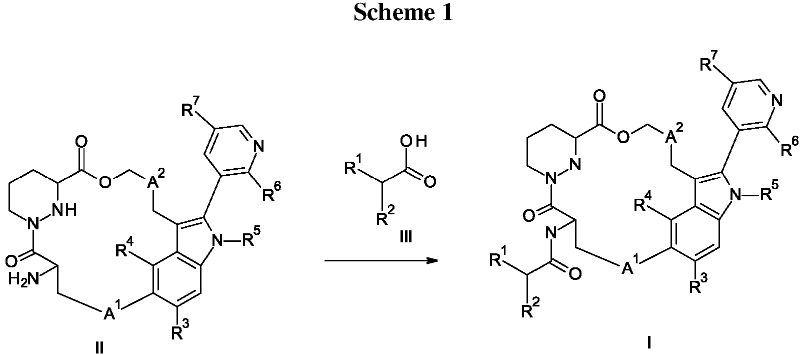

Compound of formula **II** was synthesized according to the procedure described in Intermediate **A** to **J.** Compound of formula **(I)** can be obtained by a coupling reaction between acid **(III)** and compound of formula **(II)** with coupling reagent(s), such as T₃P, HATU, PyBOP and EDCI/HOBt, in the presence of a base, such as TEA, DIEPA and DMAP.

Compounds of this invention can be obtained as mixtures of diastereomers or enantiomers, which can be separated by methods well known in the art, e.g. (chiral) HPLC or SFC. In another embodiment, compound of formula **(I)** can be obtained according to above scheme by using corresponding chiral starting materials.

This invention also relates to a process for the preparation of a compound of formula (I) comprising following step:
a) coupling reaction between compound of formula (II), 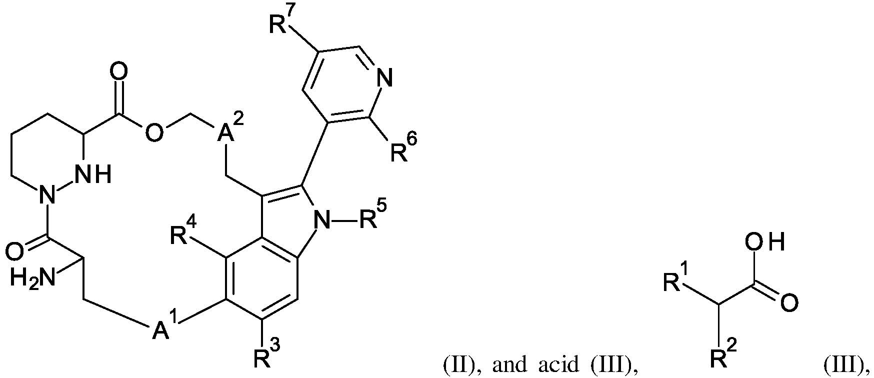
in the presence of a coupling reagent and a base to form the compound of formula (I);
wherein
in step a) the coupling reagent can be, for example, T₃P, HATU, PyBOP or EDCI/HOBt; the base can be, for example, TEA, DIEPA or DMAP.

A compound of formula (I) or (Ia) when manufactured according to the above process is also an object of the invention.

### EXAMPLES

The invention will be more fully understood by reference to the following examples. They should not, however, be construed as limiting the scope of the invention.

### ABBREVIATIONS

The invention will be more fully understood by reference to the following examples. They should not, however, be construed as limiting the scope of the invention.

Abbreviations used herein are as follows:
- ACN: acetonitrile
- aq.: Aqueous
- BOC-L-valine: (*S*)-2-((*tert*-butoxycarbonyl)amino)-3-methylbutanoic acid
- Boc-*N*-Me-Val-OH: *N*-(*tert*-Butoxycarbonyl)-*N*-methyl-*L*-valine
- (Boc)₂O: Di-tert-butyldicarbonate
- (*R*)-binap: (*R*)-(+)-2,2'-Bis(diphenylphosphino)-1,1'-binaphthyl
- CDCl₃:: deuterated chloroform
- CDI: 1,1'-Carbonyldiimidazole
- CD₃OD:: deuterated methanol
- COMU: (1-Cyano-2-ethoxy-2-oxoethylidenaminooxy)dimethylamino-morpholino-carbenium hexafluorophosphate
- DIEPA:: *N, N*-diethylpropylamine
- DIBAL-H: Diisobutylaluminium hydride
- DMAP:: 4-Dimethylaminopyridine
- DMF:: dimethyl formamide
- DMP: 1,1,1-Tris(acetyloxy)-1,1-dihydro-1,2-benziodoxol-3-(1*H*)-one
- DMSO:: dimethyl sulfoxide
- EDCI:: N-Ethyl-N'-(3-dimethylaminopropyl)carbodiimide hydrochloride
- EtOAc or EA:: ethyl acetate
- FRET: fluorescence resonance energy transfer
- HATU:: (1-[Bis(dimethylamino)methylene]-1*H*-1,2,3-triazolo[4,5-b]pyridinium 3-oxid hexafluorophosphate)
- hr(s):: hour(s)
- HPLC:: high performance liquid chromatography
- HOBt:: N-hydroxybenzotriazole
- H-VAL-OTBU HCl: (*S*)-*tert*-Butyl 2-amino-3-methylbutanoate hydrochloride
- [Ir(OMe)(COD)]₂: (1,5-Cyclooctadiene)(methoxy)iridium(I) dimer
- LDA: Lithium diisopropylamide
- MS: (ESI):: mass spectroscopy (electron spray ionization)
- min(s): minute(s)
- MTBE: Methyl *tert*-butyl ether
- NMM: *N*-Methylmorpholine
- NaBH(OAc)₃: Sodium triacetoxyborohydride
- NMR:: nuclear magnetic resonance
- NMO: 4-Methylmorpholine *N*-oxide
- obsd.: Observed
- Pd(dppf)Cl₂: [1,1'-Bis(diphenylphosphino)ferrocene]dichloropalladium(II)
- Pd(dtbpf)Cl₂: [1,1'-Bis(di-tert-butylphosphino)ferrocene]dichloropalladium(II)
- prep-HPLC: preparative high performance liquid chromatography
- PyBOP:: benzotriazol-1-yloxytripyrrolidinophosphonium hexafluorophosphate
- RT or rt:: room temperature
- sat.: saturated
- SFC: supercritical fluid chromatography
- TEA:: triethylamine
- TFA:: trifluoroacetic acid
- THF:: tetrahydrofuran
- TEA:: trimethylamine
- TMEDA: Tetramethylethylenediamine
- TMSCF₃: Trifluoromethyltrimethylsilane
- T₃P:: propylphosphonic anhydride

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃•H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.
Mass spectra (MS): generally only ions which indicate the parent mass are reported, and unless otherwise stated the mass ion quoted is the positive mass ion (MH)⁺.
NMR Spectra were obtained using Bruker Avance 400 MHz or 500MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention but should by no means represent a limitation within the meaning of the present invention:

### Preparation of Intermediate

### Intermediate A

### 1-[6-[(1S)-1-methoxyethyl]-5-(4,4,5,5-tetramethyl-1,3,2-dioxaborolan-2-yl)-3-pyridyl]-4-methyl-piperazine

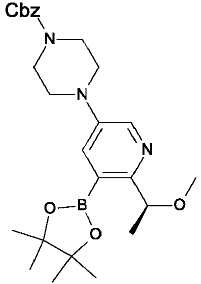

The title intermediate **A** was prepared according to the following scheme:

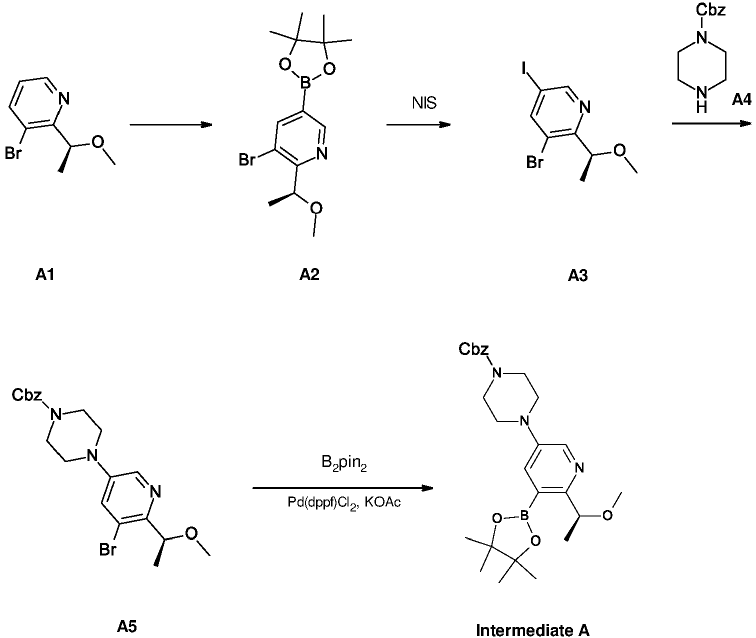

### Step 1: Preparation of 3-bromo-2-[(1S)-1-methoxyethyl]-5-(4,4,5,5-tetramethyl-1,3,2-dioxaborolan-2-yl)pyridine (compound A2)

To a solution of 3-bromo-2-[(1S)-1-methoxyethyl]pyridine (compound **A1,** 2.0 g, 9.26 mmol) and bis(pinacolato)diboron (3.5 g, 13.9 mmol) in THF (30 mL) were added 4,4'-di*-tert-*butyl-2,2'-bipyridin (372.7 mg, 1.39 mmol) and [Ir(OMe)(COD)]₂ (306.3 mg, 0.460 mmol). The mixture was stirred at 75 °C for 16 hours under N₂ protection. The mixture was filtrated and the filtrate was concentrated *in vacuo.* The residue was purified by silica gel chromatography (EA/PE: 0-20%) to afford 3-bromo-2-[(1S)-1-methoxyethyl]-5-(4,4,5,5-tetramethyl-1,3,2-dioxaborolan-2-yl)pyridine (compound **A2,** 2.4 g) as yellow oil. ¹H NMR (400 MHz, CDCl3) δ ppm 8.91 (d, *J =* 1.4 Hz, 1 H), 8.21 (d, *J =* 1.4 Hz, 1 H), 4.95 (q, *J =* 6.5 Hz, 1 H), 3.30 (s, 3 H), 1.49 (d, *J* = 6.5 Hz, 3 H), 1.35 (s, 12 H).

### Step 2: Preparation of 3-bromo-5-iodo-2-[(1S)-1-methoxyethyl]pyridine (compound A3)

To a solution of 3-bromo-2-[(1*S*)-1-methoxyethyl]-5-(4,4,5,5-tetramethyl-1,3,2-dioxaborolan-2-yl)pyridine (compound **A2,** 2.5 g, 7.3 mmol) in ACN (40 mL) was added N-iodosuccinimide (4.1 g, 18.27 mmol). The mixture was stirred at 90 °C for 40 hrs under N₂ protection. The reaction was quenched with saturated solution of Na₂SO₃ (40 mL) and the reaction mixture was extracted with EtOAc (30 mL, twice). The combined organic layer was washed with brine (50 mL), filtered and the filtrate was concentrated under vacuum. The residue was purified by silica gel chromatography (EA/PE: 0-20%) to afford 3-bromo-5-iodo-2-[(1S)-1-methoxyethyl]pyridine (compound **A3,** 660 mg) as yellow oil. MS calc'd 342 (MH⁺), measured 341.8 (MH⁺).

### Step 3: Preparation of benzyl 4-[5-bromo-6-[(1S)-1-methoxyethyl]-3-pyridyl]piperazine-1-carboxylate (compound A5)

To a solution of 3-bromo-5-iodo-2-[(1*S*)-1-methoxyethyl]pyridine (compound **A3,** 660 mg, 1.9 mmol) and 1-Cbz-piperazine (compound **A4,** 425.1 mg, 1.9 mmol) in toluene (10 mL) were added cesium carbonate (1.6 g, 4.83 mmol), (*R*)-BINAP (60.1 mg, 0.1 mmol) and palladium (II) acetate (43.3 mg, 0.19 mmol). The mixture was stirred at 100 °C for 12 hours under N₂ protection. The mixture was filtered and the filtrate was concentrated under vacuum. The residue was purified by silica gel chromatography (EA/PE: 0-50%) to afford benzyl 4-[5-bromo-6-[(1S)-1-methoxyethyl]-3-pyridyl]piperazine-1-carboxylate (compound **A5,** 740 mg) as a yellow solid. MS calc'd 434.1 (MH⁺), measured 434.1 (MH⁺).

### Step 4: Preparation of 1-[6-[(1S)-1-methoxyethyl]-5-(4,4,5,5-tetramethyl-1,3,2-dioxaborolan-2-yl)-3-pyridyl]-4-methyl-piperazine (Intermediate A)

To a solution of benzyl 4-[5-bromo-6-[(1*S*)-1-methoxyethyl]-3-pyridyl]piperazine-1-carboxylate (compound **A5,** 740 mg, 1.7 mmol) and bis(pinacolato)diboron (519.2 mg, 2.04 mmol) in toluene (12 mL) were added KOAc (418.0 mg, 4.26 mmol) and Pd(dppf)Cl₂ (124.7 mg, 0.170 mmol). The reaction mixture was stirred at 90 °C for 12 hrs under N₂ protection. The mixture was filtered and the filtrate was concentrated *in vacuo.* The residue was purified by silica gel column to afford 1-[6-[(1*S*)-1-methoxyethyl]-5-(4,4,5,5-tetramethyl-1,3,2-dioxaborolan-2-yl)-3-pyridyl]-4-methyl-piperazine (Intermediate **A,** 470 mg) as a brown solid. MS calc'd 482.3 (MH⁺), measured 482.2 (MH⁺).

### Intermediate B

### Methyl (3S)-1-[(2S)-3-(4-bromothiazol-2-yl)-2-(tert-butoxycarbonylamino)-propanoyl]hexahydropyridazine-3-carboxylate

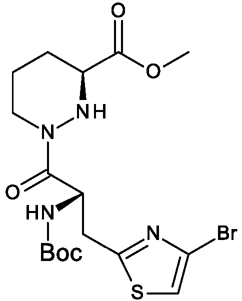

The intermediate **B** was prepared according to the following scheme:

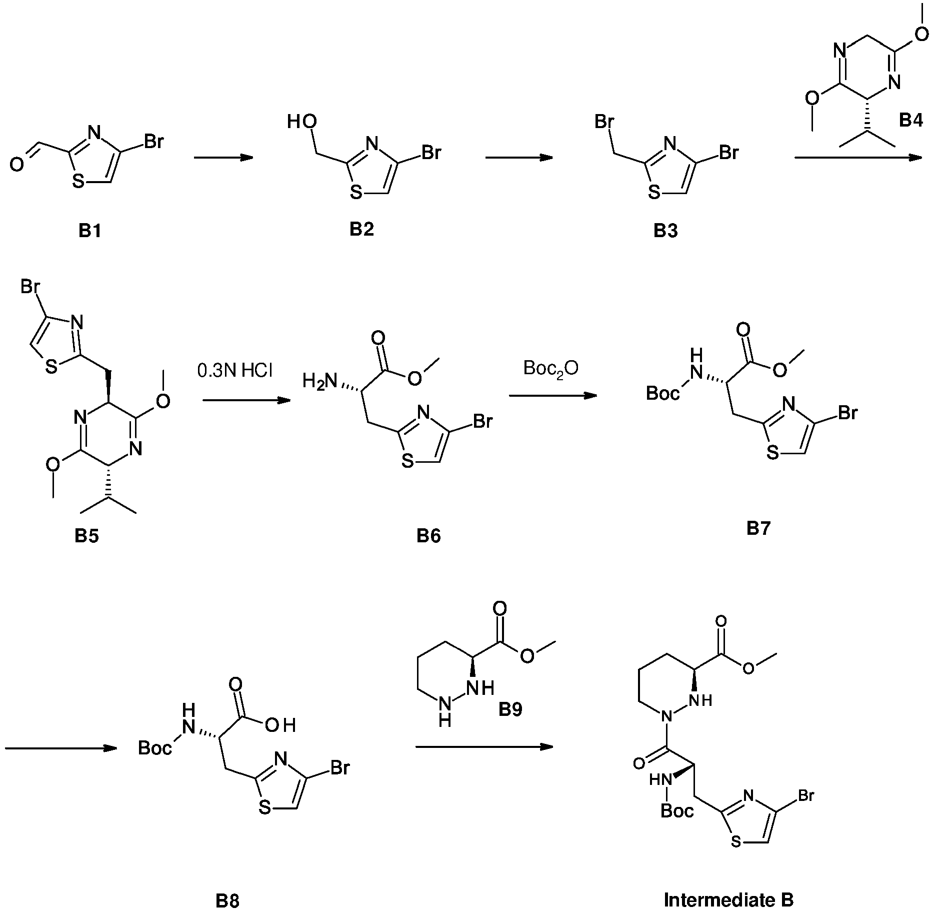

### Step 1: Preparation of (4-bromothiazol-2-yl)methanol (compound B2)

To a solution of 4-bromothiazole-2-carboxaldehyde (compound **B1,** 6.0 g, 31.25 mmol) in methanol (70 mL) was added sodium borohydride (1.7 g, 46.87 mmol) at 0 °C. The mixture was stirred at 25 °C for 1 hour. The reaction was quenched with water (300 mL) at 0 °C and the reaction mixture was extracted by ethyl acetate (200 mL, three times). The combined organic phase was washed with brine (150 mL, twice), dried over anhydrous sodium sulfate, filtered and the filtrate was concentrated under vacuum to afford (4-bromothiazol-2-yl)methanol (compound **B2,** 6g) as colorless oil.

### Step 2: Preparation of 4-bromo-2-(bromomethyl)thiazole (compound B3)

To a solution of (4-bromothiazol-2-yl)methanol (compound **B2,** 6.0 g, 30.92 mmol) in DCM (80 mL) was added CBr₄ (15.4 g, 46.38 mmol) and triphenylphosphine (12.1 g, 46.38 mmol) at 0 °C. After being stirred at 25 °C for 1 hour, the mixture was filtered and the filtrate was concentrated under vacuum. The residue was purified by silica gel column, eluted with ethyl acetate in petroleum ether (0-10%) to afford (4-bromothiazol-2-yl)methanol (compound **B3,** 6.0 g) as yellow oil. MS calc'd 255.9 (MH⁺), measured 255.9 (MH⁺).

### Step 3: Preparation of 4-bromo-2-[[(2S,5R)-5-isopropyl-3,6-dimethoxy-2,5-dihydropyrazin-2-yl]methyl]thiazole (compound B5)

To a mixture of (*R*)-2,5-dihydro-3,6-dimethoxy-2-isopropylpyrazine (compound **B4,** 4.3 g, 23.45 mmol) in THF (60 mL) was added n-butyllithium (10 mL, 25.22 mmol, 2.5 M) at -78 °C slowly. After addition, the mixture was stirred for 0.5 hour at -78 °C. 4-bromo-2-(bromomethyl)thiazole (compound **B3,** 5.4 g, 21.02 mmol) was added into above mixture at - 78 °C which was stirred for another 1 hour. The reaction was quenched with saturated solution of NH₄Cl (100 mL) and the reaction mixture was extracted with EtOAc (100 mL, twice). The combined organic layer was washed with brine (150 mL), dried over anhydrous sodium sulfate, filtered and the filtrate was concentrated under vacuum. The residue was purified by reversed-phase chromatography to afford 4-bromo-2-[[(2*S*,5*R*)-5-isopropyl-3,6-dimethoxy-2,5-dihydropyrazin-2-yl]methyl]thiazole (compound **B5,** 3.6 g) as yellow oil. MS calc'd 360 (MH⁺), measured 359.9 (MH⁺).

### Step 4: Preparation of methyl (2S)-2-amino-3-(4-bromothiazol-2-yl)propanoate (compound B6)

To a solution of 4-bromo-2-[[(2S,5R)-5-isopropyl-3,6-dimethoxy-2,5-dihydropyrazin-2-yl]methyl]thiazole (compound **B5,** 3.6 g, 10 mmol) in ACN (20 mL) was added hydrochloric acid (66.6 mL, 0.3 M). The mixture was stirred at 25 °C for 2 hours. The mixture was basified by saturated solution of NaHCO₃ until pH=8. The mixture was extracted with EtOAc (80 mL, six times). The combined organic layer was dried over anhydrous sodium sulfate, filtered and the filtrate was concentrated under vacuum to afford methyl (2*S*)-2-amino-3-(4-bromothiazol-2-yl)propanoate (compound **B6,** 3.1 g) as yellow oil. MS calc'd 264.9 (MH⁺), measured 264.9 (MH⁺).

### Step 5: Preparation of methyl (2S)-3-(4-bromothiazol-2-yl)-2-(tert-butoxycarbonylamino)propanoate (compound B7)

To a solution of methyl (2*S*)-2-amino-3-(4-bromothiazol-2-yl)propanoate (compound **B6,** 3.1 g, 11.69 mmol) in DCM (40 mL) were added triethylamine (2.9 g, 29.23 mmol) and (Boc)₂O (3.8 g, 17.54 mmol). After being stirred at 30 °C for 12 hours, the mixture was concentrated under vacuum. The residue was purified by silica gel column, eluted with ethyl acetate in petroleum ether (0-30%) to afford methyl (2*S*)-3-(4-bromothiazol-2-yl)-2-(*tert-*butoxycarbonylamino)propanoate (compound **B7,** 3.2 g) as yellow oil. MS calc'd 387(MNa⁺), measured 386.9 (MNa⁺).

### Step 6: Preparation of (2S)-3-(4-bromothiazol-2-yl)-2-(tert-butoxycarbonylamino)-propanoic acid (compound B8)

To a solution of methyl (2*S*)-3-(4-bromothiazol-2-yl)-2-(*tert-*butoxycarbonylamino)propanoate (compound **B7,** 3.2 g, 8.76 mmol) in THF (30 mL), methanol (2 mL) and water (10 mL) was added lithium hydroxide (0.4 mL, 43.81 mmol). After being stirred at 25 °C for 1 hour, the reaction mixture was acidified by 1 M solution of HCl until pH=5. The mixture was extracted with EtOAc (40 mL, twice). The combined organic layer was washed with brine (100 mL), dried over anhydrous sodium sulfate, filtered and the filtrate was concentrated under vacuum to afford (2*S*)-3-(4-bromothiazol-2-yl)-2-(*tert-*butoxycarbonylamino)propanoic acid (compound **B8,** 3.1 g) as yellow oil. MS calc'd 373(MNa⁺), measured 372.9 (MNa⁺).

### Step 7: Preparation of methyl (3S)-1-[(2S)-3-(4-bromothiazol-2-yl)-2-(tert-butoxycarbonylamino)propanoyllhexahydropyridazine-3-carboxylate (Intermediate B)

To a solution of (2*S*)-3-(4-bromothiazol-2-yl)-2-(*tert*-butoxycarbonylamino)propanoic acid (compound **B8,** 3.1 g, 8.83 mmol) in DCM (50 mL) was added methyl (3S)-hexahydropyridazine-3-carboxylate;hydrochloride (compound **B9,** 2.4 g, 13.24 mmol), EDCI (3.4 g, 17.65 mmol), 1-Hydroxybenzotriazole (238.5 mg, 1.77 mmol) and NMM (9.92 mL, 88.26 mmol) at 0 °C. After being stirred at 25 °C for 1 hour, the reaction mixture was diluted with water (60 mL) and extracted with EtOAc (60 mL, three times). The combined organic layer was washed with brine (100 mL), dried over anhydrous sodium sulfate, filtered and the filtrate was concentrated under vacuum. The residue was purified by silica gel column and eluted with ethyl acetate in petroleum ether (10-30%) to afford methyl (3*S*)-1-[(2*S*)-3-(4-bromothiazol-2-yl)-2-(tert-butoxycarbonylamino)propanoyl]hexahydropyridazine-3-carboxylate (intermediate **B,** 2.4 g). MS calc'd 477(MH⁺), measured 476.9 (MH⁺).

### Intermediate C

### (7S,13S)-7-amino-24-fluoro-(20M)-20-[2-[(1S)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

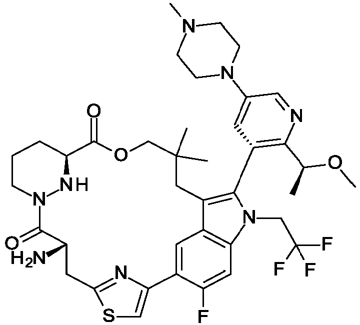

The title intermediate **C** was prepared according to the following scheme:

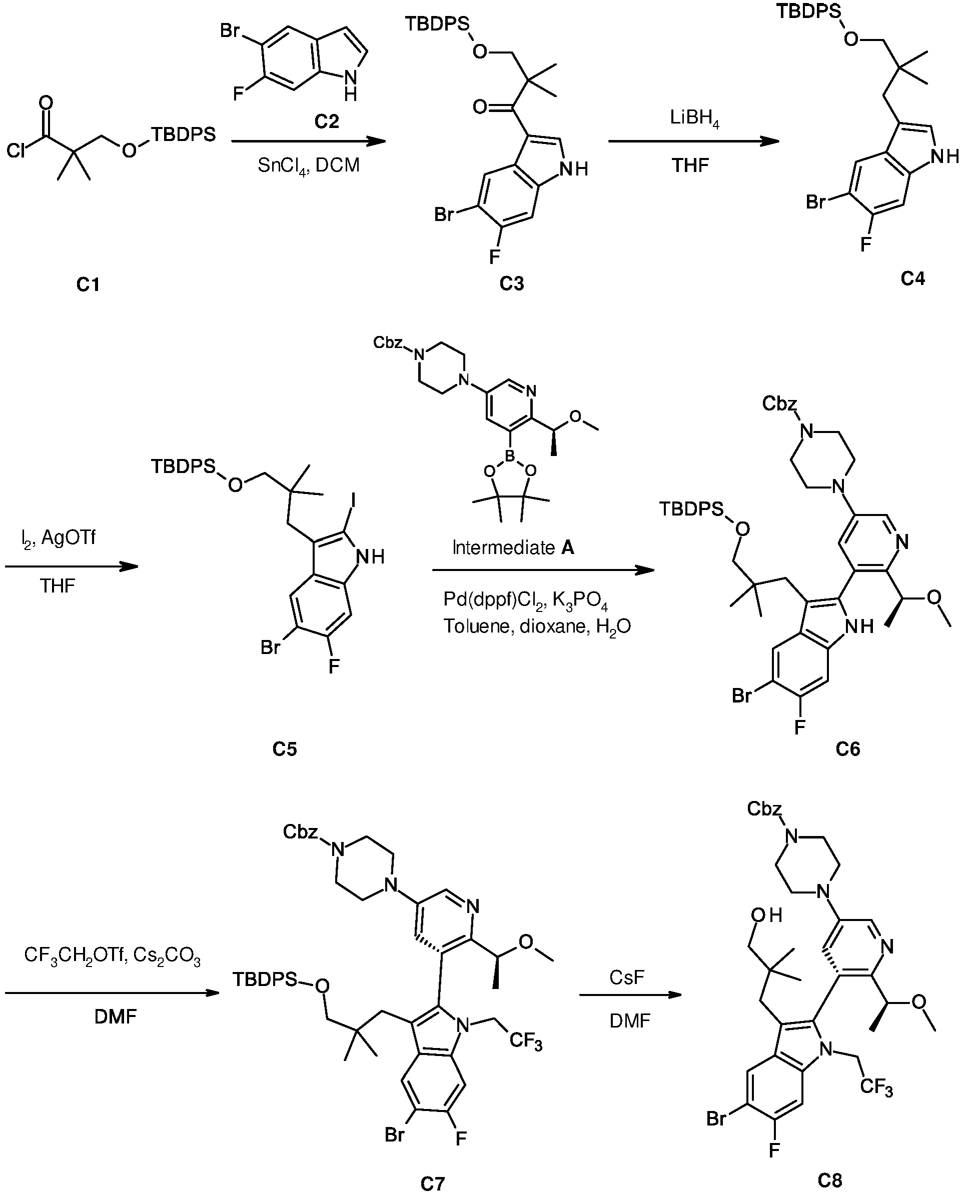

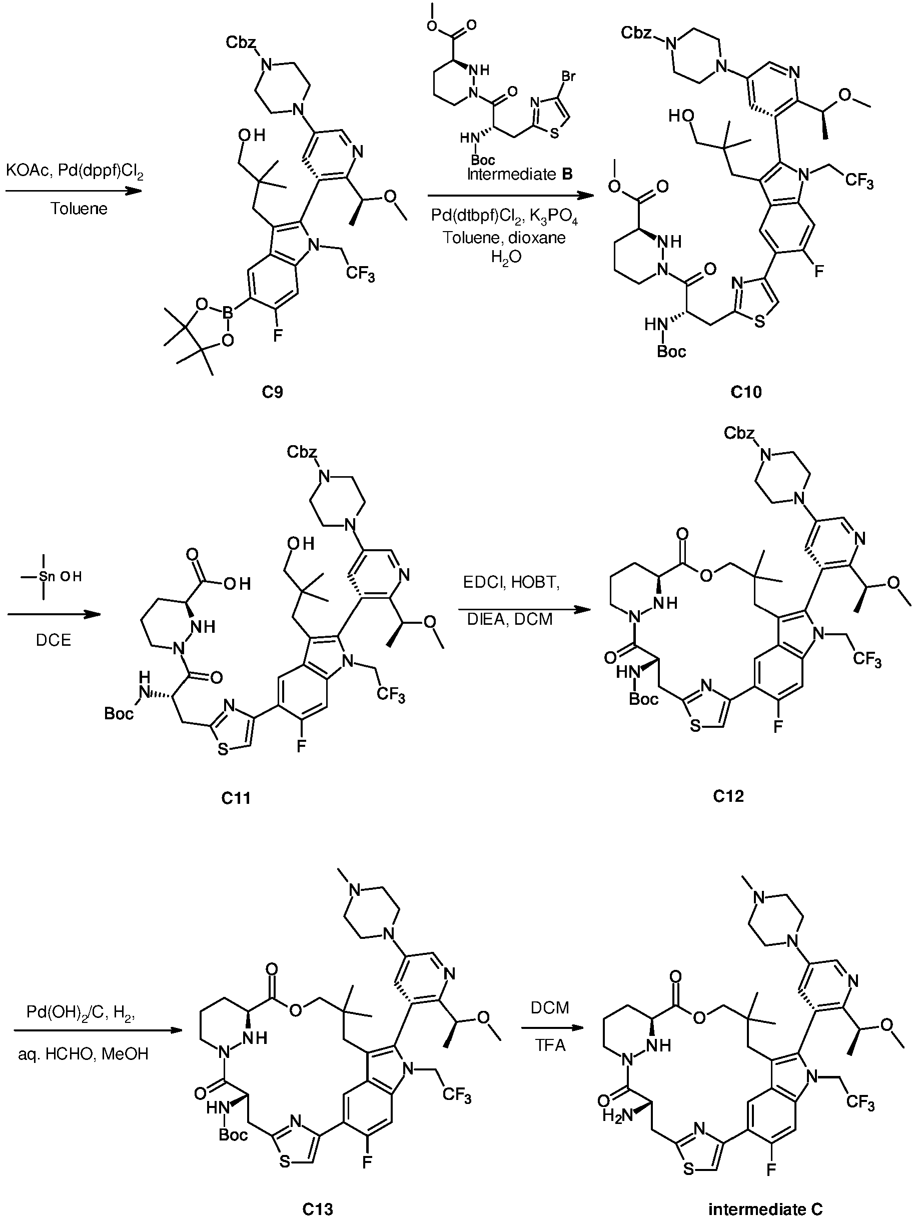

### Step 1: Preparation of 1-(5-bromo-6-fluoro-1H-indol-3-yl)-3-((tert-butyldiphenylsilyl) oxy)-2,2-dimethylpropan-1-one (compound C3)

To a mixture of 3-((*tert*-butyldiphenylsilyl)oxy)-2,2-dimethylpropanoyl chloride (compound **C1,** 35.0 g, 116.8 mmol) in DCM (400 mL) at 0 °C was added a solution of SnCl₄ (97.2 mL, 121.5 mmol) slowly. After the mixture was stirred at - 40 °C for 0.5 hour, 5-bromo-6-fluoro-1*H*-indole (compound **C2,** 25.0 g, 116.8 mmol) in DCM (200 mL) was added dropwise and the mixture was stirred at - 40 °C for another 15 min. After the reaction was completed, it was quenched with sat.NaHCO₃ aq. (800 mL), and the reaction mixture was extracted with EtOAc (900 mL, twice). The combined organic layer was washed with brine (700 mL), dried over Na₂SO₄, filtered and concentrated *in vacuo.* The residue was triturated with the solution (100 mL, Petroleum ether: Ethyl acetate = 8:1) and filtered. The filter cake was dried *in vacuo* to afford 1-(5-bromo-6-fluoro-1*H*-indol-3-yl)-3-((*tert*butyldiphenylsilyl)oxy)-2,2-dimethylpropan-1-one (compound **C3,** 50.0 g) as a yellow solid. MS calc'd 552.1 (MH⁺), measured 552.1 (MH⁺).

### Step 2: Preparation of [3-(5-bromo-6-fluoro-1H-indol-3-yl)-2,2-dimethyl-propoxy]-tert-butyl-diphenyl-silane (compound C4)

To a mixture of 1-(5-bromo-6-fluoro-1*H*-indol-3-yl)-3-((*tert*butyldiphenylsilyl)oxy)-2,2-dimethylpropan-1-one (compound **C3,** 50.0 g, 90.49 mmol) in THF (600 mL) was added LiBH₄ (48.4 mL, 193.49 mmol, 4 M in THF) dropwise at 0 °C. The mixture was stirred at 70 °C for 24 hrs under nitrogen atmosphere. After the reaction was completed, it was quenched by addition of water (600 mL) at 0 °C slowly and the reaction mixture was extracted with EtOAc (600 mL, twice). The combined organic layer was washed with brine (600 mL), dried over Na₂SO₄, filtered and concentrated *in vacuo.* The residue was purified by silica column chromatography (EtOAc in PE = 20% ~ 33%) to afford [3-(5-bromo-6-fluoro-1*H*-indol-3-yl)-2,2-dimethyl-propoxy]-*tert*-butyl-diphenyl-silane (compound **C4,** 46.0 g) as a white solid. MS calc'd 538.1 (MH⁺), measured 538.2 (MH⁺).

### Step 3: Preparation of [3-(5-bromo-6-fluoro-2-iodo-1H-indol-3-yl)-2,2-dimethyl-propoxyl-tert-butyl-diphenyl-silane (compound C5)

To a mixture of [3-(5-bromo-6-fluoro-1*H*-indol-3-yl)-2,2-dimethyl-propoxy]-*tert*-butyl-diphenyl-silane (compound **C4,** 35.4 g, 65.73 mmol) and iodine (18.4 g, 72.3 mmol) in THF (400 mL) was added silver trifluoromethanesulfonate (20.3 g, 78.88 mmol) at 0 °C. The mixture was stirred at 0 °C for 10 min. After the reaction was completed, it was quenched by sat. Na₂SO₃ aq. (400 mL) and EtOAc (400 mL) and the reaction mixture was filtered. The organic layer was washed with brine (100 mL), dried over Na₂SO₄, filtered and concentrated *in vacuo.* The residue was purified by silica column chromatography (EtOAc in PE = 0% ~ 2.5%) to afford [3-(5-bromo-6-fluoro-2-iodo-1*H*-indol-3-yl)-2,2-dimethyl-propoxy]-*tert*-butyl-diphenyl-silane (compound **C5,** 43.0 g) as a yellow solid. MS calc'd 664.0 (MH⁺), measured 664.1 (MH⁺).

### Step 4: Preparation of benzyl 4-[5-[5-bromo-3-[3-[tert-butyl(diphenyl)silyl]oxy-2,2-dimethyl-propyl]-6-fluoro-1H-indol-2-yl]-6-[(1S)-1-methoxyethyl]-3-pyridyl]piperazine-1-carboxylate (compound C6)

To a mixture of [3-(5-bromo-6-fluoro-2-iodo-1*H*-indol-3-yl)-2,2-dimethyl-propoxy]-*tert-*butyl-diphenyl-silane (compound **C5,** 16.7 g, 25.13 mmol) and benzyl 4-[6-[(1*S*)-1-methoxyethyl]-5-(4,4,5,5-tetramethyl-1,3,2-dioxaborolan-2-yl)-3-pyridyl]piperazine-1-carboxylate (Intermediate **A,** 16.7 g, 34.69 mmol) in a mixed solution of 1,4-dioxane (270 mL)/toluene (90 mL) /water (90 mL) were added potassium phosphate (15.7 g, 73.92 mmol) and Pd(dppf)Cl₂ (920 mg, 1.26 mmol). The mixture was stirred at 70 °C for 12 hrs under nitrogen atmosphere. After the reaction was completed, the mixture was filtered and concentrated *in vacuo.* The residue was purified by silica column chromatography (EtOAc in PE = 20% ~ 50%) to afford 4-[5-[5-bromo-3-[3-[*tert*-butyl(diphenyl)silyl]oxy-2,2-dimethyl-propyl]-6-fluoro-1*H-*indol-2-yl]-6-[(1*S*)-1-methoxyethyl]-3-pyridyl]piperazine-1-carboxylate (compound **C6,** 19.5 g) as a white solid. MS calc'd 891.3 (MH⁺), measured 891.3 (MH⁺).

### Step 5: Preparation of benzyl 4-[(5M)-5-[5-bromo-3-[3-[tert-butyl(diphenyl)silyl]oxy-2,2-dimethyl-propyl]-6-fluoro-1-(2,2,2-trifluoroethyl)indol-2-yl]-6-[(1S)-1-methoxyethyl]-3-pyridyl]piperazine-1-carboxylate(compound C7)

To a solution of 4-[5-[5-bromo-3-[3-[*tert*-butyl(diphenyl)silyl]oxy-2,2-dimethyl-propyl]-6-fluoro-1*H*-indol-2-yl]-6-[(1S)-1-methoxyethyl]-3-pyridyl]piperazine-1-carboxylate (compound **C6,** 14.5 g, 16.26 mmol) and Cs₂CO₃ (15. 9 g, 48.77 mmol) in DMF (200 mL) was added 2,2,2-trifluoroethyl trifluoromethanesulfonate (37.7 g, 162.56 mmol) dropwise at 0 °C, and the mixture was stirred at 20 °C for 12 hrs. After the reaction was completed, EtOAc (70 mL) and water (100 mL) were added and the layers were separated. The aqueous phase was extracted with EtOAc (70 mL, twice). Combined organic layer was washed with brine (100 mL, four times), dried over Na₂SO₄, filtered, and concentrated under vacuum to give a residue. The residue was purified by silica column chromatography to afford benzyl 4-[(5*M*)-5-[5-bromo-3-[3-[*tert-*butyl(diphenyl)silyl]oxy-2,2-dimethyl-propyl]-6-fluoro-1-(2,2,2-trifluoroethyl)indol-2-yl]-6-[(1S)-1-methoxyethyl]-3-pyridyl]piperazine-1-carboxylate (compound **C7,** 8.0 g, faster eluted) as yellow oil. MS calc'd 973.3 (MH⁺), measured 973.2 (MH⁺).

### Step 6: Preparation of benzyl 4-[(5M)-5-[5-bromo-6-fluoro-3-(3-hydroxy-2,2-dimethyl-propyl)-1-(2,2,2-trifluoroethyl)indol-2-yl]-6-[(1S)-1-methoxyethyl]-3-pyridyl]piperazine-1-carboxylate (compound C8)

To a solution of benzyl 4-[(5M)-5-[5-bromo-3-[3-[*tert*-butyl(diphenyl)silyl]oxy-2,2-dimethyl-propyl]-6-fluoro-1-(2,2,2-trifluoroethyl)indol-2-yl]-6-[(1S)-1-methoxyethyl]-3-pyridyl]piperazine-1-carboxylate (compound **C7,** 10.5 g, 10.78 mmol) in DMF (130 mL) was added cesium fluoride (8.2 g, 53.9 mmol) and the mixture was stirred at 60 °C for 24 hrs. After the reaction was completed, EtOAc (100 mL) and water (100 mL) were added and the layers were separated. The aqueous phase was extracted with EtOAc (100 mL, twice). The combined organic layer was washed with brine (80 mL, three times), dried over Na₂SO₄, filtered, and concentrated under vacuum to give a residue. The residue was purified by silica column chromatography (EtOAc in PE = 25% ~ 66%) to afford benzyl 4-[(5*M*)-5-[5-bromo-6-fluoro-3-(3-hydroxy-2,2-dimethyl-propyl)-1-(2,2,2-trifluoroethyl)indol-2-yl]-6-[(1*S*)-1-methoxyethyl]-3-pyridyl]piperazine-1-carboxylate (compound **C8,** 6.5 g) as a yellow solid. MS calc'd 735.2 (MH⁺), measured 735.1 (MH⁺).

### Step 7: Preparation of benzyl 4-[(5M)-5-[6-fluoro-3-(3-hydroxy-2,2-dimethyl-propyl)-5-(4,4,5,5-tetramethyl-1,3,2-dioxaborolan-2-yl)-1-(2,2,2-trifluoroethyl)indol-2-yl]-6-[(1S)-1-methoxyethyl]-3-pyridyl]piperazine-1-carboxylate (compound C9)

To a solution of benzyl 4-[(5*M*)-5-[5-bromo-6-fluoro-3-(3-hydroxy-2,2-dimethyl-propyl)-1-(2,2,2-trifluoroethyl)indol-2-yl]-6-[(1*S*)-1-methoxyethyl]-3-pyridyl]piperazine-1-carboxylate (compound **C8,** 5.4 g) , bis(pinacolato)diboron (2.8 g, 11.01 mmol) and potassium acetate (1.2 mL, 18.35 mmol) in toluene (70 mL) was added Pd(dppf)Cl₂ (537.1 mg, 0.73 mmol). The mixture was degassed and purged with nitrogen atmosphere for three times and the mixture was stirred at 90 °C for 12 hrs. After the reaction was completed, the mixture was cooled to room temperature. The reaction mixture was filtered and the filtrate was concentrated *in vacuo* to give a residue. The residue was purified by silica column chromatography (EtOAc in PE = 25% ~ 66%) to afford benzyl 4-[(5M)-5-[6-fluoro-3-(3-hydroxy-2,2-dimethyl-propyl)-5-(4,4,5,5-tetramethyl-1,3,2-dioxaborolan-2-yl)-1-(2,2,2-trifluoroethyl)indol-2-yl]-6-[(1S)-1-methoxyethyl]-3-pyridyl]piperazine-1-carboxylate (compound **C9,** 5.2 g) as yellow oil. MS calc'd 783.3 (MH⁺), measured 783.3 (MH⁺).

### Step 8: Preparation of methyl (3S)-1-[(2S)-3-[4-[(2M)-2-[5-(4-benzyloxycarbonylpiperazin-1-yl)-2-[(1S)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(tert-butoxycarbonylamino)-propanoyllhexahydropyridazine-3-carboxylate (compound C10)

To a mixture of methyl (3*S*)-1-[(2*S*)-3-(4-bromothiazol-2-yl)-2-(*tert-*butoxycarbonylamino)propanoyl]hexahydropyridazine-3-carboxylate (intermediate **B,** 2.7 g, 5.69 mmol), benzyl 4-[(5*M*)-5-[6-fluoro-3-(3-hydroxy-2,2-dimethyl-propyl)-5-(4,4,5,5-tetramethyl-1,3,2-dioxaborolan-2-yl)-1-(2,2,2-trifluoroethyl)indol-2-yl]-6-[(1*S*)-1-methoxyethyl]-3-pyridyl]piperazine-1-carboxylate (compound **C9,** 4.9 g, 6.32 mmol) in toluene (60 mL)/1,4-dioxane (20 mL) / water (20 mL) were added K₃PO₄ (3.4 g, 15.81 mmol) and Pd(dtbpf)Cl₂ (412.2 mg, 0.63 mmol) under nitrogen atmosphere. The mixture was stirred at 70 °C for 12 hrs. After the reaction was completed, the mixture was concentrated *in vacuo* to give a residue. The residue was purified by silica column (EtOAc in PE = 10% ~ 75%) to afford methyl (3*S*)-1-[(2*S*)-3-[4-[(2*M*)-2-[5-(4-benzyloxycarbonylpiperazin-1-yl)-2-[(1*S*)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(*tert*-butoxycarbonylamino)-propanoyl]hexahydropyridazine-3-carboxylate (compound **C10,** 3.6 g) as a brown solid. MS calc'd 1053.4 (MH⁺), measured 1053.3 (MH⁺).

### Step 9: Preparation of (3S)-1-[(2S)-3-[4-[(2M)-2-[5-(4-benzyloxycarbonylpiperazin-1-yl)-2-[(1S)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(tert-butoxycarbonylamino)propanoyl]hexahydropyridazine-3-carboxylic acid (compound C11)

To a solution of methyl (3*S*)-1-[(2*S*)-3-[4-[(2*M*)-2-[5-(4-benzyloxycarbonylpiperazin-1-yl)-2-[(1*S*)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(*tert*-butoxycarbonylamino)-propanoyl]-hexahydropyridazine-3-carboxylate (compound **C10,** 3.6 g, 3.42 mmol) in DCE (50 mL) was added trimethylstannanol (2.4 g, 13.67 mmol) and the mixture was stirred at 60 °C for 12 hrs. After the reaction was completed, EtOAc (80 mL) and water (60 mL) were added and the layers were separated. The aqueous phase was extracted with EtOAc (80 mL, twice). The combined organic layer was washed with brine (100 mL), dried over Na₂SO₄, filtered, and concentrated under vacuum to give (3*S*)-1-[(2*S*)-3-[4-[(2*M*)-2-[5-(4-benzyloxycarbonylpiperazin-1-yl)-2-[(1*S*)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(*tert*-butoxycarbonylamino)propanoyl]hexahydropyridazine-3-carboxylic acid (compound **C11,** 4.3 g) as a brown solid. MS calc'd 1039.4 (MH⁺), measured 1039.2 (MH⁺).

### Step 10: Preparation of benzyl 4-[5-[(7S,13S)-7-(tert-butoxycarbonylamino)-24-fluoro-17,17-dimethyl-8,14-dioxo-21-(2,2,2-trifluoroethyl)-15-oxa-4-thia-9,21,27,28-tetrazapentacyclo[17.5.2.1^{2,5}.1^{9,3}.0^{22,26}]octacosa-1(25),2,5(28),19,22(26),23-hexaen-(20M)-20-yl]-6-[(1S)-1-methoxyethyl]-3-pyridyl]piperazine-1-carboxylate (compound C12)

To a mixture of (3*S*)-1-[(2*S*)-3-[4-[(2*M*)-2-[5-(4-benzyloxycarbonylpiperazin-1-yl)-2-[(1*S*)-1-methoxyethyl]-3-pyridyl]-6-fluoro-3-(3-hydroxy-2,2-dimethyl-propyl)-1-(2,2,2-trifluoroethyl)indol-5-yl]thiazol-2-yl]-2-(*tert*-butoxycarbonylamino)propanoyl]hexahydropyridazine-3-carboxylic acid (compound **C11,** 4.3 g, 4.14 mmol) in DCM (430 mL) was added DIEA (14.4 mL, 82.76 mmol), EDCI (11.9 g, 62.07 mmol) and 1-hydroxybenzotriazole (1.4 g, 10.35 mmol) at 0 °C. The mixture was stirred at 15 °C for 12 hrs. After the reaction was completed, the mixture was concentrated *in vacuo,* then diluted with water (80 mL), extracted with EtOAc (80 mL, twice). The combined organic layer was washed with brine (80 mL), dried over Na₂SO₄, filtered and concentrated *in vacuo.* The residue was purified by silica column chromatography (EtOAc in PE = 25% ~ 66%) to afford benzyl 4-[5-[(7*S*,13*S*)-7-(*tert-*butoxycarbonylamino)-24-fluoro-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-(20M)-20-yl]-6-[(1S)-1-methoxyethyl]-3-pyridyl]piperazine-1-carboxylate (compound **C12,** 3.1 g) as yellow gum. MS calc'd 1021.4 (MH⁺), measured 1021.2 (MH⁺).

### Step 11: Preparation of tert-butyl N-[(7S,13S)-24-fluoro-(20M)-20-[2-[(1S)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5},1^{9,13}.0^{22,26}]octacosa-1(25),2,5(28),19,22(26),23-hexaen-7-yl]carbamate (compound C13)

To a mixture of benzyl 4-[5-[(7*S*,13*S*)-7-(tert-butoxycarbonylamino)-24-fluoro-17,17-dimethyl-8,14-dioxo-21-(2,2,2-trifluoroethyl)-15-oxa-4-thia-9,21,27,28-tetrazapentacyclo[17.5.2.1^{2,5}. 1^{9,13}.0^{22,26}]octacosa-1(25),2,5(28),19,22(26),23-hexaen-(20*M*)-20-yl]-6-[(1S)-1-methoxyethyl]-3-pyridyl]piperazine-1-carboxylate (compound **C12,** 3.1 g, 3.04 mmol) and formaldehyde aqueous (775.0 mg, 9.55 mmol) in methanol (150 mL) was added Pd(OH)₂ on activated carbon (2.79 g, 3.97 mmol). The mixture was degassed and purged with H₂ three times. The mixture was hydrogenated at 30 °C for 18 hrs. After the reaction was completed, the mixture was filtered and the filtrate was concentrated *in vacuo* to afford *tert-*butyl *N-*[(7*S*,13*S*)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-l(25),2,5(28),19,22(26),23-hexaen-7-yl]carbamate (compound **C13,** 2.6 g) as a brown solid. MS calc'd 901.3 (MH⁺), measured 901.3 (MH⁺).

### Step 12: Preparation of (7S,13S)-7-amino-24-fluoro-(20M)-20-[2-[(1S)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5},1^{9,13}.0^{22,26}]octacosa-1(25),2,5(28),19,22(26),23-hexaene-8,14-dione (intermediate C)

To a mixture of *tert*-butyl *N*-[(7*S*,13*S*)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound **C13,** 2.6 g, 2.89 mmol) in DCM (18 mL) was added TFA (14.0 mL, 181.72 mmol). The mixture was stirred at 15 °C for 0.5 h. After the reaction was completed, the mixture was concentrated *in vacuo* and diluted with sat. NaHCO₃ (30 mL), extracted with EtOAc (30 mL, three times). The combined organic layer was washed with brine (50 mL), dried over Na₂SO₄, filtered and concentrated *in vacuo* to afford (7S,13S)-7-amino-24-fluoro-(20M)-20-[2-[(1S)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (intermediate **C,** 2.0 g) as a yellow solid, which was used directly in the next step. MS calc'd 801.3 (MH⁺), measured 801.2 (MH⁺)

### Intermediate D

### (7S,13S)-7-amino-21-ethyl-24-fluoro-(20M)-20-[2-[(1S)-1-methoxyethyl]-5-(4-methylpiperazin-1-yl)-3-pyridyl]-17,17-dimethyl-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

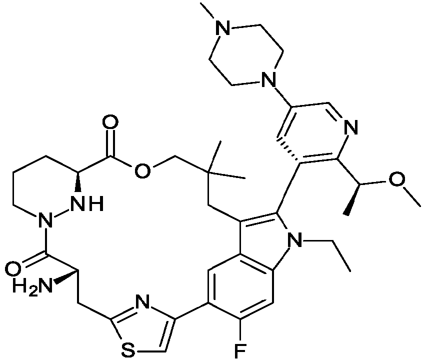

The title compound was prepared in analogy to the preparation of Intermediate **C** by using iodoethane instead of 2,2,2-trifluoroethyl trifluoromethanesulfonate.

### Intermediate E

### (7S,13S)-7-amino-24-fluoro-(20M)-20-[2-[(1S)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

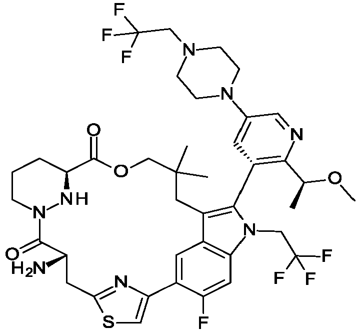

The compound was prepared according to the following scheme:

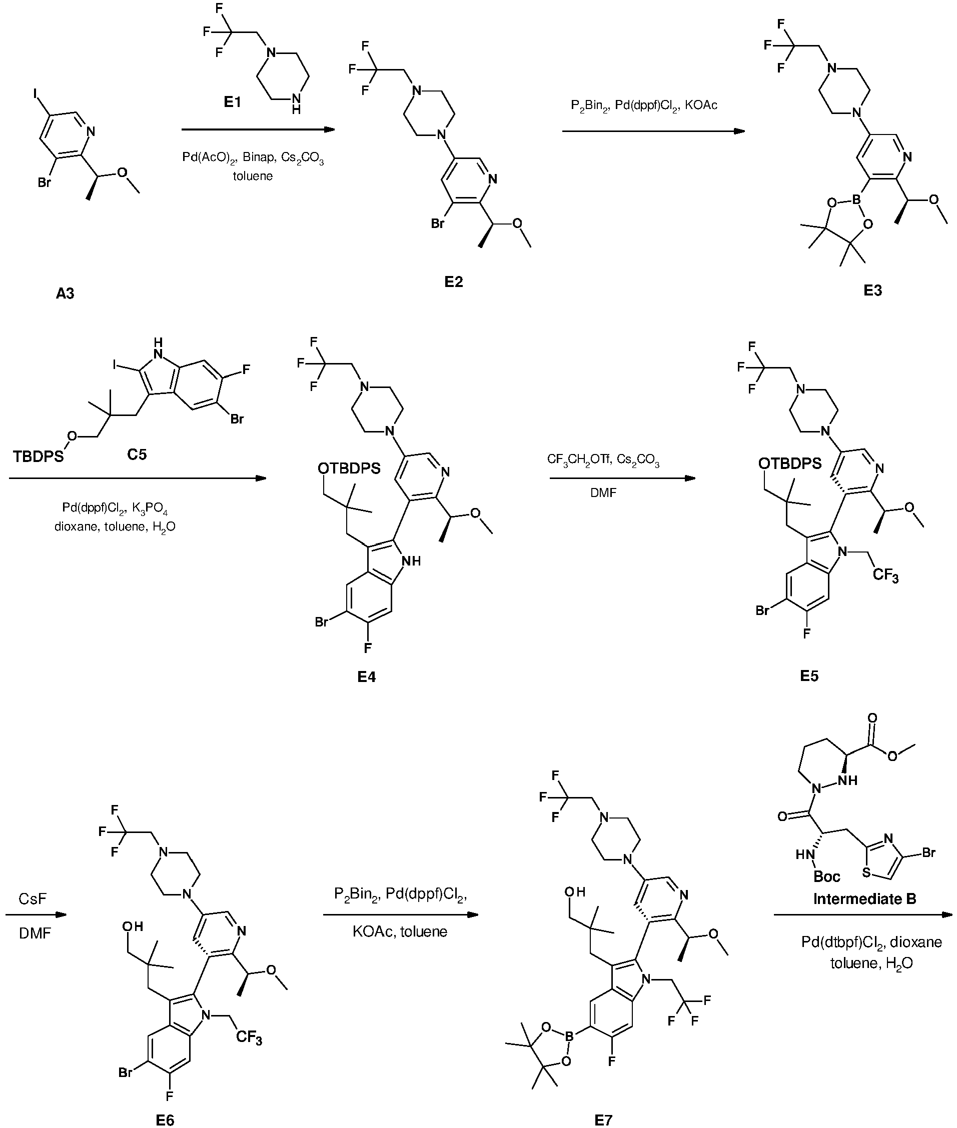

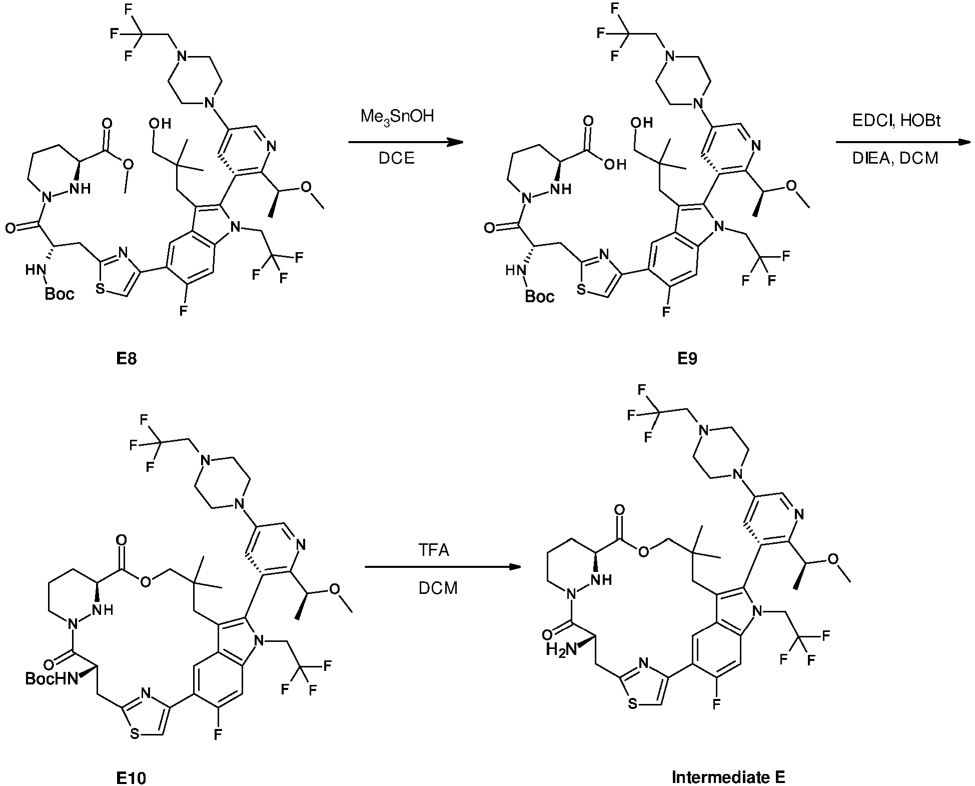

### Step 1: Preparation of 1-[5-bromo-6-[(1S)-1-methoxyethyl]-3-pyridyl]-4-(2,2,2-trifluoroethyl)piperazine (compound E2).

To a mixture of 3-bromo-5-iodo-2-[(1*S*)-1-methoxyethyl]pyridine (compound **A3,** 2.03 g, 5.95 mmol) and 1-(2,2,2-trifluoroethyl)piperazine (compound **E1,** 1.0 g, 5.95 mmol) in toluene (15 mL) were added Cs₂CO₃ (4.85 g, 14.88 mmol), (*R*)-binap (92.6 mg, 0.15 mmol) and Pd(OAc)₂ (66.8 mg, 0.3 mmol). The reaction mixture was degassed and purged with nitrogen for 3 times and the mixture was stirred at 100 °C for 12 hrs under nitrogen atmosphere. After being cooled to room temperature, the reaction mixture was filtered and the filtrate was concentrated *in vacuo* to give a residue. The residue was purified by column chromatography to afford 1-[5-bromo-6-[(1*S*)-1-methoxyethyl]-3-pyridyl]-4-(2,2,2-trifluoroethyl)piperazine (compound **E2,** 2.0 g) as yellow oil. MS calc'd 382.2 (MH⁺), measured 382.1 (MH⁺).

### Step 2: 1-[6-[(1S)-1-methoxyethyl]-5-(4,4,5,5-tetramethyl-1,3,2-dioxaborolan-2-yl)-3-pyridyl]-4-(2,2,2-trifluoroethyl)piperazine (compound E3).

To a solution of 1-[5-bromo-6-[(1S)-1-methoxyethyl]-3-pyridyl]-4-(2,2,2-trifluoroethyl)piperazine (compound **E2,** 3.2 g, 8.37 mmol), bis(pinacolato)diboron (3.19 g, 12.56 mmol) and KOAc (2.1 g, 20.93 mmol) in toluene (50 mL) was added Pd(dppf)Cl₂ (306.3 mg, 0.42 mmol). The mixture was degassed and purged with nitrogen for 3 times and the mixture was stirred at 90 °C for 12 hrs under nitrogen atmosphere. After being cooled to the room temperature, the reaction mixture was filtered, the filtrate was concentrated *in vacuo* to give a residue, which was purified by reversed phase column to afford 1-[6-[(1S)-1-methoxyethyl]-5-(4,4,5,5-tetramethyl-1,3,2-dioxaborolan-2-yl)-3-pyridyl]-4-(2,2,2-trifluoroethyl)piperazine (compound **E3,** 1.9 g) as a yellow gum. MS calc'd 430.2 (MH⁺), measured 348.4 (M-C₆H₁₀+H⁺).

### Step 3: Preparation of [3-[5-bromo-6-fluoro-2-[2-[(1S)-1-methoxyethyl]-5-[4-(2,2,2-trifluoroethyl)piperazin-1-yl]-3-pyridyl]-1H-indol-3-yl]-2,2-dimethyl-propoxy]-tert-butyl-diphenyl-silane (compound E4).

To a solution of 1-[6-[(1*S*)-1-methoxyethyl]-5-(4,4,5,5-tetramethyl-1,3,2-dioxaborolan-2-yl)-3-pyridyl]-4-(2,2,2-trifluoroethyl)piperazine (compound **E3,** 1.9 g, 4.41 mmol), [3-(5-bromo-6-fluoro-2-iodo-1*H*-indol-3-yl)-2,2-dimethyl-propoxy]-*tert*-butyl-diphenyl-silane (compound **C5,** 2.1 g, 3.15 mmol) in 1,4-dioxane (24 mL), water (8 mL) and toluene (8 mL) was added K₃PO₄ (2.1 g, 9.5 mmol) and Pd(dppf)Cl₂ (231 mg, 0.37 mmol). The mixture was degassed by bubbling nitrogen for 2 min, and the reaction mixture was stirred at 70 °C for 12 hrs. After being cooled to room temperature, the reaction mixture was filtered. The filtrate was concentrated *in vacuo* to give a residue. The residue was purified by column chromatography (EtOAc in PE : 30% - 60%) to afford [3-[5-bromo-6-fluoro-2-[2-[(1*S*)-1-methoxyethyl]-5-[4-(2,2,2-trifluoroethyl)piperazin-1-yl]-3-pyridyl]-1*H*-indol-3-yl]-2,2-dimethyl-propoxy]-*tert*-butyl-diphenyl-silane (compound **E4,** 960.0 mg) as a yellow gum. MS calc'd 839.3 (MH⁺), measured 839.3 (MH⁺).

### Step 4: Preparation of [3-[5-bromo-6-fluoro-(2M)-2-[2-[(1S)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propoxy]-tert-butyl-diphenyl-silane (compound E5).

To a solution of [3-[5-bromo-6-fluoro-2-[2-[(1S)-1-methoxyethyl]-5-[4-(2,2,2-trifluoroethyl)piperazin-1-yl]-3-pyridyl]-1*H*-indol-3-yl]-2,2-dimethyl-propoxy]-*tert*-butyl-diphenyl-silane (compound **E4,** 1 g, 1.14 mmol) in DMF (35 mL) was added Cs₂CO₃ (1.1 g, 3.44 mmol) and 2,2,2-trifluoroethyl trifluoromethanesulfonate (2.7 g, 11.63 mmol) at 0 °C. After being stirred at 20 °C for 15 hrs, the reaction mixture was poured into water (100 mL), and extracted with EtOAc (50 mL, three times). The combined organic was washed with brine (50 mL, three times), dried over Na₂SO₄, filtered and concentrated under vacuum to give a residue which was purified by column chromatography (EtOAc in PE: 30% - 40%) to afford [3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propoxy]-*tert*-butyl-diphenyl-silane (compound **E5,** 640.0 mg, 0.69 mmol, faster eluted) as a white solid. MS calc'd 921.3 (MH⁺), measured 921.4 (MH⁺).

### Step 5: Preparation of 3-[5-bromo-6-fluoro-(2M)-2-[2-[(1S)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound E6).

To a solution of [3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propoxy]-*tert*-butyl-diphenyl-silane (compound **E5,** 640.0 mg, 0.69 mmol) in DMF (7 mL) was added cesium fluoride (421.8 mg, 2.78 mmol). The mixture was stirred at 60 °C for 16 hrs. After being cooled to room temperature, the reaction mixture was filtered and the filtrate was concentrated *in vacuo* to give a residue. The residue was purified by column chromatography (EtOAc in PE : 30% - 60%) to afford 3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound **E6,** 360.0 mg) as yellow oil. MS calc'd 683.2 (MH⁺), measured 683.1 (MH⁺).

### Step 6: Preparation of 3-[5-bromo-6-fluoro-(2M)-2-[2-[(1S)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound E7).

To a solution of 3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound **E6,** 360.0 mg, 0.53 mmol), bis(pinacolato)diboron (200.6 mg, 0.79 mmol) in toluene (6 mL) was added potassium acetate (129 mg, 1.32 mmol) and Pd(dppf)Cl₂ (40 mg, 0.1 mmol). The reaction mixture was degassed by bubbling nitrogen for 5 min then stirred at 80 °C for 15 hrs. After being cooled to room temperature, the reaction mixture was filtered and the filtrate was concentrated *in vacuo* to give a residue. The residue was purified by column chromatography (EtOAc in PE : 30% - 50%) to afford 3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound **E7,** 300.0 mg) as yellow gum. MS calc'd 731.4 (MH⁺), measured 731.4 (MH⁺).

### Step 7: Preparation of methyl (3S)-1-[(2S)-2-(tert-butoxycarbonylamino)-3-[4-[6-fluoro-3-(3-hydroxy-2,2-dimethyl-propyl)-(2M)-2-[2-[(1S)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylate (compound E8).

To a mixture of 3-[5-bromo-6-fluoro-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-3-yl]-2,2-dimethyl-propan-1-ol (compound **E7,** 0.3 g, 0.41 mmol) and methyl (3*S*)-1-[(2*S*)-3-(4-bromothiazol-2-yl)-2-(*tert-*butoxycarbonylamino)propanoyllhexahydropyridazine-3-carboxylate (intermediate **B,** 196.7 mg, 0.41 mmol) in toluene (3 mL), 1,4-dioxane (1 mL) and water (1 mL) were added K₃PO₄ (221.3 mg, 1.04 mmol) and Pd(dtbpf)Cl₂ (27.05 mg, 0.04 mmol). The mixture was stirred at 70 °C for 12 hrs under nitrogen atmosphere. After being cooled to room temperature, the reaction mixture was filtered and the filtrate was concentrated *in vacuo* to give a residue. The residue was purified by column chromatography (EtOAc in PE : 60% - 80%) to afford methyl (3*S*)-1-[(2*S*)-2-(*tert-*butoxycarbonylamino)-3-[4-[6-fluoro-3-(3-hydroxy-2,2-dimethyl-propyl)-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylate (compound **E8,** 200.0 mg) as yellow gum. MS calc'd 1001.4 (MH⁺), measured 1001.4 (MH⁺).

### Step 8: Preparation of (3S)-1-[(2S)-2-(tert-butoxycarbonylamino)-3-[4-[6-fluoro-3-(3-hydroxy-2,2-dimethyl-propyl)-(2M)-2-[2-[(1S)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylic acid (compound E9).

To a mixture of methyl (3*S*)-1-[(2*S*)-2-(*tert-*butoxycarbonylamino)-3-[4-[6-fluoro-3-(3-hydroxy-2,2-dimethyl-propyl)-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylate (compound **E8,** 200.0 mg, 0.2 mmol) in DCE (5 mL) was added Me₃SnOH (200.0 mg, 1.11 mmol). The mixture was stirred at 60 °C for 12 hrs. The reaction mixture was concentrated under vacuum to give a residue. EtOAc (10 mL) and water (10 mL) were added to the residue and the layers were separated. The aqueous phase was extracted with EtOAc (15 mL, twice). The combined organic layer was washed with brine (20 mL), dried over Na₂SO₄, filtered, and concentrated under vacuum to afford (3*S*)-1-[(2*S*)-2-(*tert-*butoxycarbonylamino)-3-[4-[6-fluoro-3-(3-hydroxy-2,2-dimethyl-propyl)-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylic acid (compound **E9,** 188.0 mg) as a brown solid. MS calc'd 987.4 (MH⁺), measured 987.4 (MH⁺).

### Step 9: Preparation of tert-butyl N-[(7S,13S)-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound E10).

To a mixture of (3S)-1-[(2S)-2-(*tert*-butoxycarbonylamino)-3-[4-[6-fluoro-3-(3-hydroxy-2,2-dimethyl-propyl)-(2*M*)-2-[2-[(1*S*)-1-methoxyethyl]-5-[4-(2,2,2-trifluoroethyl)piperazin-1-yl]-3-pyridyl]-1-(2,2,2-trifluoroethyl)indol-5-yl]thiazol-2-yl]propanoyl]hexahydropyridazine-3-carboxylic acid (compound **E9,** 188.0 mg, 0.19 mmol) in DCM (20 mL) were added DIEA (0.7 mL, 3.81 mmol), EDCI (550.0 mg, 2.87 mmol) and HOBt (65.0 mg, 0.48 mmol) at 0 °C. After being stirred at 20 °C for 12 hrs, the reaction mixture was poured into water (20 mL) and extracted with EtOAc (20 mL, three times). The combined organic layer was washed with brine (30 mL), dried over Na₂SO₄, filtered and concentrated under vacuum to give a residue which was purified by column chromatography (EtOAc in PE : 50% - 70%) to afford *tert-*butyl *N-*[(7*S,*13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound **E10,** 110.0 mg) as a yellow solid. MS calc'd 969.4 (MH⁺), measured 969.5 (MH⁺).

### Step 10: Preparation of (7S,13S)-7-amino-24-fluoro-(20M)-20-[2-[(1S)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate E).

To a solution of *tert*-butyl *N*-[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound **E10,** 110.0 mg, 0.11 mmol) in DCM (1 mL) was added TFA (1.0 mL, 12.98 mmol). The mixture was stirred at 20 °C for 1 h. After the reaction was completed, the reaction mixture was concentrated under vacuum to give a residue. Sat. NaHCO₃ aq. (20 mL) was added and the mixture was extracted with EtOAc (15 mL, three times). The combined organic layer was washed with brine (20 mL), dried over anhydrous sodium sulfate, filtered and concentrated *in vacuo* to afford (7S,13S)-7-amino-24-fluoro-(20M)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **E,** 98.0 mg) as a yellow solid. MS calc'd 869.4 (MH⁺), measured 869.2 (MH⁺).

### Intermediate F

### (7S,13S)-7-amino-21-ethyl-24-fluoro-(20M)-20-[2-[(1S)-1-methoxyethyl]-5-[4-(2,2,2-trifluoroethyl)piperazin-1-yl]-3-pyridyl]-17,17-dimethyl-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

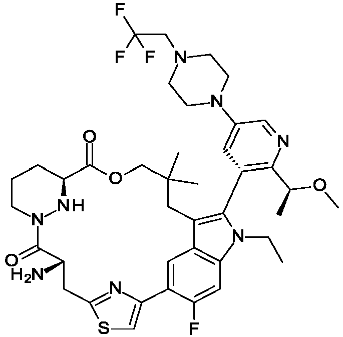

The title compound was prepared in analogy to the preparation of Intermediate **E** by using iodoethane instead of 2,2,2-trifluoroethyl trifluoromethanesulfonate.

### Intermediate G

### (7S,13S)-7-amino-21-ethyl-24-fluoro-(20M)-20-[2-[(1S)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione

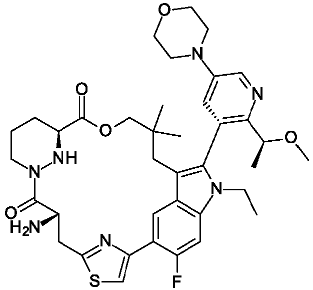

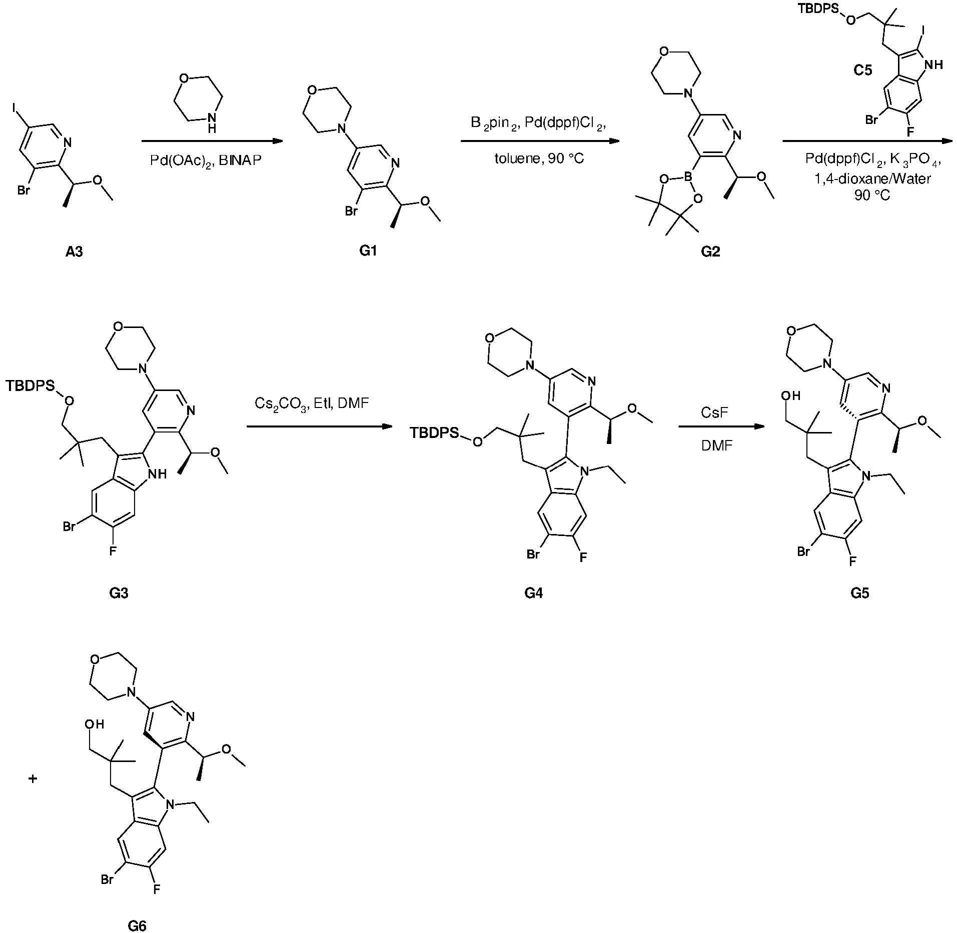

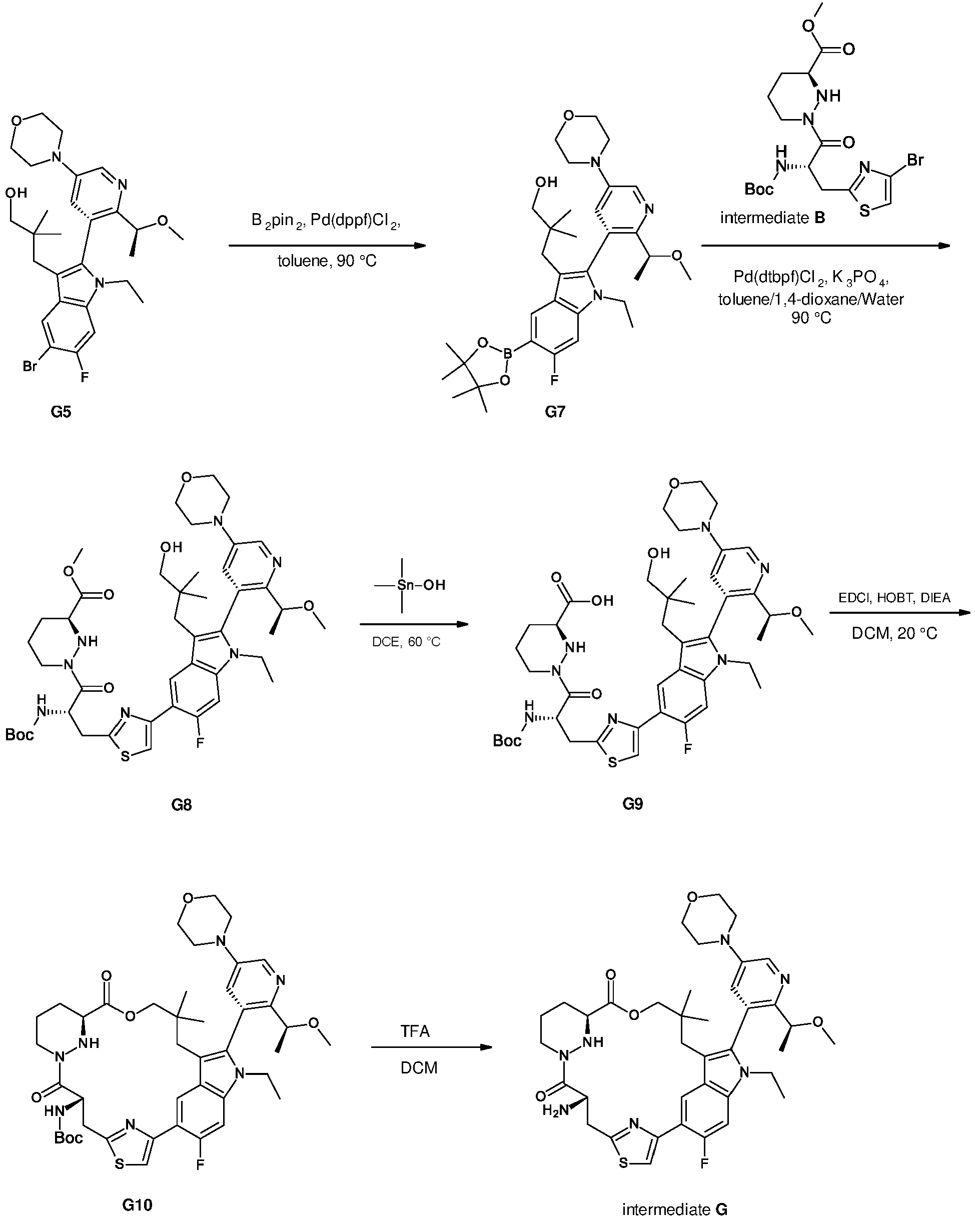

### Step 1: Preparation of 4-[5-bromo-6-[(1S)-1-methoxyethyl]-3-pyridyl]morpholine (compound G1)

To a mixture of 3-bromo-5-iodo-2-[(1S)-1-methoxyethyl]pyridine (compound **A3,** 30 g, 87.73 mmol) and morpholine (7.6 g, 87.73 mmol) in toluene (450 mL) were added Cs₂CO₃ (57.2 g, 175.45 mmol), (*R*)-binap (2.7 g, 4.39 mmol) and Pd(OAc)₂ (0.98 g, 4.39 mmol). The reaction mixture was degassed and purged with nitrogen for 3 times and the mixture was stirred at 90 °C for 12 hrs under nitrogen atmosphere. After being cooled to room temperature, the reaction mixture was filtered and the filtrate was concentrated *in vacuo* to give a residue. The residue was purified by column chromatography to afford 4-[5-bromo-6-[(1*S*)-1-methoxyethyl]-3-pyridyl]morpholine (compound **G1,** 21 g) as yellow oil. MS calc'd 301.1 (MH⁺), measured 301.1 (MH⁺).

### Step 2: Preparation of 4-[6-[(1S)-1-methoxyethyl]-5-(4,4,5,5-tetramethyl-1,3,2-dioxaborolan-2-yl)-3-pyridyl]morpholine (compound G2)

To a solution of 4-[5-bromo-6-[(1*S*)-1-methoxyethyl]-3-pyridyl]morpholine (compound **G1**, 21 g, 63.3 mmol), bis(pinacolato)diboron (24.0 g, 94.63 mmol) and KOAc (13.6 g, 138.79 mmol) in toluene (500 mL) was added Pd(dppf)Cl₂ (4.4 g, 6.31 mmol). The mixture was degassed and purged with nitrogen for 3 times and the mixture was stirred at 90 °C for 12 hrs under nitrogen atmosphere. After being cooled to the room temperature, the reaction mixture was filtered, the filtrate was concentrated *in vacuo* to give crude product 4-[6-[(1*S*)-1-methoxyethyl]-5-(4,4,5,5-tetramethyl-1,3,2-dioxaborolan-2-yl)-3-pyridyl]morpholine (compound **G2,** 45 g) as a yellow gum, which was used to the next step. MS calc'd 349.2 (MH⁺), measured 349.2 (MH⁺).

### Step 3: Preparation of [3-[5-bromo-6-fluoro-2-[2-[(1S)-1-methoxyethyl]-5-morpholino-3-pyridyl]-1H-indol-3-yl]-2,2-dimethyl-propoxy]-tert-butyl-diphenyl-silane (compound G3)

To a solution of 4-[6-[(1*S*)-1-methoxyethyl]-5-(4,4,5,5-tetramethyl-1,3,2-dioxaborolan-2-yl)-3-pyridyl]morpholine (compound **G2,** 40.6 g, 46.65 mmol), [3-(5-bromo-6-fluoro-2-iodo-1*H*-indol-3-yl)-2,2-dimethyl-propoxy]-*tert*-butyl-diphenyl-silane (compound **C5,** 31 g, 46.65 mmol) in 1,4-dioxane (420 mL) and water (80 mL) was added K₃PO₄ (29.7 g, 2.33 mmol) and Pd(dppf)Cl₂ (1.7 g, 0.29 mmol). The mixture was degassed by bubbling nitrogen for 2 min, and the reaction mixture was stirred at 90 °C for 18 hrs. After being cooled to room temperature, the reaction mixture was extracted with EA (200 mL, three times). The combined organic layer was washed with brine (200 mL), dried over Na₂SO₄, filtered and the filtrate was concentrated *in vacuo* to give a residue. The residue was purified by column chromatography to afford [3-[5-bromo-6-fluoro-2-[2-[(1*S*)-1-methoxyethyl]-5-morpholino-3-pyridyl]-1*H*-indol-3-yl]-2,2-dimethyl-propoxyl-*tert*-butyl-diphenyl-silane (compound **G3,** 17.2 g) as yellow oil. MS calc'd 758.3 (MH⁺), measured 758.3 (MH⁺).

### Step 4: Preparation of [3-[5-bromo-1-ethyl-6-fluoro-2-[2-[(1S)-1-methoxyethyl]-5-morpholino-3-pyridyl]indol-3-yl]-2,2-dimethyl-propoxy]-tert-butyl-diphenyl-silane (compound G4)

To a solution of [3-[5-bromo-6-fluoro-2-[2-[(1*S*)-1-methoxyethyl]-5-morpholino-3-pyridyl]-1*H*-indol-3-yl]-2,2-dimethyl-propoxy]-tert-butyl-diphenyl-silane (compound **G3,** 15 g, 19.77 mmol) in DMF (300 mL) was added Cs₂CO₃ (19.3 g, 59.3 mmol) and iodoethane (6.16 g, 39.53 mmol) at 0 °C. After being stirred at 20°C for 16 hrs, the reaction mixture was poured into water (200 mL), and extracted with EtOAc (200 mL, three times). The combined organic layer was washed with brine (10 mL, three times), dried over Na₂SO₄, filtered and concentrated under vacuum to give a residue. The residue was purified by column chromatography to afford [3-[5-bromo-1-ethyl-6-fluoro-2-[2-[(1*S*)-1-methoxyethyl]-5-morpholino-3-pyridyl]indol-3-yl]-2,2-dimethyl-propoxyl-*tert*-butyl-diphenyl-silane (compound **G4,** 14.7 g) as yellow oil. MS calc'd 786.3 (MH⁺), measured 786.4 (MH⁺).

### Step 5: Preparation of 3-[5-bromo-1-ethyl-6-fluoro-(2M)-2-[2-[(1S)-1-methoxyethyl]-5-morpholino-3-pyridyl]indol-3-yl]-2,2-dimethyl-propan-1-ol (compound G5) and 3-[5-bromo-1-ethyl-6-fluoro-(2P)-2-[2-[(1S)-1-methoxyethyl]-5-morpholino-3-pyridyl]indol-3-yl]-2,2-dimethyl-propan-1-ol (compound G6)

To a solution of [3-[5-bromo-1-ethyl-6-fluoro-2-[2-[(1*S*)-1-methoxyethyl]-5-morpholino-3-pyridyl]indol-3-yl]-2,2-dimethyl-propoxy]-*tert*-butyl-diphenyl-silane (compound **G4,** 14.7 g, 18.68 mmol) in DMF (160 mL) was added cesium fluoride (14.2 g, 93.41 mmol). The mixture was stirred at 60 °C for 48 hrs. After being cooled to room temperature, the reaction mixture were added with EtOAc (300 mL) and water (300 mL) and the layers were separated. The aqueous phase was extracted with EtOAc (200 mL, three times). The combined organic layer was washed with brine (200 mL, four times), dried over Na₂SO₄, filtered, and concentrated under vacuum to give a residue. The residue was purified by column chromatography to afford 3-[5-bromo-1-ethyl-6-fluoro-(2*M*)-2-[2-[(1S)-1-methoxyethyl]-5-morpholino-3-pyridyl]indol-3-yl]-2,2-dimethyl-propan-1-ol (compound **G5,** 6 g, faster eluted) as colorless foam and 3-[5-bromo-1-ethyl-6-fluoro-(2*P*)-2-[2-[(1*S*)-1-methoxyethyl]-5-morpholino-3-pyridyl]indol-3-yl]-2,2-dimethyl-propan-1-ol (compound **G6,** 4.5 g, slower eluted) as colorless foam. Compound **G5:** MS calc'd 548.2 (MH⁺), measured 548.2 (MH⁺). ¹H NMR (400MHz, Methanol-*d*₄) *δ* = 8.41 (d, *J* = 2.4 Hz, 1H), 7.92 (d, *J =* 6.8 Hz, 1H), 7.37 - 7.33 (m, 2H), 4.58 (s, 1H), 4.05 - 3.98 (m, 2H), 3.87-3.82 (m, 5H), 3.27 - 3.23 (m, 4H), 3.15 - 3.13 (m, 1H), 3.00 (s, 3H), 2.75-2.71 (m, 1H), 2.24 - 2.22 (m, 1H), 1.42 (d, *J* = 6.4 Hz, 3H), 1.22 (t, *J* = 7.2 Hz, 3H), 0.76 (s, 3H), 0.76 (s, 3H).

### X-ray crystallographic analysis of compound G5

Absolute configuration structure of compound **G5** was confirmed by X-ray crystallographic analysis of its single crystal. (Figure 1).

### Step 6: Preparation of 3-[1-ethyl-6-fluoro-(2M)-2-[2-[(1S)-1-methoxyethyl]-5-morpholino-3-pyridyl]-5-(4,4,5,5-tetramethyl-1,3,2-dioxaborolan-2-yl)indol-3-yl]-2,2-dimethyl-propan-1-ol (compound G7)

To a solution of 3-[5-bromo-1-ethyl-6-fluoro-(2*M*)-2-[2-[(1*S*)-1-methoxyethyl]-5-morpholino-3-pyridyl]indol-3-yl]-2,2-dimethyl-propan-1-ol (compound **G5,** 6 g, 10.94 mmol), bis(pinacolato)diboron (4.2 g, 16.41 mmol) in toluene (60 mL) was added potassium acetate (2.7 g, 27.35 mmol) and Pd(dppf)Cl₂ (0.8 g, 1.09 mmol). The reaction mixture was degassed by bubbling nitrogen for 5 min then stirred at 90 °C for 15 hrs. After being cooled to room temperature, the reaction mixture was filtered and the filtrate was concentrated *in vacuo* to give a residue. The residue was purified by column chromatography to afford 3-[1-ethyl-6-fluoro-(2*M*)-2-[2-[(1*S*)-1-methoxyethyl]-5-morpholino-3-pyridyl]-5-(4,4,5,5-tetramethyl-1,3,2-dioxaborolan-2-yl)indol-3-yl]-2,2-dimethyl-propan-1-ol (compound **G7,** 4.5 g) as colorless gum. MS calc'd 596.4 (MH⁺), measured 596.4 (MH⁺).

### Step 7: Preparation of methyl (3S)-1-[(2S)-2-(tert-butoxycarbonylamino)-3-[4-[1-ethyl-6-fluoro-3-(3-hydroxy-2,2-dimethyl-propyl)-(2M)-2-[2-[(1S)-1-methoxyethyl]-5-morpholino-3-pyridyl]indol-5-yl]thiazol-2-yl]propanoyl]hexahydropyridazine-3-carboxylate (compound G8)

To a mixture of 3-[1-ethyl-6-fluoro-(2*M*)-2-[2-[(1*S*)-1-methoxyethyl]-5-morpholino-3-pyridyl]-5-(4,4,5,5-tetramethyl-1,3,2-dioxaborolan-2-yl)indol-3-yl]-2,2-dimethyl-propan-1-ol (compound **G7,** 4.5 g, 7.56 mmol) and methyl (3*S*)-1-[(2*S*)-3-(4-bromothiazol-2-yl)-2-(tert-butoxycarbonylamino)propanoyllhexahydropyridazine-3-carboxylate (intermediate **B,** 3.6 g, 7.56 mmol) in toluene (45 mL), 1,4-dioxane (15 mL) and water (15 mL) were added K₃PO₄ (4.0 g, 18.89 mmol) and Pd(dtbpf)Cl₂ (492.5 mg, 0.75 mmol). The mixture was stirred at 70 °C for 12 hrs under nitrogen atmosphere. After being cooled to room temperature, the reaction mixture was filtered and the filtrate was concentrated *in vacuo* to give a residue. The residue was purified by column chromatography to afford methyl (3*S*)-1-[(2*S*)-2-(tert-butoxycarbonylamino)-3-[4-[1-ethyl-6-fluoro-3-(3-hydroxy-2,2-dimethyl-propyl)-(2*M*)-2-[2-[(1*S*)-1-methoxyethyl]-5-morpholino-3-pyridyl]indol-5-yl]thiazol-2-yl]propanoyl]hexahydropyridazine-3-carboxylate (compound **G8,** 3.8 g) as colorless gum. MS calc'd 866.4 (MH⁺), measured 866.4 (MH⁺).

### Step 8: Preparation of (3S)-1-[(2S)-2-(tert-butoxycarbonylamino)-3-[4-[1-ethyl-6-fluoro-3-(3-hydroxy-2,2-dimethyl-propyl)-(2M)-2-[2-[(1S)-1-methoxyethyl]-5-morpholino-3-pyridyl]indol-5-yl]thiazol-2-yl]propanoyl]hexahydropyridazine-3-carboxylic acid (compound G9)

To a mixture of methyl (3*S*)-1-[(2*S*)-2-(tert-butoxycarbonylamino)-3-[4-[1-ethyl-6-fluoro-3-(3-hydroxy-2,2-dimethyl-propyl)-(2*M*)-2-[2-[(1*S*)-1-methoxyethyl]-5-morpholino-3-pyridyl]indol-5-yl]thiazol-2-yl]propanoyl]hexahydropyridazine-3-carboxylate (compound **G8,** 3.8 g, 4.39 mmol) in DCE (76 mL) was added Me₃SnOH (3.2 g, 17.55 mmol). The mixture was stirred at 60 °C for 48 hrs. The reaction mixture was concentrated under vacuum to give a residue. EtOAc (200 mL) and water (100 mL) were added to the residue and the layers were separated. The aqueous phase was extracted with EtOAc (150 mL, twice). The combined organic layer was washed with brine (200 mL), dried over Na₂SO₄, filtered, and concentrated under vacuum to afford (3*S*)-1-[(2*S*)-2-(*tert*-butoxycarbonylamino)-3-[4-1-ethyl-6-fluoro-3-(3-hydroxy-2,2-dimethyl-propyl)-(2*M*)-2-[2-[(1*S*)-1-methoxyethyl]-5-morpholino-3-pyridyl]indol-5-yl]thiazol-2-yl]propanoyl]hexahydropyridazine-3-carboxylic acid (compound **G9,** 3.7 g) as a brown solid. MS calc'd 852.4 (MH⁺), measured 852.4 (MH⁺).

### Step 9: Preparation of tert-butyl N-[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound G10)

To a mixture of (3*S*)-1-[(2*S*)-2-(*tert*-butoxycarbonylamino)-3-[4-[1-ethyl-6-fluoro-3-(3-hydroxy-2,2-dimethyl-propyl)-(2*M*)-2-[2-[(1*S*)-1-methoxyethyl]-5-morpholino-3-pyridyl]indol-5-yl] thiazol-2-yl]propanoyl]hexahydropyridazine-3-carboxylic acid (compound **G9,** 2.5 g, 2.93 mmol) in DCM (250 mL) were added DIEA (7.58 mL, 58.68 mmol), EDCI (8.4 g, 44.01 mmol) and HOBt (991.2 mg, 0.91 mmol) at 0 °C. After being stirred at 20 °C for 12 hrs, the reaction mixture was poured into water (100 mL) and extracted with EtOAc (100 mL, three times). The combined organic layer was washed with brine (30 mL), dried over Na₂SO₄, filtered and concentrated under vacuum to give a residue which was purified by column chromatography to afford *tert*-butyl *N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound **G10,** 1.2 g) as a yellow oil. MS calc'd 834.4 (MH⁺), measured 834.4 (MH⁺).

### Step 10: Preparation of (7S,13S)-7-amino-21-ethyl-24-fluoro-(20M)-20-[2-[(1S)-1-methoxyethyl]-5-morpholino-3-pyridyl]-17,17-dimethyl-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate G)

To a solution of *tert*-butyl *N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamate (compound **G10,** 1.2 g, 1.44 mmol) in DCM (12 mL) was added TFA (6.0 mL). The mixture was stirred at 20 °C for 3 hrs. After the reaction was completed, the reaction mixture was concentrated under vacuum to give a residue. Sat. NaHCO₃ aq. (60 mL) was added and the mixture was extracted with EtOAc (80 mL, three times). The combined organic layer was washed with brine (100 mL), dried over anhydrous sodium sulfate, filtered and concentrated *in vacuo* to afford (7*S*,13*S*)-7-amino-21-ethyl-24-fluoro-(20*M*)-20-[2-[(1*S*)-1-methoxyethyl]-5-morpholino-3-pyridyl]-17,17-dimethyl- 15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **G,** 1 g) as a yellow solid. MS calc'd 734.3 (MH⁺), measured 734.3 (MH⁺).

### Intermediate H

### (7S,13S)-7-amino-25-fluoro-(20M)-20-[2-[(1S)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

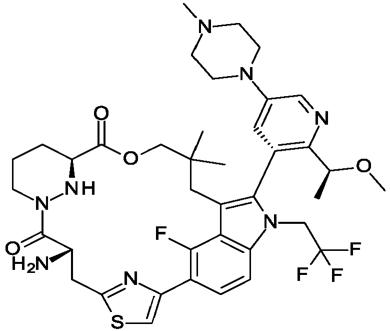

The title compound was prepared in analogy to the preparation of Intermediate **C** by using 5-bromo-4-fluoro-1*H*-indole instead of 5-bromo-6-fluoro-1*H*-indole (compound **C2**).

### Intermediate I

### (7S,13S)-7-amino-24-fluoro-(20M)-20-[2-[(1S)-1-methoxyethyl]-5-morpholino-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

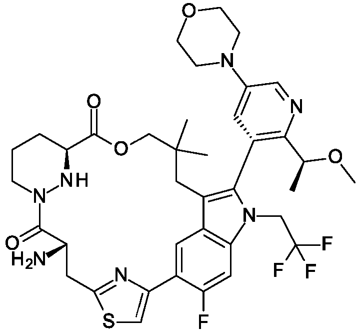

The title compound was prepared in analogy to the preparation of Intermediate **E** by using morpholine instead of 1-(2,2,2-trifluoroethyl)piperazine (compound **E1**).

### Intermediate J

### (7S,13S)-7-amino-25-fluoro-(20M)-20-[2-[(1S)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione

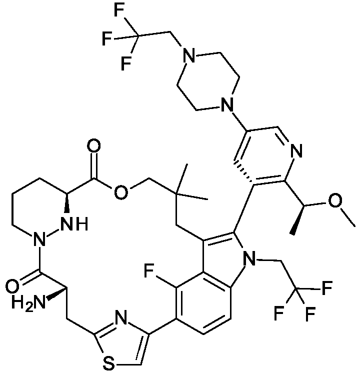

The title compound was prepared in analogy to the preparation of Intermediate **E** by using [3-(5-bromo-4-fluoro-2-iodo-1*H*-indol-3-yl)-2,2-dimethyl-propoxy]-*tert*-butyl-diphenyl-silane (compound **J1**) instead of [3-(5-bromo-6-fluoro-2-iodo-1*H*-indol-3-yl)-2,2-dimethyl-propoxy]-*tert*-butyl-diphenyl-silane (compound **C5**).

The compound **J1** was prepared in analogy to the preparation of compound **C5** by using 5-bromo-4-fluoro-1H-indole instead of 5-bromo-6-fluoro-1*H*-indole (compound **C2**).

### Example 1

### 3-(dimethylamino)-N-[(1S)-1-[[(7S,13S)-24-fluoro-(20M)-20-[2-[(1S)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-azetidine-1-carboxamide

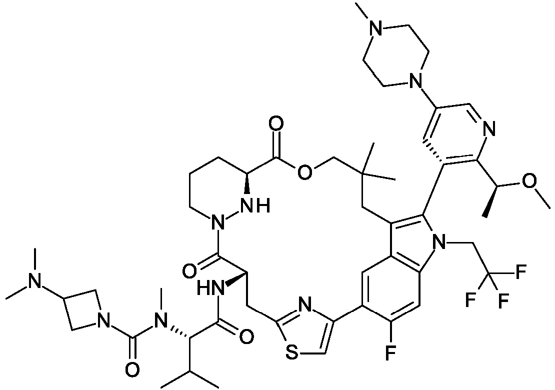

The compound was prepared according to the following scheme:

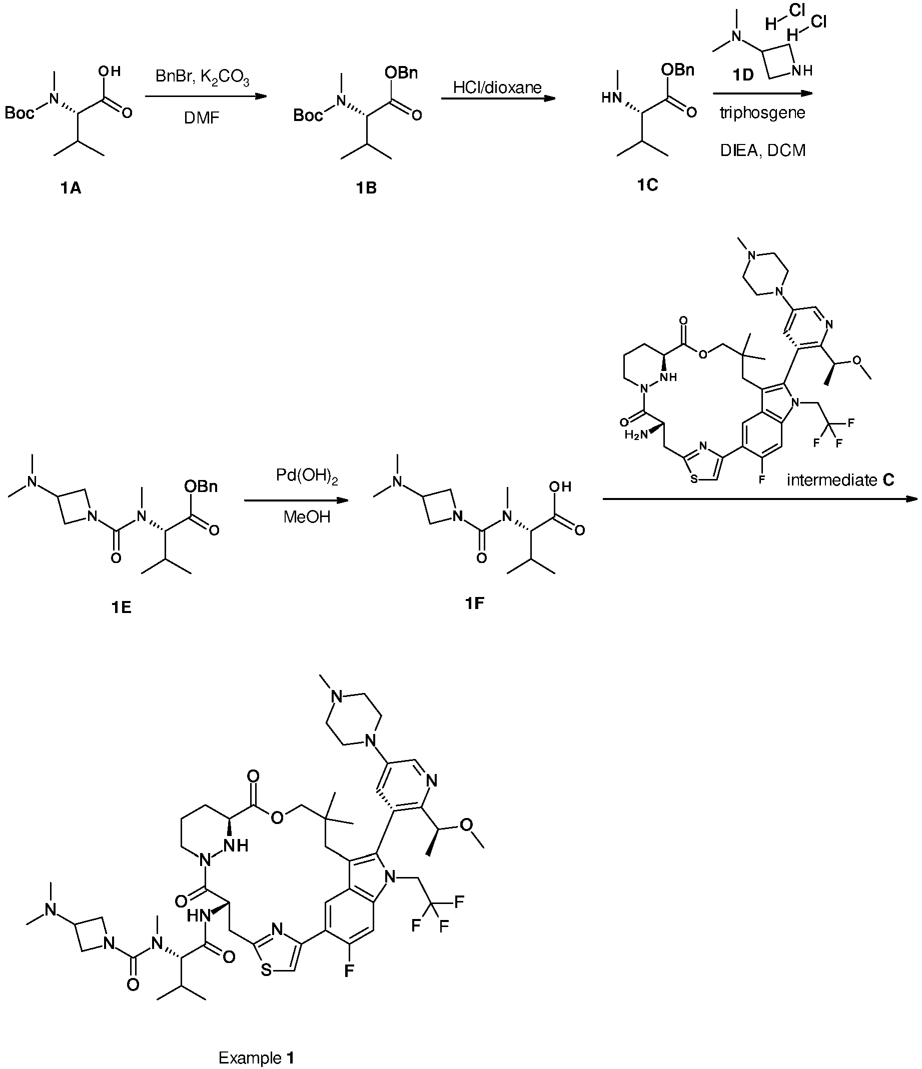

### Step 1: Preparation of benzyl (2S)-2-[tert-butoxycarbonyl(methyl)amino]-3-methylbutanoate (compound 1B)

To a mixture of BOC-*N*-ME-VAL-OH (5.0 g, 21.62 mmol) and potassium carbonate (4.5 g, 32.43 mmol) in DMF (70 mL) was added benzyl bromide (4.1 g, 23.78 mmol) at 0 °C. The mixture was stirred at 25 °C for 12 hrs. After the reaction was completed, the reaction mixture was diluted with water (70 mL), extracted with EtOAc (70 mL, twice). The combined organic layer was washed with brine (200 mL), dried over Na₂SO₄, filtered and concentrated *in vacuo.* The residue was purified by reversed phase column to afford benzyl (2*S*)-2-[*tert-*butoxycarbonyl(methyl)amino]-3-methyl-butanoate (compound **1B,** 6.7 g) as yellow oil. ¹H NMR (400 MHz, CHLOROFORM-*d*) δ = 7.34 (br s, 5H), 5.21 - 5.10 (m, 2H), 2.88 - 2.74 (m, 3H), 2.31 - 2.12 (m, 1H), 1.68 - 1.64 (m, 1H), 1.44 (br d, *J =* 15.0 Hz, 9H), 0.96 (d, *J =* 6.6 Hz, 3H), 0.90 (br d, *J* = 3.8 Hz, 3H). MS calc'd 322.2 (MH⁺), measured 344.2 (MNa⁺).

### Step 2: Preparation of benzyl (2S)-3-methyl-2-(methylamino)butanoate (compound 1C)

A mixture of benzyl (2*S*)-2-[*tert*-butoxycarbonyl(methyl)amino]-3-methyl-butanoate (compound **1B,** 6.7 g, 21.47 mmol) in HCl/1,4-dioxane (50.0 mL, 2 M) was stirred at 25 °C for 1 h. After the reaction was completed, the reaction mixture was concentrated *in vacuo* to get a residue. The residue was diluted with sat. NaHCO₃ (40 mL), extracted with EtOAc (50 mL, three times). The combined organic layer was washed with brine (100 mL), dried over Na₂SO₄, filtered and concentrated *in vacuo* to afford benzyl (2S)-3-methyl-2-(methylamino)butanoate (compound **1C,** 4.7 g) as colorless oil. MS calc'd 222.1 (MH⁺), measured 222.2 (MH⁺).

### Step 3: Preparation of benzyl (2S)-2-[[3-(dimethylamino)azetidine-1-carbonyl]-methyl-amino]-3-methyl-butanoat (compound 1D)

To a mixture of benzyl (2S)-3-methyl-2-(methylamino)butanoate (compound **1C,** 1.0 g, 4.52 mmol) and DIEA (0.9 mL, 4.97 mmol) in DCM (15 mL) was added triphosgene (1.3 g, 4.52 mmol) at 0 °C. After being stirred for 0.5 h, the reaction was added with a solution of *N,N-*dimethylazetidin-3-amine dihydrochloride (compound **1D,** 782.1 mg, 4.52 mmol) and DIEA (1.97 mL, 11.3 mmol) in DCM (4 mL). The reaction mixture was stirred at 20 °C for another 1 h. After the reaction was completed, the reaction mixture was quenched with water (40 mL), extracted with EtOAc (40 mL, twice). The combined organic layer was washed with brine (60 mL), dried over Na₂SO₄, filtered and concentrated *in vacuo* to get a residue. The residue was purified by prep-HPLC to afford benzyl (2S)-2-[[3-(dimethylamino)azetidine-1-carbonyl]-methyl-amino]-3-methyl-butanoat (compound **1E**, 1.0 g) as yellow oil. MS calc'd 348.2 (MH⁺), measured 348.2 (MH⁺).

### Step 4: Preparation of (2S)-2-[[3-(dimethylamino)azetidine-1-carbonyl]-methyl-amino]-3-methyl-butanoic acid (compound 1F)

To a mixture of benzyl (2*S*)-2-[[3-(dimethylamino)azetidine-1-carbonyl]-methyl-amino]-3-methyl-butanoat (compound **1E,** 500.0 mg, 1.44 mmol) in methanol (15 mL) was added Pd(OH)₂ on activated carbon (400.0 mg, 0.57 mmol). The reaction mixture was degassed and purged into hydrogen for three times and stirred at 20 °C for 12 hrs under hydrogen atmosphere (15 psi). After the reaction was completed, the reaction mixture was filtered and the filtrate was concentrated *in vacuo* to afford (2*S*)-2-[[3-(dimethylamino)azetidine-1-carbonyl]-methyl-amino]-3-methyl-butanoic acid (compound **1F,** 370.0 mg) as yellow oil. MS calc'd 258.1 (MH⁺), measured 258.3 (MH⁺).

### Step 5: Preparation of 3-(dimethylamino)-N-[(1S)-1-[[(7S,13S)-24-fluoro-(20M)-20-[2-[(1S)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-azetidine-1-carboxamide (Example 1)

To a solution of (2*S*)-2-[[3-(dimethylamino)azetidine-1-carbonyl]-methyl-amino]-3-methyl-butanoic acid (compound **1F,** 32.13 mg, 0.12 mmol) in DMF (2 mL) were added DIEA (0.1 mL, 0.25 mmol), HATU (47.47 mg, 0.12 mmol) and (7S,13S)-7-amino-24-fluoro-17,17-dimethyl-20-[5-(4-methylpiperazin-1-yl)-2-[rac-(1S)-1-methoxyethyl]-3-pyridyl]-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (intermediate C, 50.0 mg, 0.06 mmol) at 0 °C. After being stirred at 20 °C for 1 h, the reaction mixture was diluted with water (15 mL), extracted with EtOAc (15 mL, three times). The combined organic layer was washed with brine (20 mL), dried over Na₂SO₄, filtered and concentrated *in vacuo* to get a residue. The residue was purified by prep-HPLC to afford Example **1** (35.1 mg) as a white solid. MS calc'd 1040.5 (MH⁺), measured 1040.8 (MH⁺), ¹H NMR (400 MHz, Methanol-*d*₄) δ = 8.68 (d, *J =* 7.6 Hz, 1H), 8.51 (d, *J =* 2.8 Hz, 1H), 7.69 (d, *J =* 2.4 Hz, 1H), 7.57 - 7.44 (m, 2H), 5.79 - 5.69 (m, 1H), 5.25 - 5.11 (m, 1H), 4.97 - 4.89 (m, 2H), 4.51 - 4.39 (m, 2H), 4.31 - 4.12 (m, 7H), 4.12 - 3.88 (m, 2H), 3.87 - 3.67 (m, 3H), 3.67 - 3.52 (m, 2H), 3.47 (d, *J =* 14.8 Hz, 2H), 3.35 (s, 3H), 3.29 - 3.22 (m, 2H), 3.22 - 3.10 (m, 2H), 2.99 (s, 3H), 2.92 (s, 8H), 2.87 - 2.77 (m, 1H), 2.58 (d, *J =* 14.4 Hz, 1H), 2.30 - 2.16 (m, 2H), 2.03 - 1.90 (m, 1H), 1.88 - 1.74 (m, 1H), 1.72 - 1.58 (m, 1H), 1.45 (d, *J =* 6.0 Hz, 3H), 1.03 - 0.89 (m, 9H), 0.45 (s, 3H).

### Example 2

### 3-(dimethylamino)-N-[(1S)-1-[[(7S,13S)-25-fluoro-(20M)-20-[2-[(1S)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-azetidine-1-carboxamide

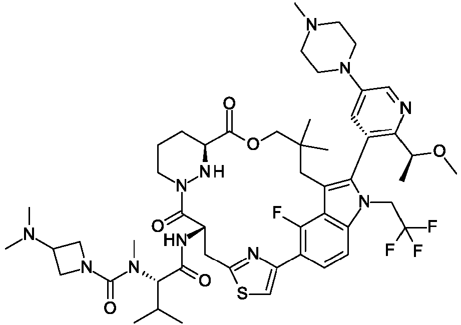

The title compound was prepared in analogy to the preparation of Example **1** by using (7*S*,13*S*)-7-amino-25-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.19,13.0^{22,26}]octacosa-1(25),2,5(28),19,22(26),23-hexaene-8,14-dione (intermediate **H**) instead of (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **2** (50.0 mg) was obtained as a yellow solid. MS calc'd 1040.5 (MH⁺), measured 1040.8 (MH⁺). ¹H NMR (400MHz, Methanol-*d*₄) *δ* = 8.55 - 8.49 (m, 1H), 7.63 - 7.56 (m, 1H), 7.52 - 7.39 (m, 3H), 6.03 - 5.81 (m, 1H), 5.20 - 5.01 (m, 1H), 4.70 - 4.61 (m, 1H), 4.45 - 4.36 (m, 2H), 4.30 - 4.23 (m, 2H), 4.17 - 3.93 (m, 7H), 3.77 - 3.63 (m, 3H), 3.56 - 3.48 (m, 2H), 3.42 - 3.34 (m, 2H), 3.29 - 3.21 (m, 3H), 3.20 - 3.11 (m, 3H), 3.00 - 2.98 (m, 3H), 2.94 - 2.87 (m, 10H), 2.68 - 2.51 (m, 2H), 2.23 - 2.14 (m, 1H), 1.68 - 1.53 (m, 1H), 1.47 - 1.41 (m, 3H), 1.35 - 1.15 (m, 3H), 0.95 - 0.88 (m, 6H), 0.81 (s, 3H), 0.72 - 0.58 (m, 3H).

### Example 3

### N-[(1S)-1-[[(7S,13S)-24-fluoro-(20M)-20-[2-[(1S)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

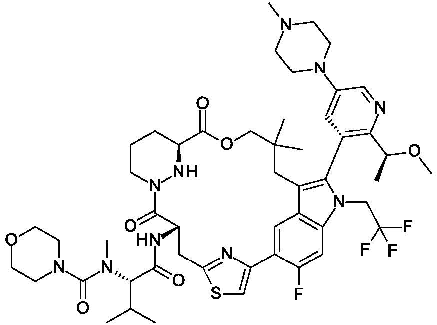

The title compound was prepared in analogy to the preparation of Example **1** by using morpholine instead of *N,N*-dimethylazetidin-3-amine dihydrochloride (compound 1**D**). Example **3** (25 mg) was obtained as an off-white solid. MS calc'd 1027.4 (MH⁺), measured 1027.7 (MH⁺), ¹H NMR (400 MHz, Methanol-*d*₄) *δ* = 8.71 (d, *J* = 7.6 Hz, 1H), 8.49 (d, *J* = 2.8 Hz, 1H), 7.72 - 7.67 (m, 2H), 7.48 (d, *J =* 12.8 Hz, 1H), 5.76 (d, *J* = 8.8 Hz, 1H), 5.26 - 5.13 (m, 1H), 4.84 - 4.75 (m, 1H), 4.43 (d, *J* = 12 Hz, 1H), 4.30 - 4.24 (m, 1H), 4.20 (dd, *J* = 12, 2.8 Hz, 1H), 4.17 - 4.03 (m, 2H), 4.00 (s, 1H), 3.85 - 3.62 (m, 8H), 3.55 - 3.44 (m, 2H), 3.42 - 3.34 (m, 6H), 3.30 - 3.18 (m, 5H), 3.15 - 3.09 (m, 1H), 2.99 (s, 3H), 2.91 (s, 3H), 2.87 - 2.77 (m, 1H), 2.71 - 2.58 (m, 1H), 2.33 - 2.14 (m, 2H), 2.01 - 1.90 (m, 1H), 1.87 - 1.74 (m, 1H), 170 - 1.57 (m, 1H), 1.46 (d, *J =* 6.0 Hz, 3H), 1.01 - 0.87 (m, 9H), 0.50 (s, 3H).

### Example 4

### 3-(dimethylamino)-N-[(1S)-1-[[(7S,13S)-24-fluoro-(20M)-20-[2-[(1S)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.^{19,13}.0^{22,26}]octacosa-1(25),2,5(28),19,22(26),23-hexaen-7-yl]carbamoyl]-2-methyl-propyl]-N-methyl-azetidine-1-carboxamide

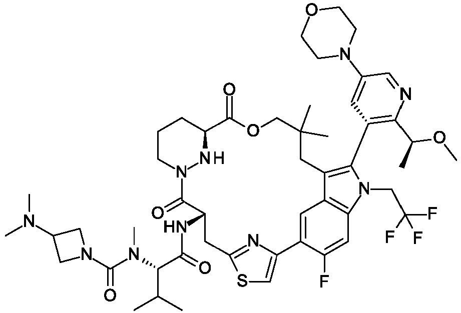

The title compound was prepared in analogy to the preparation of Example **1** by using (7*S*,13*S*)-7-amino-24-fluoro-(20*M*)-20-[2-[(1*S*)-1-methoxyethyl]-5-morpholino-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **I**) instead of (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **4** (12.9 mg) was obtained as a yellow solid. MS calc'd 1027.5 (MH⁺), measured 1027.5 (MH⁺). ¹H NMR (400MHz, Methanol-*d*₄) *δ* = 8.69 (d, *J =* 7.8 Hz, 1H), 8.42 (d, *J* = 2.9 Hz, 1H), 7.69 (d, *J* = 2.4 Hz, 1H), 7.55 (d, *J* = 2.4 Hz, 1H), 7.48 (d, *J* = 12.7 Hz, 1H), 5.74 (br d, *J =* 9.3 Hz, 1H), 5.18 (br dd, *J* = 8.3, 16.6 Hz, 1H), 4.87 - 4.79 (m, 2H), 4.49 - 4.38 (m, 2H), 4.33 - 4.11 (m, 7H), 3.87 (t, *J =* 4.6 Hz, 4H), 3.78 (br d, *J =* 11.2 Hz, 1H), 3.72 - 3.66 (m, 1H), 3.48 (br d, *J =* 14.7 Hz, 1H), 3.39 - 3.33 (m, 6H), 3.26 - 3.21 (m, 1H), 3.15 - 3.10 (m, 1H), 2.97 - 2.90 (m, 8H), 2.89 - 2.76 (m, 2H), 2.66 - 2.60 (m, 1H), 2.26 - 2.17 (m, 2H), 2.01 - 1.91 (m, 1H), 1.87 - 1.78 (m, 1H), 1.70 - 1.60 (m, 1H), 1.49 - 1.41 (m, 3H), 1.01 - 0.84 (m, 9H), 0.48 (s, 3H).

### Example 5

### N-[(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

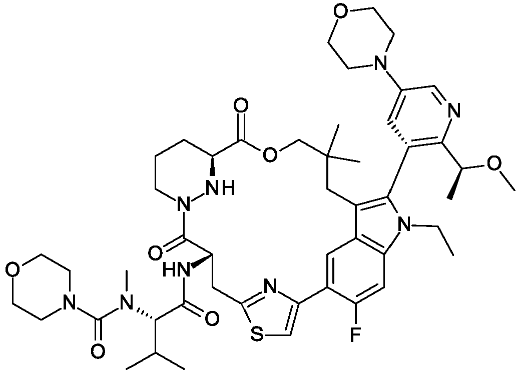

The title compound was prepared in analogy to the preparation of Example **1** by using morpholine and (7*S*,13*S*)-7-amino-21-ethyl-24-fluoro-(20*M*)-20-[2-[(1*S*)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione (Intermediate **G**) instead of *N,N*-dimethylazetidin-3-amine dihydrochloride (compound **1D**) and (7S,13S)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **5** (131.3 mg) was obtained as a yellow solid. MS calc'd 960.5 (MH⁺), measured 960.3 (MH⁺). ¹H NMR (400 MHz, CHLOROFORM *-d*) *δ* = 8.97 (br d, *J =* 1.6 Hz, 1H), 8.69 (d, *J =* 7.5 Hz, 1H), 8.14 - 7.98 (m, 1H), 7.61 (d, *J* = 1.7 Hz, 1H), 7.38 (d, *J =* 1.5 Hz, 1H), 7.13 (d, *J* = 12.5 Hz, 1H), 5.81 (br d, *J* = 17.7 Hz, 1H), 4.68 - 4.57 (m, 1H), 4.41 (d, *J* = 6.5 Hz, 1H), 4.29 - 4.22 (m, 1H), 4.19 - 4.07 (m, 2H), 4.00 - 3.88 (m, 6H), 3.84 - 3.78 (m, 2H), 3.77 - 3.70 (m, 3H), 3.49 - 3.42 (m, 6H), 3.39 - 3.31 (m, 6H), 3.23 - 3.14 (m, 2H), 2.91 (s, 3H), 2.77 - 2.68 (m, 1H), 2.49 - 2.42 (m, 1H), 2.38 - 2.28 (m, 2H), 2.02 - 1.96 (m, 2H), 1.82 (br d, *J =* 12.7 Hz, 1H), 1.69 - 1.60 (m, 1H), 1.57 (d, *J =* 6.2 Hz, 3H), 1.03 - 0.96 (m, 9H), 0.91 (d, *J =* 6.6 Hz, 3H), 0.55 - 0.43 (m, 3H).

### Example 6

### N-[(1S)-1-[[(7S,13S)-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5},1^{9,13}.0^{22,26}]octacosa-1(25),2,5(28),19,22(26),23-hexaen-7-yl]carbamoyl]-2-methyl-propyl]-N-methyl-morpholine-4-carboxamide

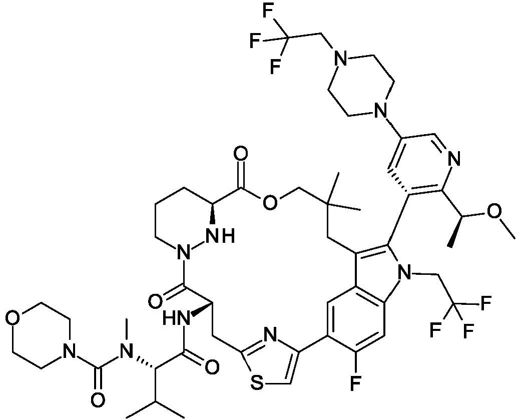

The title compound was prepared in analogy to the preparation of Example **1** by using morpholine and (7*S*,13*S*)-7-amino-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **E**) instead of *N,N*-dimethylazetidin-3-amine dihydrochloride (compound 1**D**) and (7*S*,13*S*)-7-amino-24-fluoro-(20*M*)-20-[2-[(1*S*)-1-methoxyethyl]-5-(4-methylpiperazin-1-yl)-3-pyridyl]-17, 17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **6** (8.8 mg) was obtained as an off-white solid. MS calc'd 1095.5 (MH⁺), measured 1095.5 (MH⁺). ¹H NMR (400 MHz, Methanol-*d*₄) δ = 8.71 (d, *J =* 7.2 Hz, 1H), 8.45 - 8.38 (m, 1H), 7.69 (d, *J =* 2.0 Hz, 1H), 7.63 - 7.56 (m, 1H), 7.48 (d, *J =* 12.8 Hz, 1H), 5.87 - 5.69 (m, 1H), 5.24 - 5.11 (m, 1H), 4.49 - 4.37 (m, 1H), 4.29 - 4.16 (m, 2H), 4.01 (d, *J* = 11.2 Hz, 1H), 3.83 - 3.57 (m, 7H), 3.50 - 3.36 (m, 10H), 3.26 - 3.06 (m, 5H), 2.93 - 2.77 (m, 8H), 2.71 - 2.60 (m, 1H), 2.31 - 2.16 (m, 2H), 2.00 - 1.91 (m, 1H), 1.87 - 1.75 (m, 1H), 1.70 - 1.58 (m, 1H), 1.46 (d, *J =* 6.0 Hz, 3H), 1.34 - 1.26 (m, 1H), 1.00 - 0.88 (m, 9H), 0.50 (s, 3H).

### Example 7

### N-[(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3,3-difluoro-N-methyl-azetidine-1-carboxamide

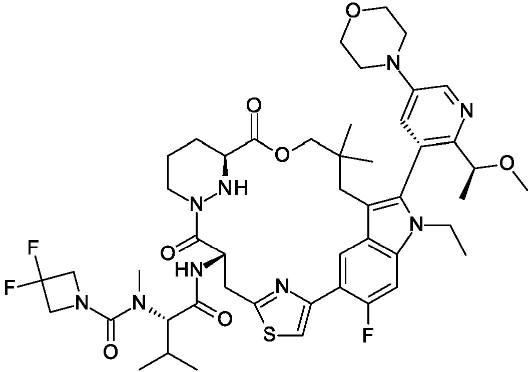

The compound was prepared according to the following scheme:

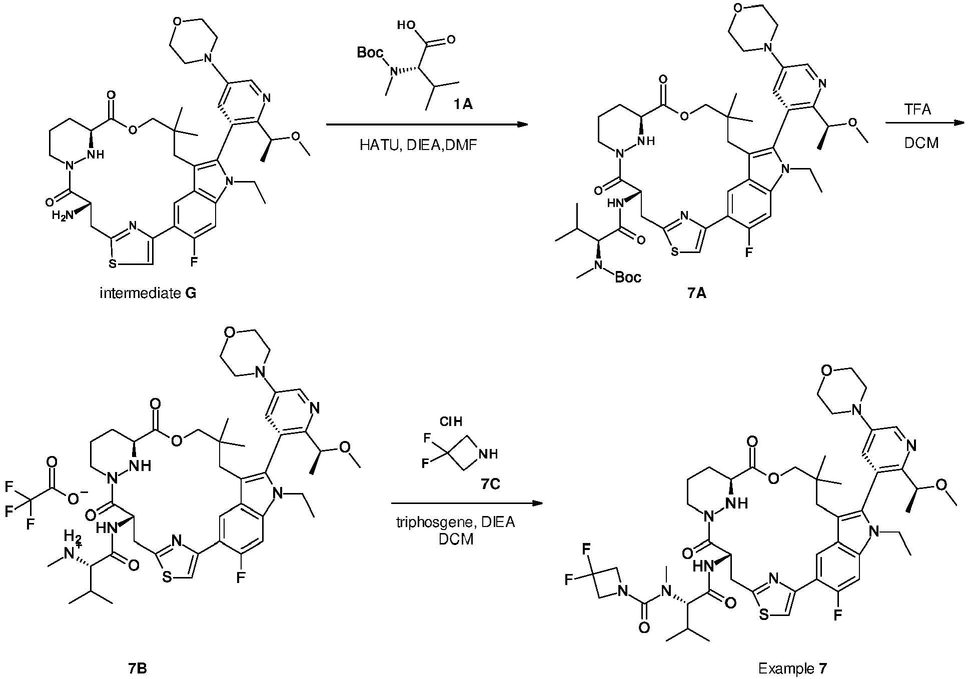

### Step 1: tert-butyl N-[(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-carbamate (compound 7A)

To a solution of BOC-N-ME-VAL-OH (**compound 1A,** 66.2 mg, 0.29 mmol) in DMF (1 mL) were added DIEA (0.3 mL, 1.90 mmol) and HATU (67.3 mg, 0.29 mmol) at 0 °C. After being stirred at 0 °C for 15 min, the reaction mixture was added with (7*S*,13*S*)-7-amino-21-ethyl-24-fluoro-(20*M*)-20-[2-[(1*S*)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione (intermediate **G,** 70.0 mg, 0.09 mmol). After the reaction was stirred at 20 °C for 0.5 h, the reaction mixture was poured into water (10 mL) and extracted with ethyl acetate (10 mL, three times). The combined organic layer was washed with brine (10 mL), and dried over Na₂SO₄, filtered and concentrated under vacuum to give a residue. The residue was purified by prep-TLC to afford *tert*-butyl *N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-carbamate (compound **7A,** 65.1 mg) as a light yellow solid. MS calc'd 947.5 (MH⁺), measured 947.4 (MH⁺).

### Step 2: [(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-methyl-ammonium;2,2,2-trifluoroacetate (compound 7B)

To the mixture solution of *tert*-butyl *N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-carbamate (compound **7A,** 40.0 mg, 0.04 mmol) in DCM (1 mL) was added TFA (0.2 mL, 2.7 mmol) at 20 °C. After being stirred at 20 °C for 1 h, the reaction mixture was concentrated *in vacuo* to get a residue. The residue was purified prep-HPLC to afford [(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-methyl-ammonium;2,2,2-trifluoroacetate (compound **7B,** 20.0 mg) as a white solid. MS calc'd 847.5 (MH⁺), measured 847.4 (MH⁺). ¹H NMR (400 MHz, Methanol-*d*₄) δ = 8.68 (d, *J =* 7.8 Hz, 1H), 8.40 (d, *J =* 2.8 Hz, 1H), 7.65 (d, *J =* 2.4 Hz, 1H), 7.56 (br s, 1H), 7.35 (d, *J =* 12.6 Hz, 1H), 5.95 - 5.92 (m, 1H), 5.04 - 4.96 (m, 1H), 4.94 - 4.91 (m, 3H), 4.45 - 4.40 (m, 1H), 4.35 - 4.05 (m, 4H), 3.88 - 3.83 (m, 4H), 3.80 - 3.69 (m, 3H), 3.56 - 3.50 (d, *J =* 14.5 Hz, 1H), 3.35 - 3.31 (m, 4H), 3.07 - 2.97 (m, 1H), 2.90 - 2.80 (m, 1H), 2.75 - 2.65 (m, 4H), 2.30 - 2.15 (m, 2H), 2.03 - 1.91 (m, 1H),1.89 - 1.75 (m, 1H), 1.73 - 1.56 (m, 1H), 1.45 - 1.43 (m, 3H), 1.15 - 1.09 (m, 3H), 1.09 - 1.07 (m, 3H), 1.03 - 1.01 (m, 3H), 0.95 (s, 3H), 0.56 (s, 3H).

### Step 3: N-[(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3,3-difluoro-N-methyl-azetidine-1-carboxamide (Example 7)

To a solution of [(1*S*)-1-[[(7*S*,13*S*)-21-ethyl-24-fluoro-(20*M*)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-methyl-ammonium;2,2,2-trifluoroacetate (compound **7B,** 50.0 mg, 0.06 mmol) and DIEA (45.8 mg, 0.35 mmol) in DCM (1 mL) was added triphosgene (6.1 mg, 0.02 mmol) at 0 °C. After being stirred at 20 °C for 1 h, the reaction mixture was added with 3,3-difluoroazetidine hydrochloride (compound **7C,** 7.6 mg, 0.06 mmol) at 0 °C and stirred at 20 °C for 11 hrs. After the reaction was completed, the reaction mixture was poured into H₂O (10 mL), extracted with EtOAc (10 mL, three times). The organic layer was washed with brine (10 mL, twice), dried over Na₂SO₄, filtered and concentrated under vacuum to get a residue. The residue was purified by prep-HPLC to afford Example **7** (13.0 mg) as a light yellow solid. MS calc'd 966.5 (MH⁺), measured 966.4 (MH⁺). ¹H NMR (400 MHz, Methanol-*d*₄) δ = 8.70 - 8.64 (m, 1H), 8.49 - 8.35 (m, 1H), 7.62 (d, *J =* 2.2 Hz, 1H), 7.51 (d, *J =* 2.4 Hz, 1H), 7.33 (d, *J =* 12.8 Hz, 1H), 5.78 (br t, *J =* 8.2 Hz, 1H), 5.02 - 4.96 (m, 1H), 4.68 - 4.52 (m, 1H), 4.46 - 4.43 (m, 1H), 4.42 - 4.32 (m, 4H), 4.22 - 4.05 (m, 3H), 3.93 - 3.82 (m, 4H), 3.81 - 3.66 (m, 2H), 3.50 - 3.42 (m, 1H), 3.35 - 3.31 (m, 6H), 3.28 - 3.21 (m, 1H), 3.09 - 2.96 (m, 1H), 2.88 (s, 3H), 2.84 - 2.77 (m, 1H), 2.26 - 2.14 (m, 2H), 1.95 - 1.90 (m, 1H), 1.87 - 1.74 (m, 1H), 1.69 - 1.59 (m, 1H), 1.43 (d, *J* = 6.2 Hz, 3H), 1.36 - 1.27 (m, 2H), 1.03 - 0.89 (m, 12H), 0.53 (s, 3H).

### Example 8

### (2S)-N-[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-methyl-2-oxo-imidazolidin-1-yl)butanamide

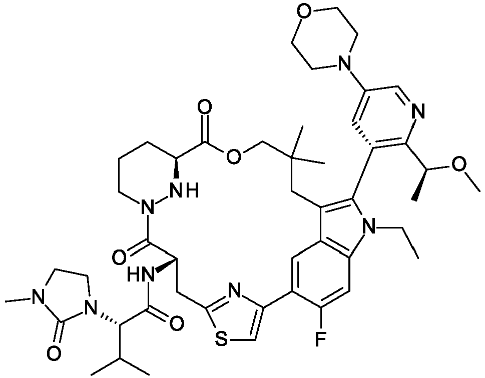

The title compound was prepared in analogy to the preparation of Example **1** by using (2*S*)-3-methyl-2-(3-methyl-2-oxo-imidazolidin-1-yl)butanoic acid (compound **8H**) and (7S,13S)-7-amino-21-ethyl-24-fluoro-(20*M*)-20-[2-[(1*S*)-1-methoxyethyl]-5-morpholino-3-pyridyl]-17,17-dimethyl-15-oxa-4-thia-9,21,27,28-tetrazapentacyclo-[17.5.2.1^{2,5}.19,13.0^{22,26}]octacosa-1(25),2,5(28),19,22(26),23-hexaene-8,14-dione (intermediate **G**) instead of (2S)-2-[[3-(dimethylamino)azetidine-1-carbonyl]-methyl-amino]-3-methyl-butanoic acid (compound **1F**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **8** (50.4 mg) was obtained as a yellow solid. MS calc'd 916.5 (MH⁺), measured 916. 3 (MH⁺). ¹H NMR (400 MHz, CHLOROFORM-*d*) *δ* = 8.84 (br s, 1H), 8.66 (d, *J* = 7.6 Hz, 1H), 7.58 (d, *J =* 2.0 Hz, 1H), 7.10 (d, *J =* 12.3 Hz, 1H), 7.05 (d, *J* = 9.2 Hz, 1H), 5.83 (t, *J* = 9.0 Hz, 1H), 4.59 (br d, *J =* 11.9 Hz, 1H), 4.39 - 4.31 (m, 1H), 4.27 - 4.14 (m, 2H), 4.11 - 4.03 (m, 1H), 3.95 (s, 1H), 3.93 - 3.89 (m, 4H), 3.84 (br d, *J =* 11.5 Hz, 1H), 3.71 (d, *J =* 11.0 Hz, 1H), 3.52 - 3.46 (m, 1H), 3.42 - 3.31 (m, 11H), 3.21 - 3.07 (m, 3H), 2.89 (s, 3H), 2.76 - 2.59 (m, 2H), 2.46 (br d, *J =* 14.4 Hz, 1H), 2.29 - 2.18 (m, 2H), 2.00 - 1.91 (m, 1H), 1.80 (br d, *J* = 12.2 Hz, 1H), 1.66 - 1.59 (m, 1H), 1.54 - 1.50 (m, 3H), 1.00 - 0.90 (m, 12H), 0.49 (s, 3H).

The compound **8H** was prepared according to the following scheme:

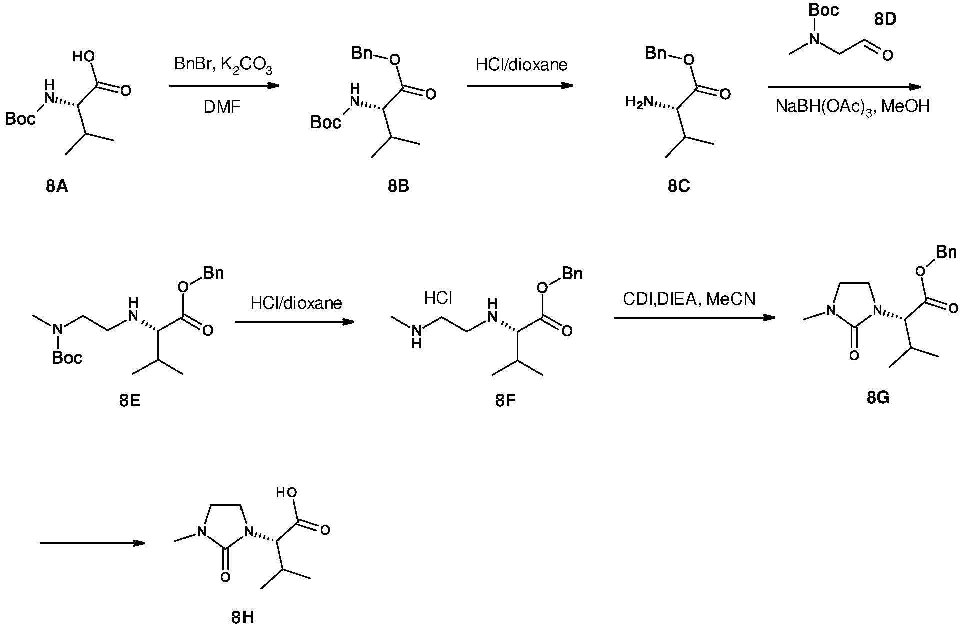

### Step 1: Preparation of benzyl (2S)-2-(tert-butoxycarbonylamino)-3-methyl-butanoate (compound 8B)

To a mixture of BOC-L-valine (compound **8A,** 1.0 g, 4.6 mmol) and potassium carbonate (763.4 mg, 5.52 mmol) in DMF (10 mL) was added benzyl bromide (0.6 mL, 5.06 mmol) at 0 °C. The mixture was stirred at 25 °C for 12 hrs. After the reaction was completed, the reaction mixture was diluted with water (70 mL), extracted with EtOAc (70 mL, three times). The combined organic layer was washed with brine (50 mL) and dried over Na₂SO₄, filtered and concentrated under vacuum to give a residue. The residue was purified by column chromatography to afford benzyl (2*S*)-2-(*tert*-butoxycarbonylamino)-3-methyl-butanoate (compound **8B,** 1.0 g) as colorless oil. MS calc'd 307.4 (MH⁺), measured 208.0 (M-Boc+H⁺).

### Step 2: Preparation of benzyl (2S)-2-amino-3-methyl-butanoate (compound 8C)

A mixture of benzyl (2*S*)-2-(*tert*-butoxycarbonylamino)-3-methyl-butanoate (compound **8B,** 1.0 g, 3.11 mmol) in THF (5 mL) was added with HCl/dioxane (20.0 mL, 80.0 mmol, 4 N), at 0 °C. After the reaction being stirred at 25 °C for 1 h, the reaction mixture was concentrated under vacuum to give a yellow gum. The gum was diluted with aq.NaHCO₃ (40 mL), extracted with EtOAc (50 mL, three times). The combined organic layer was washed with brine (100 mL), dried over Na₂SO₄, filtered and concentrated under vacuum to afford benzyl (2S)-2-amino-3-methyl-butanoate (compound **8C,** 600.0 mg) as colorless oil, which was used to the next step without purification.

### Step 3: Preparation of benzyl (2S)-2-[2-[tert-butoxycarbonyl(methyl)amino]-ethylamino]-3-methyl-butanoate(compound 8E)

To a solution of benzyl (2*S*)-2-amino-3-methyl-butanoate (compound **8C,** 600.0 mg, 2.89 mmol) and *N*-BOC-(methylamino)acetaldehyde (compound **8D,** 601.7 mg, 3.47 mmol) in Methanol (20 mL) was added MgSO₄ (2.1 g, 14.47 mmol). After being stirred at 20 °C for 1 h, the reaction mixture was added with NaBH(OAc)₃ (1.2 g, 5.79 mmol) and stirred at 20 °C for another 1 h. After the reaction was completed, the reaction mixture was filtered and the filtrate was concentrated under vacuum to give colorless oil. The oil was purified by column chromatography to afford benzyl (2*S*)-2-[2-[*tert*-butoxycarbonyl(methyl)amino]-ethylamino]-3-methyl-butanoate(compound **8E,** 700.0 mg) as a colorless gum. MS calc'd 365.2 (MH⁺), measured 365.1 (MH⁺).

### Step 4: Preparation of benzyl (2S)-3-methyl-2-[2-(methylamino)ethylamino]butanoate;hydrochloride (compound 8F)

To a solution of benzyl (2*S*)-2-[2-[*tert*-butoxycarbonyl(methyl)amino]-ethylamino]-3-methyl-butanoate(compound **8E,** 700.0 mg, 1.92 mmol) in THF (5 mL) at 0 °C was added HCl/dioxane (15.0 mL, 60.0 mmol, 4N). After being stirred at 25 °C for 2 hrs, the reaction mixture was concentrated under vacuum to give a white solid. The residual solvent was removed via azeotrope distillation with THF twice to afford benzyl (2*S*)-3-methyl-2-[2-(methylamino)ethylamino]butanoate;hydrochloride (compound **8F,** 600.0 mg) as a white solid. It was used directly to next step without further purification. MS calc'd 265. 2 (MH⁺), measured 265.2 (MH⁺).

### Step 5: Preparation of benzyl (2S)-3-methyl-2-(3-methyl-2-oxo-imidazolidin-1-yl)butanoate (compound 8G)

To a solution of benzyl (2*S*)-3-methyl-2-[2-(methylamino)ethylamino]butanoate;hydrochloride (compound **8F,** 600.0 mg, 1.99 mmol) and DIEA (772.5 mg, 5.98 mmol) in ACN (50 mL) was added CDI (646.4 mg, 3.99 mmol). After being stirred at 0 °C for 2 hrs, the reaction mixture was added with water (10 mL), extracted with EA (40 mL, twice). The combined organic layer was washed with brine (20 mL), dried (Na₂SO₄) and concentrated under vacuum to give a yellow gum. The gum was purified by reversed-phase column to afford benzyl (2*S*)-3-methyl-2-(3-methyl-2-oxo-imidazolidin-1-yl)butanoate (compound **8G,** 300.0 mg) as a colorless gum. MS calc'd 291.2 (MH⁺), measured 291.2 (MH⁺).

### Step 6: Preparation of (2S)-3-methyl-2-(3-methyl-2-oxo-imidazolidin-1-yl)butanoic acid (compound 8H)

To a solution of benzyl (2*S*)-3-methyl-2-(3-methyl-2-oxo-imidazolidin-1-yl)butanoate (compound **8G,** 280.0 mg, 0.96 mmol) in Methanol (10 mL) was added Pd(OH)₂ on activated carbon (0.2 g, 10% purity). The reaction mixture was hydrogenated at rt for 5 hrs. The reaction mixture was filtered and the filtrate was concentrated under vacuum to afford (2*S*)-3-methyl-2-(3-methyl-2-oxo-imidazolidin-1-yl)butanoic acid (compound **8H,** 150.0 mg) as a colorless gum. MS calc'd 201.1 (MH⁺), measured 201.0 (MH⁺).

### Example 9

### N-[(1S)-1-[[(7S,13S)-21-ethyl-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

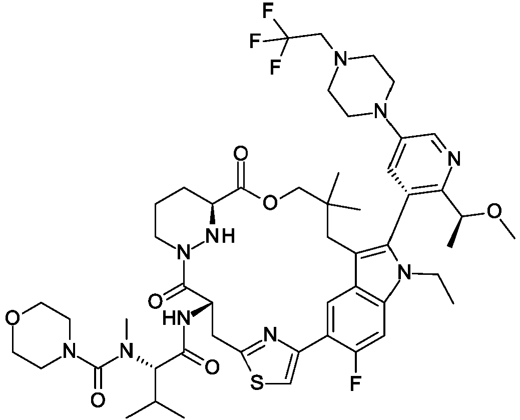

The title compound was prepared in analogy to the preparation of Example **1** by using morpholine and (7*S*,13*S*)-7-amino-21-ethyl-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **F**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **9** (26.8 mg) was obtained as an off-white solid. MS calc'd 1041.5 (MH⁺), measured 1041.7 (MH⁺). ¹H NMR (400 MHz, Methanol-*d*₄) δ = 8.71 (d, *J =* 7.6 Hz, 1H), 8.38 (d, *J* = 2.8 Hz, 1H), 7.87 (d, *J* = 2.8 Hz, 1H), 7.64 (d, *J =* 2.4 Hz, 1H), 7.36 (d, *J =* 12.8 Hz, 1H), 5.86 (d, *J =* 8.4 Hz, 1H), 4.48 - 4.32 (m, 2H), 4.30 - 4.19 (m, 1H), 4.18 - 4.11 (m , 1H), 4.08 - 3.97 (m, 2H), 3.80 - 3.67 (m, 6H), 3.51 - 3.47 (m, 4H), 3.39 - 3.33 (m, 5H), 3.29 - 3.08 (m, 6H), 3.03 - 2.96 (m, 1H), 2.93 - 2.84 (m, 7H), 2.84 - 2.70 (m, 2H), 2.28 - 2.14 (m, 2H), 1.99 - 1.92 (m, 1H), 1.86 - 1.74 (m, 1H), 1.70 - 1.58 (m, 1H), 1.48 - 1.43 (d, *J =* 6.0 Hz, 3H) 1.03 (t, *J =* 6.8 Hz, 3H), 0.96 - 0.89 (m, 9H), 0.61 (s, 3H).

### Example 10

### N-[(1S)-1-[[(7S,13S)-21-ethyl-24-fluoro-(20M)-20-[2-[(1S)-1-methoxyethyl]-5-(4-methylpiperazin-1-yl)-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

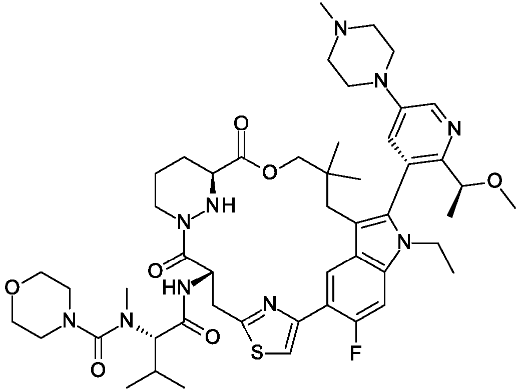

The title compound was prepared in analogy to the preparation of Example **1** by using morpholine and (7*S*,13*S*)-7-amino-21-ethyl-24-fluoro-(20M)-20-[2-[(1S)-1-methoxyethyl]-5-(4-methylpiperazin-1-yl)-3-pyridyl]-17,17-dimethyl-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **D**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **10** (10.3 mg) was obtained as a yellow solid. MS calc'd 973.5 (MH⁺), measured 973.5 (MH⁺). ¹H NMR (400 MHz, Methanol-*d*₄) δ = 8.67 (d, *J =* 7.6 Hz, 1H), 8.49 (d, *J =* 2.8 Hz, 1H), 7.70 - 7.60 (m, 2H), 7.34 (d, *J =* 12.4 Hz, 1H), 5.80 (d, *J =* 8.8 Hz, 1H), 4.43 (d, *J* = 10.8 Hz, 1H), 4.36 - 4.28 (m, 1H), 4.27 - 4.05 (m, 4H), 4.01 (d, *J =* 10.8 Hz, 1H), 3.81 - 3.62 (m, 8H), 3.49 - 3.43 (m, 2H), 3.42 - 3.33 (m, 7H), 3.29 - 3.17 (m, 4H), 3.07 - 3.02 (m, 1H), 2.99 (s, 3H), 2.89 (s, 3H), 2.86 - 2.79 (m, 1H), 2.67 - 2.63 (m, 1H), 2.30 - 2.15 (m, 2H), 2.01 - 1.91 (m, 1H), 1.86 - 1.74 (m, 1H), 1.70 - 1.57 m, 1H), 1.44 (d, *J =* 6.0 Hz, 3H), 1.03 - 0.88 (m, 13H), 0.53 (s, 3H).

### Example 11

### N-[(1S)-1-[[(7S,13S)-24-fluoro-20-(20M)-[2-[(1S)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

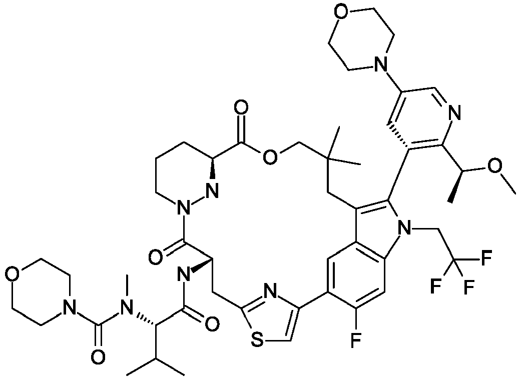

The title compound was prepared in analogy to the preparation of Example **1** by using morpholine and (7*S*,13*S*)-7-amino-24-fluoro-(20*M*)-20-[2-[(1*S*)-1-methoxyethyl]-5-morpholino-3-pyridyl]-17,17-dimethyl-21-(2,2,2-trifluoroethyl)-15-oxa-4-thia-9,21,27,28-tetrazapentacyclo[17.5.2.12,5.19,13.022,26]octacosa-1(25),2,5(28),19,22(26),23-hexaene-8,14-dione (Intermediate **I**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **11** (26.3 mg) was obtained as a white solid. MS calc'd 1013.5 (MH⁺), measured 1013.8 (MH⁺). ¹H NMR (400 MHz, Methanol-*d*₄) *δ* = 8.71 (d, *J =* 7.6 Hz, 1H), 8.49 - 8.35 (d, *J =* 2.8, 1H), 7.73 - 7.69 (d, *J =* 2.4, 1H), 7.66 - 7.59 (d, *J* = 2.4, 1H), 7.53 - 7.43 (d, *J =* 12.4, 1H), 5.88 - 5.70 (d, *J =* 8.8, 1H), 5.25 - 5.13 (m, 1H), 4.53 - 4.38 (m, 1H), 4.31 - 4.15 (m, 2H), 4.07 - 3.98 (d, *J =* 10.8, 1H), 3.91 - 3.84 (t, *J* = 4.8, 4H), 3.83 - 3.67 (m, 6H), 3.52 - 3.45 (m, 1H), 3.43 - 3.34 (m, 10H), 3.30 - 3.18 (m, 3H), 3.11 (m, 1H), 2.99 - 2.89 (s, 3H), 2.88 - 2.78 (m, 1H), 2.72 - 2.60 (d, *J =* 14.8, 1H), 2.34 - 2.15 (m, 2H), 2.01 - 1.91 (m, 1H), 1.90 - 1.74 (m, 1H), 1.72 - 1.57 (m, 1H), 1.52 - 1.42 (d, *J =* 6.0, 3H), 1.05 - 0.88 (m, 9H), 0.57 - 0.45 (s, 3H).

### Example 12

### 3-(dimethylamino)-N-[(1S)-1-[[(7S,13S)-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1²'^{S},1^{9,13}.0^{22,26}]octacosa-1(25),2,5(28),19,22(26),23-hexaen-7-yl]carbamoyl]-2-methyl-propyl]-N-methyl-azetidine-1-carboxamide

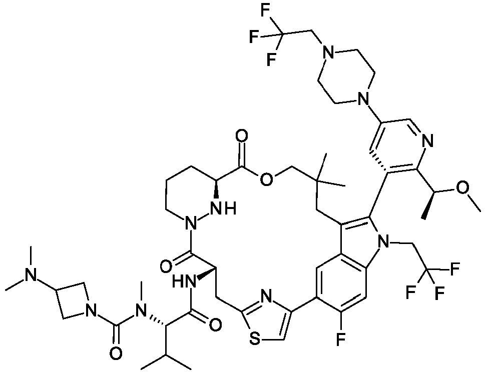

The title compound was prepared in analogy to the preparation of Example **1** by using (7*S*,13*S*)-7-amino-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **E**) instead of (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **12** (45.3 mg) was obtained as a white solid. MS calc'd 1108.5 (MH⁺), measured 1108.5 (MH⁺). ¹H NMR (400MHz, Methanol-*d*₄) *δ* = 8.68 (d, *J =* 7.6 Hz, 1H), 8.42 (d, *J =* 2.8 Hz, 1H), 7.70 (d, *J =* 2.4 Hz, 1H), 7.45 (d, *J* = 12.8 Hz, 1H), 7.32 -7.27 (m, 1H), 5.74 - 5.68 (m, 1H), 5.18 - 5.06 (m, 2H), 4.93 - 5.00 (m, 2H), 4.58 - 4.56(m, 5H), 4.32 - 4.23 (m, 1H), 4.22 - 4.14 (m, 2H), 4.14 - 4.07 (m, 1H), 4.00 - 3.89 (m, 1H), 3.87 - 3.67 (m, 2H), 3.53 - 3.39 (m, 1H), 3.34 - 3.32 (m, 4H), 3.19 - 3.09 (m, 4H), 2.90 - 2.79 (m, 8H), 2.63 - 2.54 (m, 1H), 2.26 - 2.15 (m, 8H), 1.57 - 1.98 (m, 3H), 1.43 (d, *J* = 6.0 Hz, 3H), 0.99 - 0.89 (m, 9H), 0.44 (s, 3H).

### Example 13

### (2S)-N-[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-[methyl(2,2,2-trifluoroethylcarbamoyl)amino]butanamide

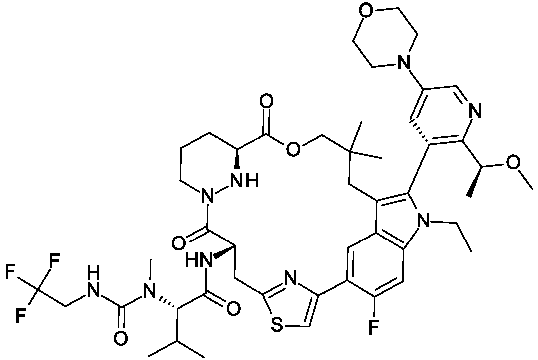

The title compound was prepared in analogy to the preparation of Example **1** by using 2,2,2-Trifluoroethylamine and (7*S*,13*S*)-7-amino-21-ethyl-24-fluoro-(20M)-20-[2-[(1S)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione (Intermediate **G**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **13** (13.4 mg) was obtained as a white solid. MS calc'd 972.4 (MH⁺), measured 972.4 (MH⁺). ¹H NMR (400 MHz, CHLOROFORM-*d*) *δ* = 8.64 (d, *J =* 7.6 Hz, 1H), 8.47 (d, *J =* 2.8 Hz, 1H), 7.55 (d, *J =* 1.7 Hz, 1H), 7.10 (s, 1H), 7.08 - 7.03 (m, 2H), 5.84 (t, *J* = 9.1 Hz, 1H), 5.21 (br s, 1H), 4.58 (br d, *J =* 12.1 Hz, 1H), 4.32 - 4.21 (m, 3H), 4.08 - 4.02 (m, 2H), 3.92 - 3.88 (m, 4H), 3.84 (br d, *J =* 11.0 Hz, 1H), 3.73 - 3.70 (m, 1H), 3.38 (s, 3H), 3.27 - 3.21 (m, 4H), 3.17 - 3.04 (m, 2H), 2.97 (s, 3H), 2.74 - 2.65 (m, 1H), 2.46 (br d, *J =* 14.4 Hz, 1H), 2.31 - 2.21 (m, 5H), 1.96 (br d, *J =* 13.7 Hz, 1H), 1.85 - 1.72 (m, 1H), 1.66 - 1.54 (m, 1H), 1.44 (d, *J =* 6.1 Hz, 3H), 1.25 (t, *J =* 7.0 Hz, 1H), 0.99 - 0.86 (m, 12H), 0.45 (s, 3H).

### Example 14

### N-[(1S)-1-[[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-3-(trifluoromethyl)azetidine-1-carboxamide

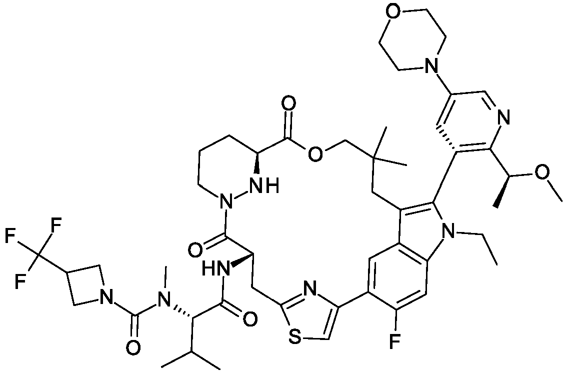

The title compound was prepared in analogy to the preparation of Example **1** by using 3-(Trifluoromethyl)azetidine and (7*S*,13*S*)-7-amino-21-ethyl-24-fluoro-(20M)-20-[2-[(1S)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione (Intermediate **G**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **14** (3.1 mg) was obtained as a white solid. MS calc'd 998.5 (MH⁺), measured 998.5 (MH⁺). ¹H NMR (400 MHz, CHLOROFORM-*d*) *δ* = 8.64 (d, *J =* 7.5 Hz, 1H), 8.47 (d, *J =* 2.4 Hz, 1H), 7.58 (s, 1H), 7.32 (br d, *J =* 9.6 Hz, 1H), 7.11 - 7.04 (m, 2H), 5.81 (br t, *J =* 8.8 Hz, 1H), 4.59 (br d, *J =* 9.8 Hz, 1H), 4.35 (br t, *J =* 8.7 Hz, 1H), 4.30 - 4.21 (m, 4H), 4.13 (br dd, *J* = 7.9, 15.0 Hz, 3H), 4.08 - 3.99 (m, 3H), 3.90 (br s, 4H), 3.84 (br d, *J =* 11.3 Hz, 1H), 3.43 (br d, *J =* 14.6 Hz, 1H), 3.37 (s, 3H), 3.24 (br d, *J =* 4.3 Hz, 4H), 3.18 - 3.13 (m, 1H), 2.84 (s, 3H), 2.75 - 2.62 (m, 1H), 2.47 (br d, *J =* 14.4 Hz, 1H), 2.31 - 2.17 (m, 2H), 2.05 (s, 3H), 1.94 (br d, *J =* 1.8 Hz, 1H), 1.51 (br s, 2H), 1.46 - 1.46 (m, 1H), 1.44 - 1.44 (m, 1H), 1.15 (td, *J* = 6.4, 13.0 Hz, 12H), 0.46 (s, 3H).

### Example 15

### (2S)-N-[(7S,13S)-21-ethyl-24-fluoro-(20M)-20-[2-[(1S)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-oxo-5,6,8,8a-tetrahydro-1H-imidazo[5,1-c][1,4]oxazin-2-yl)butanamide

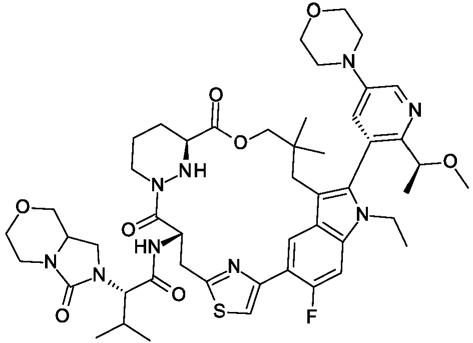

The title compound was prepared in analogy to the preparation of Example **1** by using (2S)-3-methyl-2-(3-oxo-5,6,8,8a-tetrahydro-1H-imidazo[5,1-c][1,4]oxazin-2-yl)butanoic acid (compound **15g**) and (7*S*,13*S*)-7-amino-21-ethyl-24-fluoro-(20M)-20-[2-[(1S)-1-methoxyethyl]-5-morpholino-3-pyridyl]-17,17-dimethyl-15-oxa-4-thia-9,21,27,28-tetrazapentacyclo-[17.5.2.1^{2,5}.1^{9,13}.0^{22,26}]octacosa-1(25),2,5(28),19,22(26),23-hexaene-8,14-dione (intermediate **G**) instead of (2*S*)-2-[[3-(dimethylamino)azetidine-1-carbonyl]-methyl-amino]-3-methyl-butanoic acid (compound **1F**) and (7*S*,13*S*)-7-amino-24-fluoro-(20M)-20-[2-[(1S)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **15** (37.5 mg) was obtained as a white solid. MS calc'd 958.5 (MH⁺), measured 958.5 (MH⁺). ¹H NMR (400 MHz, METHANOL-*d*₄) δ = 8.70 - 8.61 (m, 1H), 8.53 - 8.30 (m, 1H), 7.64 - 7.59 (m, 1H), 7.38 - 7.27 (m, 2H), 5.86 - 5.72 (m, 1H), 5.03 - 4.92 (m, 1H), 4.82 - 4.78 (m, 1H), 4.67 - 4.53 (m, 3H), 4.47 - 4.36 (d, *J*=12.8 Hz,1H), 4.29 - 4.12 (m, 4H), 4.10 - 4.01 (d, *J*=10.8 Hz,1H), 3.89 - 3.84 (m, 4H), 3.79 - 3.68 (m, 4H), 3.65 - 3.54 (m, 1H), 3.50 - 3.36 (m, 4H), 3.28 (m, 4H), 3.20 - 3.10 (m, 2H), 3.03 - 2.95 (m, 1H), 2.88 - 2.75 (m, 1H), 2.69 - 2.58 (m, 1H), 2.24 - 2.07 (m, 2H), 2.01 - 1.89 (m, 1H), 1.87 - 1.71 (m, 1H), 1.70 - 1.56 (m, 1H), 1.51 - 1.37 (m, 3H), 1.03 - 0.87 (m, 12H), 0.62 - 0.42 (m, 3H).

The compound **15g** was prepared according to the following scheme:

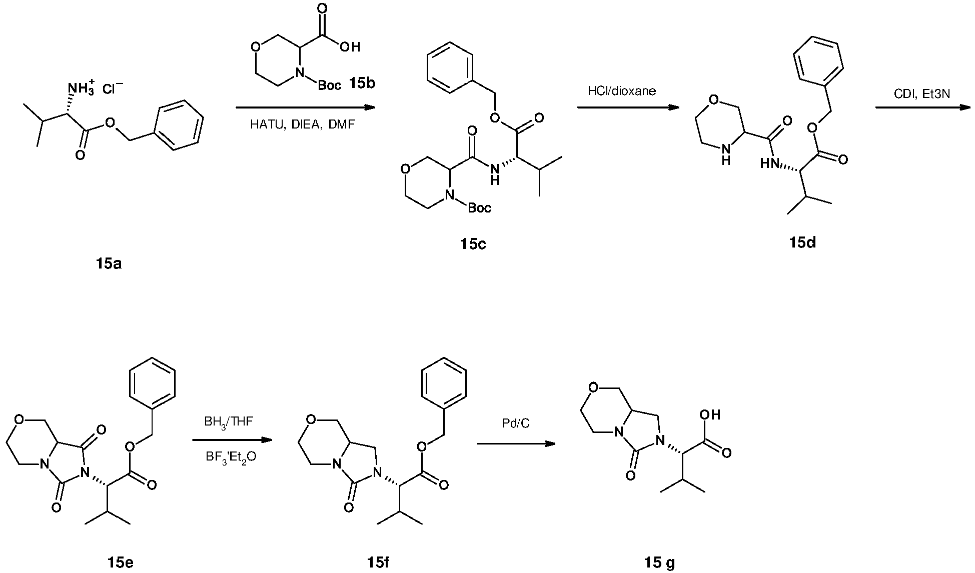

### Step1: tert-butyl 3-[[(1S)-1-benzyloxycarbonyl-2-methyl-propyl]carbamoyl]-morpholine-4-carboxylate (compound 15c)

The solution of 4-*tert*-butoxycarbonylmorpholine-3-carboxylic acid (compound **15b,** 2.8 g, 12.31 mmol) and L-valine benzyl ester hydrochloride (compound **15a,** 3.0 g, 12.31 mmol) in DMF (30 mL) were added with DIEA (4.29 mL, 24.62 mmol) and HATU (5.1 g, 13.54 mmol). After being stirred at 25 °C for 1 h, the reaction mixture were added with EtOAc (40 mL) and water (40 mL). The layers were separated and the aqueous phase was extracted with EtOAc (30 mL, twice). The combined organic layer was washed with brine (60 mL), dried over Na₂SO₄, filtered, and concentrated under vacuum to give *tert*-butyl 3-[[(1*S*)-1-benzyloxycarbonyl-2-methyl-propyl]carbamoyl]morpholine-4-carboxylate (compound **15c,** 7.5 g) as white oil. MS calc'd 420.23(MH⁺), measured 321.2 (M-Boc+H⁺).

### Step2: benzyl (2S)-3-methyl-2-(morpholine-3-carbonylamino)-butanoate; hydrochloride (compound 15d)

To a solution of *tert*-butyl 3-[[(1*S*)-1-benzyloxycarbonyl-2-methylpropyl]carbamoyl]morpholine-4-carboxylate (compound **15c,** 7.2 g, 17.16 mmol) in 1,4-dioxane (10 mL) was added 1,4-dioxane/HCl (4M, 15.0 mL, 60.0 mmol). The mixture was stirred at 25 °C for 2 hrs. The mixture was concentrated under vacuum to afford benzyl (2*S*)-3-methyl-2-(morpholine-3-carbonylamino)-butanoate; hydrochloride (compound **15d,** 6.7 g) as white oil. MS calc'd 321.2 (MH⁺), measured 321.2 (MH⁺).

### Step3: benzyl (2S)-2-(1,3-dioxo-5,6,8,8a-tetrahydroimidazo[5,1-c][1,4]oxazin-2-yl)-3-methyl-butanoate (compound 15e)

The solution of benzyl (2S)-3-methyl-2-(morpholine-3-carbonylamino)butanoate (compound **15d,** 6.5 g, 20.29 mmol) in THF (1 mL) was added TEA (8.5 mL, 60.87 mmol) and *N.N'*-carbonyldiimidazole (3.9 g, 24.35 mmol). After being stirred at 25 °C for 2 hrs, the reaction mixture was concentrated under vacuum to give a residue. The residue was purified by reversed-phase chromatography column to afford benzyl (2S)-2-(1,3-dioxo-5,6,8,8a-tetrahydroimidazo[5,1-c][1,4]oxazin-2-yl)-3-methyl-butanoate (compound **15e,** 2.36 g) as white oil. MS calc'd 346.4 (MH⁺), measured 369.4 (MNa⁺).

### Step4: benzyl (2S)-3-methyl-2-(1-oxo-5,6,8,8a-tetrahydro-3H-imidazo[5,1-c][1,4]oxazin-2-yl)butanoate (compound 15e)

To a solution of benzyl (2*S*)-2-(1,3-dioxo-5,6,8,8a-tetrahydroimidazo[5,1-c][1,4]oxazin-2-yl)-3-methyl-butanoate (compound **15e,** 1 g, 2.89 mmol) in THF (5 mL) were added boron trifluoride diethyl etherate (1M, 1.2 mL, 1.2 mmol) and borane-THF (5.77 mL, 5.77 mmol) at 0°C. After being stirred at 20 °C for 18 hrs, the reaction was quenched with water (40 mL). The aqueous phase was extracted with EtOAc (30 mL, twice). The combined organic layer was washed with brine (60 mL), dried over Mg₂SO₄, filtered, and concentrated under vacuum to give a residue. The residue was purified by reversed phase column to give benzyl (2*S*)-3-methyl-2-(1-oxo-5,6,8,8a-tetrahydro-3*H*-imidazo[5,1-c][1,4]oxazin-2-yl)butanoate (compound **15f,** 220.0 mg) as colorless oil. MS calc'd 333.2 (MH⁺), measured 333.2 (MH⁺).

### Step5: (2S)-3-methyl-2-(3-oxo-5,6,8,8a-tetrahydro-1H-imidazo[5,1-c][1,4]oxazin-2-yl)butanoic acid (compound 15f)

To a solution of benzyl (2*S*)-3-methyl-2-(1-oxo-5,6,8,8a-tetrahydro-3*H*-imidazo[5,1-c][1,4]oxazin-2-yl)butanoate (compound **15e,** 130.0 mg, 0.39 mmol) in THF (1 mL) was added Pd/C (40.0 mg, 0.78 mmol). The reaction mixture was stirred at 25 °C for 1 h under hydrogen atmosphere. The mixture was filtered, and the filtrate was concentrated under vacuum to give (2S)-3-methyl-2-(3-oxo-5,6,8,8a-tetrahydro-1*H*-imidazo[5,1-c][1,4]oxazin-2-yl)butanoic acid (compound **15g,** 62.0 mg) as colorless oil. MS calc'd 243.1 (MH⁺), measured 243.2 (MH⁺).

### Example 16

### (3R)-N-[(1S)-1-[[(7S,13S)-24-fluoro-(20M)-20-[2-[(1S)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.^{022,26}]octacosa-1(25),2,5(28),19,22(26),23-hexaen-7-yl]carbamoyl]-2-methyl-propyl]-3-hydroxy-N,3-dimethyl-piperidine-1-carboxamide

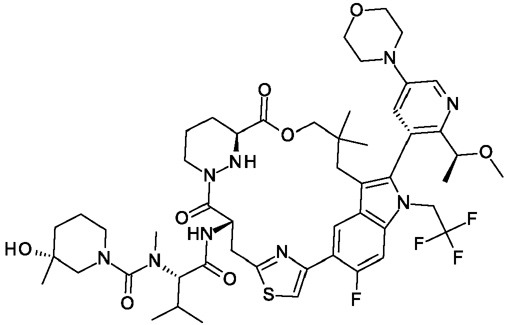

The title compound was prepared in analogy to the preparation of Example **1** by using (3*R*)-3-methylpiperidin-3-ol;hydrochloride and (7*S*,13*S*)-7-amino-24-fluoro-(20*M*)-20-[2-[(1*S*)-1-methoxyethyl]-5-morpholino-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **I**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **16** (13.9 mg) was obtained as a white solid. MS calc'd 1042.5 (MH⁺), measured 1042.5 (MH⁺). ¹H NMR (400 MHz, Methanol-*d*₄) *δ* = 8.73 - 8.69 (d, *J* = 7.2 Hz, 1H), 8.44 - 8.39 (d, *J =* 2.8 Hz, 1H), 7.70 - 7.67 (d, *J* = 2.8 Hz, 1H), 7.67 - 7.64 (d, *J =* 2.4 Hz, 1H), 7.51 - 7.45 (d, *J =* 12.4 Hz, 1H), 5.80 - 5.74 (d, *J =* 8.4 Hz, 1H), 5.23 - 5.13 (m, 1H), 4.83 - 4.76 (m, 1H), 4.47 - 4.40 (m, 1H), 4.29 - 4.23 (q, *J* = 6.4 Hz, *J =* 12.0 Hz, 1H), 4.23 - 4.17 (dd, *J* = 2.4, 11.6 Hz, 1H), 4.05 - 3.99 (d, *J =* 10.8 Hz, 1H), 3.89 - 3.85 (t, *J* = 4.4 Hz, 4H), 3.82 - 3.78 (d, *J* = 11.2 Hz, 1H), 3.74 - 3.69 (d, *J =* 11.2 Hz, 1H), 3.56 - 3.45 (m, 2H), 3.39 - 3.36 (m, 6H), 3.29 - 3.19 (m, 2H), 3.14 - 3.06 (m, 1H), 3.03 - 2.96 (d, *J =* 12.8 Hz, 2H), 2.91 - 2.89 (s, 3H), 2.87 - 2.78 (m, 1H), 2.71 - 2.63 (m, 1H), 2.29 - 2.16 (m, 2H), 2.00 - 1.90 (m, 3H), 1.88 - 1.76 (m, 1H), 1.74 - 1.68 (m, 1H), 1.67 - 1.53 (m, 3H), 1.49 - 1.45 (d, *J =* 6.4 Hz, 3H), 1.34 - 1.27 (m, 1H), 1.25 - 1.21 (s, 3H), 0.99 - 0.93 (dd, *J =* 6.0, 12.0 Hz, 9H), 0.56 - 0.49 (s, 3H).

### Example 17

### N-[(1S)-1-[[(7S,13S)-25-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-morpholine-4-carboxamide

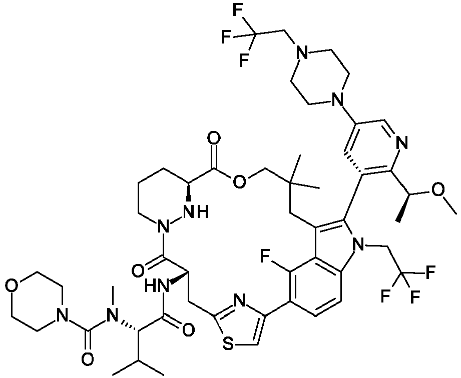

The title compound was prepared in analogy to the preparation of Example **1** by using morpholine and (7*S*,13*S*)-7-amino-25-fluoro-(20*M*)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **J**) instead of *N,N*-dimethylazetidin-3-amine dihydrochloride (compound **1D**) and (7*S*,13*S*)-7-amino-24-fluoro-(20*M*)-20-[2-[(1S)-1-methoxyethyl]-5-(4-methylpiperazin-1-yl)-3-pyridyl]-17, 17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **17** (12.9 mg) was obtained as a yellow solid. MS calc'd 1094.5 (MH⁺), measured 1094.5 (MH⁺). ¹H NMR (400 MHz, Methanol-*d*₄) *δ* = 8.49 - 8.39 (m, 1H), 7.74 (br s, 1H), 7.53 - 7.44 (m, 3H), 5.97 (br d, *J =* 5.9 Hz, 1H), 5.18 - 5.11 (m, 1H), 4.67 - 4.58 (m, 1H), 4.42 - 4.33 (m, 1H), 4.10 - 3.97 (m, 1H), 3.94 - 3.80 (m, 2H), 3.76 - 3.63 (m, 5H), 3.58 - 3.42 (m, 7H), 3.26 - 3.14 (m, 8H), 3.13 - 2.99 (m, 2H), 2.93 (br d, *J =* 14.4 Hz, 8H), 2.62 - 2.53 (m, 1H), 2.30 - 2.14 (m, 1H), 1.61 - 1.49 (m, 1H), 1.43 (br d, *J =* 6.1 Hz, 3H), 1.34 - 1.15 (m, 3H), 0.92 (br t, *J =* 7.3 Hz, 6H), 0.85 - 0.69 (m, 6H).

### Example 18

### N-[(1S)-1-[[(7S,13S)-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5},1^{9,13}.0^{22,26}]octacosa-1(25),2,5(28),19,22(26),23-hexaen-7-yl]carbamoyl]-2-methyl-propyl]-N,4-dimethylpiperazine-1-carboxamide

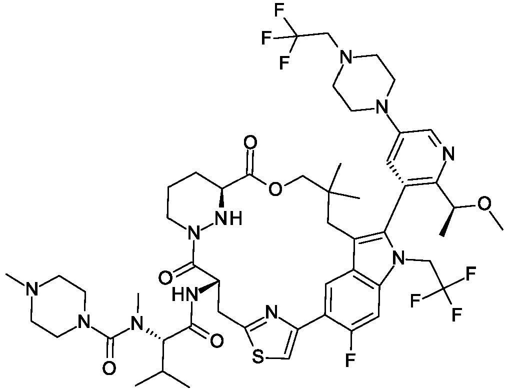

The title compound was prepared in analogy to the preparation of Example **1** by using 1-methylpiperazine and (7*S*,13*S*)-7-amino-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **E**) instead of *N,N*-dimethylazetidin-3-amine dihydrochloride (compound **1D**) and (7*S*,13*S*)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **18** (10.8 mg) was obtained as a white solid. MS calc'd 1108.5 (MH⁺), measured 1108.5 (MH⁺). ¹H NMR (400 MHz, Methanol-*d*₄) *δ* = 8.70 - 8.65 (d, *J* = 7.6 Hz, 1H), 8.45 - 8.40 (d, *J* = 2.8 Hz, 1H), 7.70 - 7.65 (d, *J* = 2.4 Hz, 1H), 7.50 - 7.43 (d, *J =* 12.8 Hz, 1H), 7.33 - 7.28 (d, *J* = 2.8 Hz, 1H), 5.78 - 5.70 (d, *J* = 9.2 Hz, 1H), 5.20 - 5.05 (m, 1H), 4.62 - 4.61 (s, 1H), 4.46 - 4.40 (m, 1H), 4.28 - 4.20 (m, 1H), 4.19 - 4.13 (m, 1H), 4.06 - 3.99 (d, *J* = 11.2 Hz, 1H), 3.83 - 3.65 (m, 3H), 3.53 - 3.40 (m, 4H), 3.27 - 3.07 (m, 6H), 2.94 - 2.81 (m, 9H), 2.63 - 2.45 (m, 6H), 2.37-2.34 (s, 3H), 2.29 - 2.17 (m, 3H), 2.00 - 1.93 (m, 1H), 1.90 - 1.75 (m, 2H), 1.71 - 1.56 (m, 1H), 1.46 - 1.41 (d, *J =* 6.4 Hz, 3H), 1.33 - 1.15 (m, 1H), 0.98 - 0.91 (m, 9H), 0.47 - 0.42 (s, 3H).

### Example 19

### N-[(1S)-1-[[(7S,13S)-24-fluoro-(20M)-20-[2-[(1S)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}loctacosa-1(25),2,5(28),19,22(26),23-hexaen-7-yl]carbamoyl]-2-methyl-propyl]-N-methyl-4-(2,2,2-trifluoroethyl)piperazine-1-carboxamide

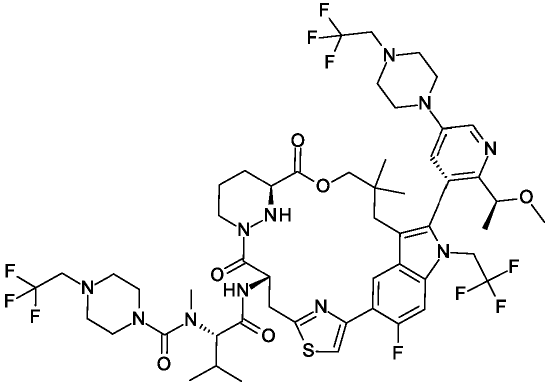

The title compound was prepared in analogy to the preparation of Example **1** by using 1-(2,2,2-trifluoroethyl)piperazine and (7*S*,13*S*)-7-amino-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0²²'²⁶]octacosa-1(25),2,5(28),19,22(26),23-hexaene-8,14-dione (Intermediate **E**) instead of *N,N-*dimethylazetidin-3-amine dihydrochloride (compound **1D**) and (7S,13S)-7-amino-24-fluoro-(20*M*)-20-[2-[(1*S*)-1-methoxyethyl]-5-(4-methylpiperazin-1-yl)-3-pyridyl]-17,17-dimethyl-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaene-8,14-dione (Intermediate **C**). Example **19** (12.1 mg) was obtained as a white solid. MS calc'd 1176.5 (MH⁺), measured 1176.5 (MH⁺). ¹H NMR (400 MHz, Methanol-*d*₄) *δ* = 8.82 - 8.63 (d, *J* = 7.2 Hz, 1H), 8.48 - 8.38 (d, *J =* 2.8 Hz, 1H), 7.75 - 7.67 (d, *J* = 2.0 Hz, 1H), 7.54 - 7.43 (d, *J =* 12.4 Hz, 1H), 7.35 - 7.28 (d, *J =* 2.4 Hz, 1H), 5.86 - 5.72 (d, *J* = 8.8 Hz, 1H), 5.26 - 5.03 (m, 1H), 4.98 - 4.90 (m, 3H), 4.66 - 4.56 (m, 1H), 4.51 - 4.41 (m, 1H), 4.32 - 4.13 (m, 2H), 4.07 - 4.03 (d, *J* = 11.3 Hz, 1H), 3.86 - 3.77 (d, *J =* 11.6 Hz, 1H), 3.76 - 3.69 (d, *J =* 11.2 Hz, 1H), 3.57 - 3.48 (m, 1H), 3.48 - 3.41 (m, 2H), 3.39 - 3.35 (d, *J* = 3.1 Hz, 4H), 3.29 - 3.23 (m, 1H), 3.22 - 3.10 (m, 6H), 2.96 - 2.92 (s, 3H), 2.91 - 2.86 (t, *J =* 4.8 Hz, 4H), 2.85 - 2.76 (m, 3H), 2.76 - 2.69 (m, 2H), 2.67 - 2.57 (d, *J =* 14.4 Hz, 1H), 2.33 - 2.19 (m, 2H), 2.03 - 1.94 (m, 1H), 1.92 - 1.79 (m, 1H), 1.73 - 1.59 (m, 1H), 1.50-1.41 (d, *J =* 6.0 Hz, 3H), 1.35 - 1.30 (m, 1H), 1.00 - 0.92 (m, 9H), 0.54 - 0.41 (s, 3H).

### BIOLOGICAL EXAMPLE

Compound **A555** (page 158 of Table.1) from WO2021091956 was cited as reference compound for this invention.

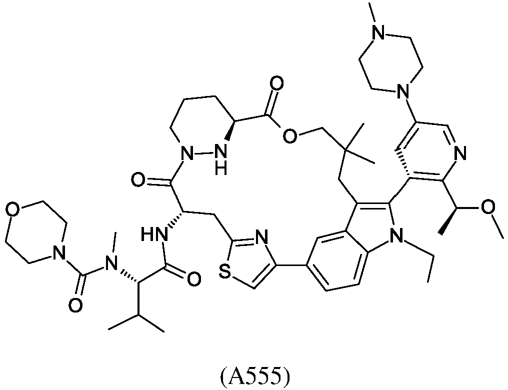

### Example 20

### Single dose pharmacokinetics (PK) study in female BALB/c mice

The purpose of this study was to determine the pharmacokinetics of selected compounds following single intravenous bolus or oral gavage administration in female BALB/c mice. Briefly, two groups of female BALB/c mice (available from Shanghai Lingchang Biotechnology Co., Ltd) (N=3/group) were treated with a single dose of compound intravenously at 3 mg/kg (IV) or orally at 30 mg/kg (PO). Blood samples were collected at 5 min (only for IV), 15 min, 30 min, 1 h, 2 h, 4 h, 7 h and 24 h post-dose. Blood samples were placed on ice until centrifugation to obtain plasma samples. The concentration of compound in plasma samples was determined using LC-MS/MS method. The pharmacokinetic parameters were calculated by non-compartmental analysis.

**Table 1. Results of SDPK**

| compound | 3 mg/kg, iv | 30 mg/kg, po | | |
|---|---|---|---|---|
| | CL (mL/min/kg) | Cₘₐₓ (ng/mL) | AUC₀₋ₗₐₛₜ (h×ng/mL) | F(%) |
| **Example 6** | 3.70 | 4889 | 36825 | 29.7 |
| A555 | 42.1 | 725 | 2477 | 21.5 |

From Table 1, it can be seen that Example 6 has excellent pharmacokinetic properties in mouse model, including almost 7 folds of Cₘₐₓ, 15 folds of AUC₀₋ₗₐₛₜ and improved bioavailability and significant lower clearance compared with compound A555.

### Example 21

### Human hepatocyte stability Assay

The hepatocyte stability assay measures the rate of disappearance of a compound from incubations with cryopreserved suspension hepatocytes from human. Positive controls, including Midazolam, Raloxifene and Dextromethorphan, are included in every experiment. Incubations consist of 1 µM tested compound and suspension of human hepatocytes (1×10 ⁶ cells/mL) in supplemented Williams' E Medium with 10% FBS and 0.5% Penicillinstreptomycin. The hepatocyte suspension was incubated with intermittent shaking 900 rpm at 37°C, in a 5% CO₂ incubator. The reaction was stopped by adding methanol containing internal standard (2 µM Tolbutamide) at 2, 10, 20, 40, 60 and 120 minutes after compound addition, depletion of the parent compound was monitored by LC-MS/MS analysis.

**Table 2. Human hepatocytes stability of Examples and Compounds of present invention**

| Example | CL_hep (Human) (ml/min/kg) |
|---|---|
| A555 | 6.5 |
| Example 6 | < 2.4 |

Above result clearly shows that reference compounds (**A555**) had much higher clearance while Example 6 maintained the low clearance in human hepatocytes stability assay. Achieving low clearance is advantageous to improve in vivo performance of the compound, such as dose reduction, exposure enhancement, and half-life prolongation.

### Example 22

### Cell viability assay

The purpose of this cellular assay was to determine the effects of test compounds on the proliferation of human cancer cell lines NCI-H358 (ATCC-CRL5807) cells, AGS (ATCC-CRL-1739) cells, SW620 (ATCC-CCL-227) over a 3-day treatment period by quantifying the amount of NADPH present at endpoint using Cell Counting Kit-8.

Cells were seeded at 5,000 cells/well (NCI-H358), 2,000 cells/well (AGS) 2,000 cells/well (SW620) in 96-well assay plates (Corning-3699) and incubated overnight. On the day of the assay, diluted compounds were then added in a final concentration of 0.5% DMSO. After 72 hrs incubation, a tenth of the volume of cell counting kit 8(Dnjindo-CK04) was added into each well. Read the signal (OD450 minus OD650) using EnVision after 2 hrs incubation. IC₅₀ was determined by fitting a 4-parameter sigmoidal concentration response model.

**Table 3. Activity of Examples and Compounds of present invention in KRAS Cell viability assay**

| Example | G12C IC50 (µM) | G12D IC50 (µM) | G12V IC50 (µM) |
|---|---|---|---|
| A555 | 0.003 | 0.008 | 0.003 |
| Example 1 | 0.002 | 0.013 | 0.001 |
| Example 2 | 0.004 | 0.016 | 0.003 |
| Example 3 | 0.001 | 0.015 | 0.001 |
| Example 4 | 0.003 | 0.017 | 0.001 |
| Example 5 | 0.002 | 0.004 | 0.001 |
| Example 6 | 0.005 | 0.020 | 0.002 |
| Example 8 | 0.012 | 0.057 | 0.007 |
| Example 9 | 0.024 | 0.091 | 0.014 |
| Example 10 | 0.006 | 0.040 | 0.006 |
| Example 11 | 0.002 | 0.006 | 0.001 |
| Example 12 | 0.009 | 0.038 | 0.007 |
| Example 13 | 0.026 | 0.447 | 0.029 |
| Example 14 | 0.100 | >10 | 0.073 |
| Example 15 | 1.201 | 1.375 | 0.064 |
| Example 16 | 0.016 | 0.069 | 0.009 |
| Example 17 | 0.004 | 0.046 | 0.006 |

### Example 23

### KRAS-BRAF with CYPA (500 nM) interaction assay

In this example, TR-FRET was also used to measure the compound or compound-CYPA dependent disruption of the KRAS G12C-BRAF complex. This protocol was also used to measure disruption of KRAS G12D or KRAS G12V binding to BRAF by a compound of the invention, respectively. In assay buffer containing 25mM HEPES PH=7.4 (4-(2-hydroxyethyl)-1-piperazineethanesulfonic acid, Thermo, 15630080), 0.002% Tween20, 0.1% BSA, 100mM NaCl, 5mM MgCl₂, 10 µM GMPPNP (Guanosine 5'-[β,γ-imido]triphosphate trisodium salt hydrate, Sigma, G0635), tagless CYPA, GMPPNP loaded 6His-KRAS proteins, and GST-BRAF^{RBD} were mixed in a well of a 384-well assay plate at final concentrations of 50 nM, 6.25 nM and 1nM, respectively. Compound was present in plate wells as a 16-point 3-fold dilution series starting at a final concentration of 10 µM and incubated for 3 hours. A mixture of MAb Anti-6His-XL665 (Cisbio, 61HISXLB) and Mab anti-GST-TB cryptate (Cisbio, 61GSTTLB)was then added at a final concentration of 6.67 nM and 0.21 nM, respectively, and the plate was incubated for an additional 1.5 hours. TR-FRET signal was read on a PHERstar FSX microplate reader (Ex320 nm, Em 665/615 nm). Compounds that facilitate disruption of the KRAS-BRAF complex were identified as those eliciting a decrease in the TR-FRET ratio relative to DMSO control wells.

**Table 4. Activity of Examples and Compounds of present invention in KRAS-BRAF with CYPA (500 nM) interaction assay**

| Example | G12C IC50 (µM) | G12D IC50 (µM) | G12V IC50 (µM) |
|---|---|---|---|
| A555 | 0.005 | 0.043 | 0.023 |
| Example 1 | 0.046 | 0.147 | 0.070 |
| Example 5 | 0.050 | 0.089 | 0.129 |
| Example 6 | 0.023 | 0.071 | 0.061 |
| Example 7 | 0.095 | 0.472 | 0.611 |
| Example 9 | 0.049 | 0.225 | 0.124 |
| Example 10 | 0.061 | 0.186 | 0.149 |
| Example 11 | 0.024 | 0.112 | 0.059 |
| Example 12 | 0.029 | 0.140 | 0.085 |
| Example 13 | >10 | >10 | >10 |
| Example 14 | >10 | >10 | >10 |
| Example 15 | 0.892 | 1.648 | 0.924 |
| Example 16 | 0.026 | 0.059 | 0.097 |
| Example 17 | 0.002 | 0.007 | 0.004 |

### Example 24

### pERK inhibition assay

This assay is to measure the ability of test compounds in inhibiting the phosphorylation of ERK, the downstream signaling of KRAS G12C in NCI-H358 cells, KRAS G12D in AGS cells, and KRAS G12V in SW620. NCI-H358 (ATCC-CRL5807) cells, AGS (ATCC-CRL-1739) cells, SW620 (ATCC-CCL-227) cells were all grown and maintained using RPMI-1640 medium (Thermo Fisher Scientific) with 10% fetal bovine serum and 1% penicillin/streptomycin. On the day prior to compound addition, cells were plated in tissue culture-treated 96 well plates (Corning-3699) at a density of 30,000 cell/well, 20,000 cell/well, 30,000 cell/well for NCI-H358, AGS and SW620 respectively, and allowed for attachment overnight. Diluted compounds were then added in a final concentration of 0.5% DMSO. After 4 hours of incubation, the medium was removed, 100 µL of 4% formaldehyde was added, and the assay plates were incubated at room temperature for 20 minutes. The plates were then washed once with phosphate buffered saline (PBS), and permeabilized with 100 µL of chilled methanol for 10 minutes. Non-specific antibody binding to the plates was blocked using 50 µL 1X BSA blocking buffer (Thermo-37520, 10-fold dilution by Phosphate-Buffered Saline Tween (PBST) for at least 1 hour at room temperature.

The amount of phosphor-ERK was determined using an antibody specific for phosphorylated form of ERK. Primary antibody (pERK, CST-4370, Cell Signaling Technology) was diluted 1:300 in blocking buffer, with 50 µL aliquoted to each well, and incubated overnight at 4 °C. Cells was washed five times for 5 minutes with PBST. Secondary antibody (HRP-linked anti-rabbit IgG, CST-7074, Cell Signaling Technology) was diluted 1:1000 in blocking buffer, and 50 µL was added to each well and incubated 1-2 hrs at room temperature. Cells was washed 5 times for 5 minutes with PBST, 100µL TMB ELISA substrate (abcam-ab171523) were added and gently shake for 20 minutes. 50µL stop solution (abcam-ab171529) were added, and then read the signal (OD450) by EnVision.

IC₅₀ was determined by fitting a 4-parameter sigmoidal concentration response model.

**Table 5. Activity of Examples and Compounds of present invention in KRAS pERK inhibition assay**

| Example | G12C IC₅₀ (µM) | G12D IC₅₀ (µM) | G12V IC₅₀ (µM) |
|---|---|---|---|
| A555 | 0.004 | 0.003 | 0.003 |
| Example 1 | 0.001 | 0.002 | 0.001 |
| Example 2 | 0.001 | 0.005 | 0.004 |
| Example 3 | 0.001 | 0.001 | 0.001 |
| Example 4 | 0.001 | 0.003 | 0.001 |
| Example 5 | 0.002 | 0.002 | <0.001 |
| Example 6 | 0.002 | 0.003 | 0.002 |
| Example 8 | 0.024 | 0.024 | 0.005 |
| Example 9 | 0.024 | 0.023 | 0.009 |
| Example 10 | 0.014 | 0.008 | 0.006 |
| Example 11 | 0.003 | 0.001 | 0.001 |
| Example 12 | 0.006 | 0.015 | 0.004 |
| Example 13 | 0.019 | 0.046 | 0.008 |
| Example 14 | 0.028 | 0.032 | 0.012 |
| Example 15 | 0.058 | 0.047 | 0.031 |
| Example 16 | 0.013 | 0.008 | 0.007 |
| Example 17 | 0.008 | 0.004 | 0.004 |
| Example 18 | 0.023 | 0.012 | 0.007 |
| Example 19 | 0.066 | 0.054 | 0.016 |

### Example 25

### Stable KRAS mutant cell lines and cell viability assay.

The aim of the study was to determine the potency and efficacy of compounds for cell proliferation using CellTiter-Glo^{®} (CTG) Luminescent Cell Viability Assay (Promega Corp., Madison, WI) . We cloned 14 KRAS^{G12C} variant sequences with secondary mutations (V8A, V9Y, S17E, T58I, A59T, S65W, R68S, D69P, M72I, D92R, H95N, Y96D, Q99F, Q99W, Y96H, and F156L) into the Miapaca-2. Totally 14 stable Miapaca-2 mutant cell lines were established through lentivirus infection. For the cell viability assay, cells were dosed with compounds in a 9-point dose-response using a 4 fold dilution series at a top dose of 10µM. KRAS mutant cells were maintained in DMEM+10%FBS+2.5%HI Horse serum+1%PS+ 1µg/mL Puromycin and seeded into 96-well plates at 800-1,500 cells per well 24 h before compound addition and then incubated with compound for 3 d before assaying viability (CellTiter-Glo, Promega). Assays were performed in biological duplicates. Nonlinear regression curves were fitted using Xfit. IC50 (absolute IC50) is the dose at which the estimated viability is 50% relative to untreated wells. Inhibition rate of the compound is calculated according to the formula below: %inhibition=100-100×(Luminescence value-HPE) / (ZPE-HPE).
HPE: Luminescence value from the wells with only medium
ZPE: Luminescence value from the wells with DMSO

**Table 8. cell viability in mutant cells with KRAS G12C and other mutations**

| | G12C + V8A, IC50 (µM) | G12C + V9Y, IC50 (µM) | G12C + S17E, IC50 (µM) | G12C + A59T, IC50 (µM) | G12C + T58I, IC50 (µM) | G12C + D60P, IC50 (µM) | G12C + S65W, IC50 (µM) | G12C + R68S, IC50 (µM) | G12C + M72I, IC50 (µM) | G12C + D92R, IC50 (µM) | G12C + H95N, IC50 (µM) | G12C + Y96D, IC50 (µM) | G12C + Q99W IC50 (µM) | G12C + F156L IC50 (µM) |
|---|---|---|---|---|---|---|---|---|---|---|---|---|---|---|
| Example 6 | 0.002 | 0.002 | 0.005 | 0.011 | 0.007 | 0.008 | 0.006 | 0.034 | 0.003 | 0.005 | 0.005 | 0.007 | 0.007 | 0.008 |

## Claims

1. A compound of formula (I), 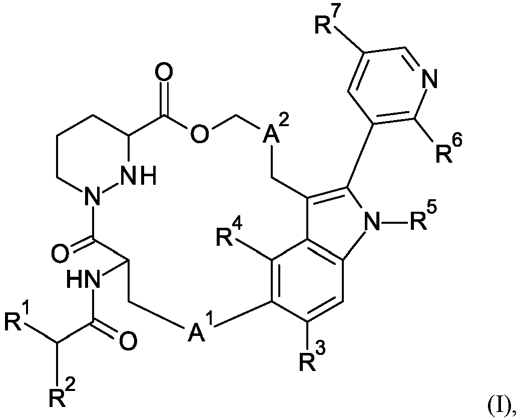 wherein
R¹ is 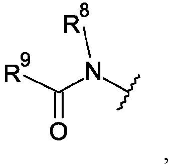 3-oxo-5,6,8,8a-tetrahydro-1H-imidazo[5,1-c][1,4]oxazinyl or (C₁₋₆alkyl)oxoimidazolidinyl;
wherein
R⁸ is C₁₋₆alkyl;
R⁹ is ((C₁₋₆alkyl)₂amino)azetidinyl, C₁₋₆alkylpiperazinyl, haloazetidinyl, haloC₁₋₆alkylamino, haloC₁₋₆alkylaminoazetidinyl, haloC₁₋₆alkylpiperazinyl, hydroxy(C₁₋₆alkyl)piperidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

2. A compound according to claim 1 of formula (Ia), 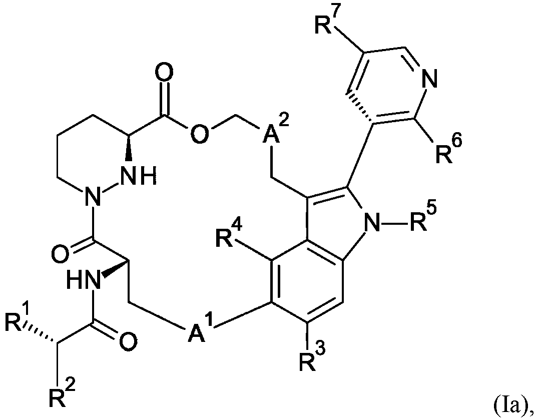 wherein
R¹ is 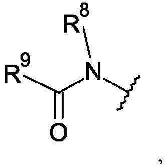 3-oxo-5,6,8,8a-tetrahydro-1H-imidazo[5,1-c][1,4]oxazinyl or (C₁₋₆alkyl)oxoimidazolidinyl;
wherein
R⁸ is C₁₋₆alkyl;
R⁹ is ((C₁₋₆alkyl)₂amino)azetidinyl, C₁₋₆alkylpiperazinyl, haloazetidinyl, haloC₁₋₆alkylamino, haloC₁₋₆alkylaminoazetidinyl, haloC₁₋₆alkylpiperazinyl, hydroxy(C₁₋₆alkyl)piperidinyl or morpholinyl;
R² is C₁₋₆alkyl;
R³ is H or halogen;
R⁴ is H or halogen;
R⁵ is C₁₋₆alkyl or haloC₁₋₆alkyl;
R⁶ is C₁₋₆alkoxyC₁₋₆alkyl;
R⁷ is morpholinyl, (haloC₁₋₆alkyl)piperazinyl or C₁₋₆alkylpiperazinyl;
A¹ is thiazolylene;
A² is C₁₋₆alkylene;
with the proviso that R³ and R⁴ are not H simultaneously;
or a pharmaceutically acceptable salt thereof.

3. A compound according to claim 1 or 2, wherein R¹ is 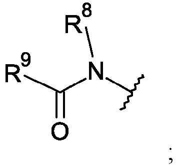 wherein R⁸ is C₁₋₆alkyl; R⁹ is C₁₋₆alkylpiperazinyl, haloC₁₋₆alkylpiperazinyl or morpholinyl.

4. A compound according to any one of claims 1-3, wherein R² is isopropyl.

5. A compound according to any one of claims 1-6, wherein R³ is H or fluoro.

6. A compound according to any one of claims 1-5, wherein R⁵ is haloC₁₋₆alkyl.

7. A compound according to any one of claims 1-6, wherein R⁷ is (haloC₁₋₆alkyl)piperazinyl.

8. A compound according to any one of claims 1-7, wherein A¹ is 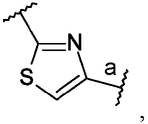 wherein bond "a" connects to indole ring.

9. A compound according to claim 1 or 2, wherein
R¹ is 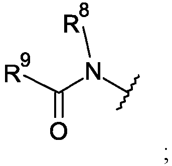 wherein R⁸ is C₁₋₆alkyl; R⁹ is C₁₋₆alkylpiperazinyl, haloC₁₋₆alkylpiperazinyl or morpholinyl;
R² is C₁₋₆alkyl;
R³ is halogen;
R⁴ is H;
R⁵ is haloC₁₋₆alkyl;
R⁶ is C₁₋₆alkoxyC₁₋₆alkyl;
R⁷ is (haloC₁₋₆alkyl)piperazinyl;
A¹ is 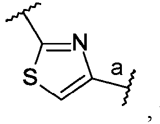 wherein bond "a" connects to indole ring;
A² is C₁₋₆alkylene;
or a pharmaceutically acceptable salt thereof.

10. A compound according to claim 9, wherein
R¹ is methyl-(4-methylpiperazine-1-carbonyl)amino, methyl-[4-(2,2,2-trifluoroethyl)piperazine-1-carbonyl]amino or methyl(morpholine-4-carbonyl)amino;
R² is isopropyl;
R³ is fluoro;
R⁴ is H;
R⁵ is 2,2,2-trifluoroethyl;
R⁶ is (1S)-1-methoxyethyl;
R⁷ is 4-(2,2,2-trifluoroethyl)piperazin-1-yl;
A¹ is 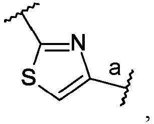 wherein bond "a" connects to indole ring;
A² is dimethylmethylene;
or a pharmaceutically acceptable salt thereof.

11. A compound according to claim 1 selected from:
3-(dimethylamino)-*N*-[(1*S*)-1-[[(7*S*,13*S*)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
3-(dimethylamino)-*N*-[(1*S*)-1-[[(7S,13S)-25-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
*N*-[(1*S*)-1-[[(7*S*,13*S*)-24-fluoro-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
3-(dimethylamino)-*N*-[(1*S*)-1-[[(7S,13S)-24-fluoro-(20M)-20-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,113}.0^{22,26}]octacosa-1(25),2,5(28),19,22(26),23-hexaen-7-yl]carbamoyl]-2-methyl-propyl]-*N*-methyl-azetidine-1-carboxamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-24-fluoro-(20M)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3,3-difluoro-*N*-methyl-azetidine-1-carboxamide;
(2*S*)-*N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-methyl-2-oxo-imidazolidin-1-yl)butanamide;
*N*-[(1*S*)-1-[[(7*S*,13*S*)-21-ethyl-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-21-ethyl-24-fluoro-(20*M*)-20-[2-[(1*S*)-1-methoxyethyl]-5-(4-methylpiperazin-1-yl)-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
*N*-[(1*S*)-1-[[(7*S*,13*S*)-24-fluoro-20-(20M)-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholine-4-carboxamide;
3-(dimethylamino)-*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidine-1-carboxamide;
(2*S*)-*N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-[methyl(2,2,2-trifluoroethylcarbamoyl)amino]butanamide;
*N*-[(1*S*)-1-[[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-3-(trifluoromethyl)azetidine-1-carboxamide;
(2*S*)-*N*-[(7*S*,13*S*)-21-ethyl-24-fluoro-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-oxo-5,6,8,8a-tetrahydro-1*H*-imidazo[5,1-c][1,4]oxazin-2-yl)butanamide;
(3*R*)-*N*-[(1*S*)-1-[[(7*S*,13*S*)-24-fluoro-(20*M*)-20-[2-[(1*S*)-1-methoxyethyl]-5-morpholino-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3-hydroxy-*N*,3-dimethyl-piperidine-1-carboxamide;
*N*-[(1*S*)-1-[[(7*S*,13*S*)-25-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-ylcarbamoyl]-2-methyl-propyl]-*N*-methyl-morpholine-4-carboxamide;
*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*,4-dimethyl-piperazine-1-carboxamide; and
*N*-[(1*S*)-1-[[(7*S*,13*S*)-24-fluoro-(20*M*)-20-[2-[(1*S*)-1-methoxyethyl]-5-[4-(2,2,2-trifluoroethyl)piperazin-1-yl]-3-pyridyl]-17,17-dimethyl-8,14-dioxo-21-(2,2,2-trifluoroethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-4-(2,2,2-trifluoroethyl)piperazine-1-carboxamide;
or a pharmaceutically acceptable salt thereof.

12. A process for the preparation of a compound according to any one of claims 1 to 11 comprising the following step:
a) coupling reaction between compound of formula (II), 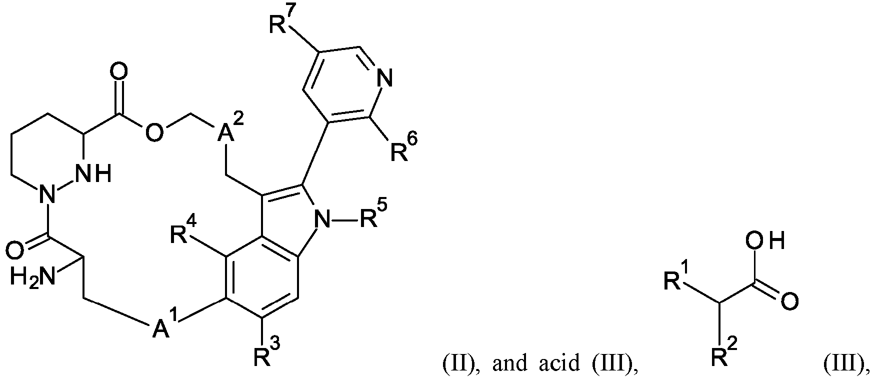 in the presence of a coupling reagent and a base to form the compound of formula (I);
wherein R¹, R², R³, R⁴, R⁵, R⁶, R⁷, A¹ and A² are defined as in any one of claims 1 to 10; the coupling reagent is T₃P, HATU, PyBOP or EDCI/HOBt; the base is TEA, DIEPA or DMAP.

13. A compound or pharmaceutically acceptable salt according to any one of claims 1 to 11 for use as therapeutically active substance.

14. A pharmaceutical composition comprising a compound in accordance with any one of claims 1 to 11 and a pharmaceutically acceptable excipient.

15. A compound or pharmaceutically acceptable salt according to any one of claims 1 to 11 for the treatment or prophylaxis of KRAS mutation driven cancers, wherein the cancer is selected from pancreatic adenocarcinoma, colorectal cancer and non-small cell lung cancer.

## Patentansprüche

1. Verbindung der Formel (I), 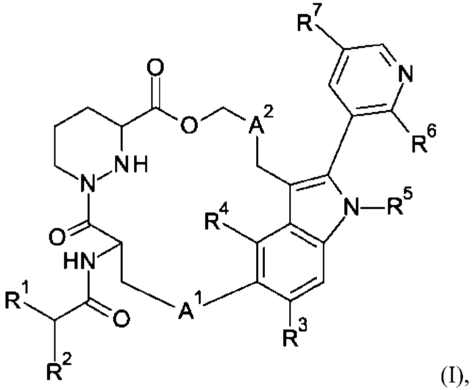 wobei
R¹ 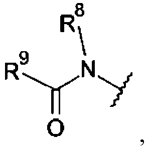 3-Oxo-5,6,8,8a-tetrahydro-1H-imidazo[5,1-c][1,4]oxazinyl oder (C₁₋₆-Alkyl)oxoimidazolidinyl ist;
wobei
R⁸ C₁₋₆-Alkyl ist;
R⁹ ((C₁₋₆-Alkyl)₂amino)azetidinyl, C₁₋₆-Alkylpiperazinyl, Halogenazetidinyl, Halogen-C₁₋₆-alkylamino, Halogen-C₁₋₆-alkylaminoazetidinyl, Halogen-C₁₋₆-alkylpiperazinyl, Hydroxy(C₁₋₆-alkyl)piperidinyl oder Morpholinyl ist;
R² C₁₋₆-Alkyl ist;
R³ H oder Halogen ist;
R⁴ H oder Halogen ist;
R⁵ C₁₋₆-Alkyl oder Halogen-C₁₋₆-alkyl ist;
R⁶ C₁₋₆-Alkoxy-C₁₋₆-alkyl ist;
R⁷ Morpholinyl, (Halogen-C₁₋₆-alkyl)piperazinyl oder C₁₋₆-Alkylpiperazinyl ist;
A¹ Thiazolylen ist;
A² C₁₋₆-Alkylen ist;
mit der Maßgabe, dass R³ und R⁴ nicht gleichzeitig H sind;
oder ein pharmazeutisch verträgliches Salz davon.

2. Verbindung nach Anspruch 1 der Formel (Ia), 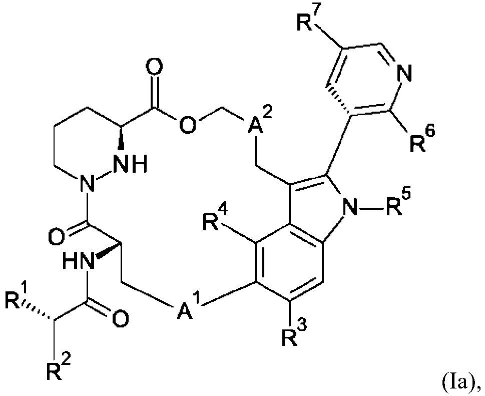 wobei
R¹ 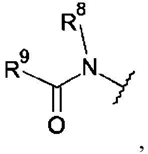 3-Oxo-5,6,8,8a-tetrahydro-1H-imidazo[5,1-c][1,4]oxazinyl oder (C₁₋₆-Alkyl)oxoimidazolidinyl ist;
wobei
R⁸ C₁₋₆-Alkyl ist;
R⁹ ((C₁₋₆-Alkyl)zamino)azetidinyl, C₁₋₆-Alkylpiperazinyl, Halogenazetidinyl, Halogen-C₁₋₆-alkylamino, Halogen-C₁₋₆-alkylaminoazetidinyl, Halogen-C₁₋₆-alkylpiperazinyl, Hydroxy(C₁₋₆-alkyl)piperidinyl oder Morpholinyl ist;
R² C₁₋₆-Alkyl ist;
R³ H oder Halogen ist;
R⁴ H oder Halogen ist;
R⁵ C₁₋₆-Alkyl oder Halogen-C₁₋₆-alkyl ist;
R⁶ C₁₋₆-Alkoxy-C₁₋₆-alkyl ist;
R⁷ Morpholinyl, (Halogen-C₁₋₆-alkyl)piperazinyl oder C₁₋₆-Alkylpiperazinyl ist;
A¹ Thiazolylen ist;
A² C₁₋₆-Alkylen ist;
mit der Maßgabe, dass R³ und R⁴ nicht gleichzeitig H sind;
oder ein pharmazeutisch verträgliches Salz davon.

3. Verbindung nach Anspruch 1 oder 2, wobei R¹ 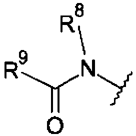 ist; wobei R⁸ C₁₋₆-Alkyl ist; R⁹ C₁₋₆-Alkylpiperazinyl, Halogen-C₁₋₆-alkylpiperazinyl oder Morpholinyl ist.

4. Verbindung nach einem der Ansprüche 1-3, wobei R² Isopropyl ist.

5. Verbindung nach einem der Ansprüche 1-6, wobei R³ H oder Fluor ist.

6. Verbindung nach einem der Ansprüche 1-5, wobei R⁵ Halogen-C₁₋₆-alkyl ist.

7. Verbindung nach einem der Ansprüche 1-6, wobei R⁷ (Halogen-C₁₋₆-alkyl)piperazinyl ist.

8. Verbindung nach einem der Ansprüche 1-7, wobei A¹ 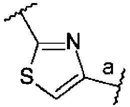 ist, wobei die Bindung "a" den Indolring bindet.

9. Verbindung nach Anspruch 1 oder 2, wobei
R¹ 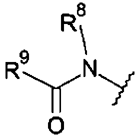 ist; wobei R⁸ C₁₋₆-Alkyl ist; R⁹ C₁₋₆-Alkylpiperazinyl, Halogen-C₁₋₆-alkylpiperazinyl oder Morpholinyl ist;
R² C₁₋₆-Alkyl ist;
R³ Halogen ist;
R⁴ H ist;
R⁵ Halogen-C₁₋₆-alkyl ist;
R⁶ C₁₋₆-Alkoxy-C₁₋₆-alkyl ist;
R⁷ (Halogen-C₁₋₆-alkyl)piperazinyl ist;
A¹ 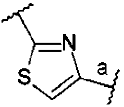 ist, wobei die Bindung "a" den Indolring bindet;
A² C₁₋₆-Alkylen ist;
oder ein pharmazeutisch verträgliches Salz davon.

10. Verbindung nach Anspruch 9, wobei
R¹ Methyl-(4-methylpiperazin-1-carbonyl)amino, Methyl-[4-(2,2,2-trifluorethyl)piperazin-1-carbonyl]amino oder Methyl(morpholin-4-carbonyl)amino ist;
R² Isopropyl ist;
R³ Fluor ist;
R⁴ H ist;
R⁵ 2,2,2-Trifluorethyl ist;
R⁶ (1S)-1-Methoxyethyl ist;
R⁷ 4-(2,2,2-Trifluorethyl)piperazin-1-yl ist;
A¹ 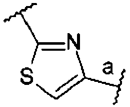 ist, wobei die Bindung "a" den Indolring bindet;
A² Dimethylmethylen ist;
oder ein pharmazeutisch verträgliches Salz davon.

11. Verbindung nach Anspruch 1, ausgewählt aus:
3-(Dimethylamino)-*N*-[(1*S*)-1-[[(7*S*,13*S*)-24-fluor-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidin-1-carboxamid;
3-(Dimethylamino)-*N*-[(1*S*)-1-[[(7*S*,13*S*)-25-fluor-(20*M*)-20-[2-[(1*S*)-1-methoxyethyl]-5-(4-methylpiperazin-1-yl)-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidin-1-carboxamid;
*N*-[(1*S*)-*1*-[[(7*S,*13*S*)-24-Fluor-(20*M*)-20-[2-[(1*S*)*-*1*-*methoxyethyl]-5-(4-methylpiperazin-1-yl)-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
3-(Dimethylamino)-*N*-[(1*S*)-1-[[(7*S*,13*S*)-24-fluor-(20*M*-20-[2-[(1*S*)-1-methoxyethyl]-5-morpholino-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidin-1-carboxamid;
*N-*[(1*S*)-1-[[(7*S,*13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,1}.1^{9,11}.0^{22,26}]octacosa-1(25),2,5(28),19,22(26),23-hexaen-7-yl]carbamoyl]-2-methyl-propyl]-*N*-methyl-morpholin-4-carboxamid;
*N*-[(1*S*)-1-[[(7*S,*13*S*)-24-Fluor-(20*M*)-20-[2-[(1*S*)-1-methoxyethyl]-5-[4-(2,2,2-trifluorethyl)piperazin-1-yl]-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
*N-*[(1*S*)-1-[[(7*S,*13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3,3-difluor-N-methyl-azetidin-1-carboxamid;
(2*S*)-*N*-[(7*S*,13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-methyl-2-oxo-imidazolidin-1-yl)butanamid;
*N*-[(1*S*)-1-[[(7*S,*13*S*)-21-Ethyl-24-fluor-(20*M*)-20-[2-[(1*S*)-1-methoxyethyl]-5*-*[4-(2,2,2-trifluorethyl)piperazin-1-yl]-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
*N-*[(1*S*)-1-[[(7*S,*13*S*)-21-Ethyl-24-fluor-(20*M*)-20-[2-[(1*S*)-1-methoxyethyl]-5-(4-methylpiperazin-1-yl)-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
*N-*[(1*S*)-1-[[(7*S,*13*S*)-24-Fluor-20-(20*M*)-[2-[(1*S*)-1*-*methoxyethyl]-5-morpholino-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
3-(Dimethylamino)-N-[(1*S*)-1-[[(7S*,*13*S*)-24-fluor-(20*M*)-20-[2-[(1*S*)-1-methoxyethyl]-5-[4-(2,2,2-trifluorethyl)piperazin-1-yl]-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-azetidin-1-carboxamid;
(2*S*)-*N*-[(7*S*,13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1,^{9,13}.0^{22,26}]octacosa-1(25),2,5(28),19,22(26),23-hexaen-7-yl]-3-methyl-2-[methyl(2,2,2-trifluorethylcarbamoyl)amino]butanamid;
*N*-[(1*S*)-1-[[(7*S,*13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-3-(trifluormethyl)azetidin-1-carboxamid;
(2*S*)-*N*-[(7*S*,13*S*)-21-Ethyl-24-fluor-(20*M*)-20-[2-[(1*S*)-1-methoxyethyl]-5-morpholino-3-pyridyl]-17,17-dimethyl-8,14-dioxo-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]-3-methyl-2-(3-oxo-5,6,8,8a-tetrahydro-1*H*-imidazo[5,1-c][1,4]oxazin-2-yl)butanamid;
(3*R*)-N-[(1*S*)-1-[[(7*S,*13*S*)-24-Fluor-(20*M*)-20-[2-[(1*S*)-1-methoxyethyl]-5-morpholino-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-3-hydroxy-*N*,3-dimethyl-piperidin-1-carboxamid;
*N*-[(1*S*)-1-[[(7*S,*13*S*)-25-Fluor-(20*M*)-20-[2-[(1*S*)-1-methoxyethyl]-5-[4-(2,2,2-trifluorethyl)piperazin-1-yl]-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*-methyl-morpholin-4-carboxamid;
*N*-[(1*S*)-1-[[(7*S,*13*S*)-24-Fluor-(20*M*)-20-[2-[(1*S*)-1-methoxyethyl]-5-[4-(2,2,2-trifluorethyl)piperazin-1-yl]-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-*N*,4-dimethyl-piperazin-1-carboxamid; und
*N*-[(1*S*)-1-[[(7*S,*13*S*)-24-Fluor-(20*M*)-20-[2-[(1*S*)-1-methoxyethyl]-5-[4-(2,2,2-trifluorethyl)piperazin-1-yl]-3-pyridyl]-17,17-dimethyl-8,14-dioxo-21-(2,2,2-trifluorethyl)-15-oxa-4-thia-9,21,27,28-tetrazapentacyclo[17.5.2.1^{2,5}.1^{9,13}.0^{22,26}]octacosa-1(25),2,5(28),19,22(26),23-hexaen-7-yl]carbamoyl]-2-methyl-propyl]-N-methyl-4-(2,2,2-trifluorethyl)piperazin-1-carboxamid;
oder ein pharmazeutisch verträgliches Salz davon.

12. Verfahren zur Herstellung einer Verbindung nach einem der Ansprüche 1 bis 11, umfassend den folgenden Schritt:
a) Kupplungsreaktion zwischen Verbindung der Formel (II), 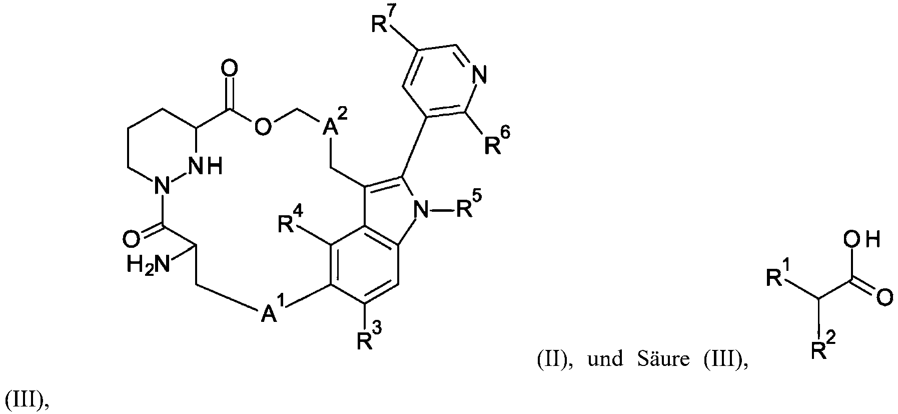 in Gegenwart eines Kupplungsreagens und einer Base, um die Verbindung der Formel (I) zu bilden;
wobei R¹, R², R³, R⁴, R⁵, R⁶, R⁷, A¹ und A² wie in einem der Ansprüche 1 bis 10 definiert sind; das Kupplungsreagens T₃P, HATU, PyBOP oder EDCI/HOBt ist; die Base TEA, DIEPA oder DMAP ist.

13. Verbindung oder pharmazeutisch verträgliches Salz nach einem der Ansprüche 1 bis 11 zur Verwendung als therapeutisch aktive Substanz.

14. Pharmazeutische Zusammensetzung, umfassend eine Verbindung nach einem der Ansprüche 1 bis 11 und einen pharmazeutisch verträglichen Hilfsstoff.

15. Verbindung oder pharmazeutisch verträgliches Salz nach einem der Ansprüche 1 bis 11 zur Behandlung oder Prophylaxe von durch KRAS-Mutation bedingten Krebserkrankungen, wobei die Krebserkrankung ausgewählt ist aus Bauchspeicheldrüsenadenokarzinom, Kolorektalkrebs und nicht-kleinzelligem Lungenkrebs.

## Revendications

1. Composé de formule (I), 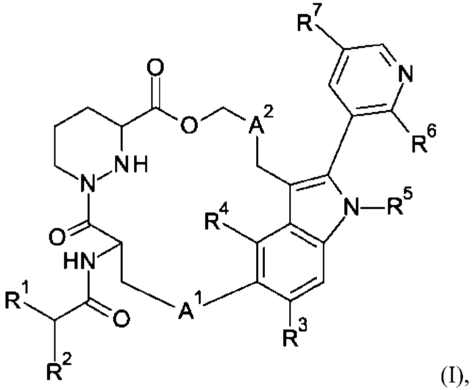 dans lequel
R¹ représente 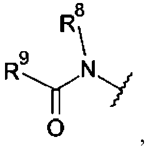 un 3-oxo-5,6,8,8a-tétrahydro-1H-imidazo[5,1-c][1,4]oxazinyle ou un (alkyle en C₁₋₆)oxoimidazolidinyle ;
dans lequel
R⁸ représente un alkyle en C₁₋₆ ;
R⁹ représente un ((alkyle en C₁₋₆)₂amino)azétidinyle, un alkyle en C₁₋₆-pipérazinyle, un halogénoazétidinyle, un halogénoalkyle en C₁₋₆-amino, un halogénoalkyle en C₁₋₆-aminoazétidinyle, un halogénoalkyle en C₁₋₆-pipérazinyle, un hydroxy(alkyle en C₁₋₆)pipéridinyle ou un morpholinyle ;
R² représente un alkyle en C₁₋₆ ;
R³ représente H ou un halogène ;
R⁴ représente H ou un halogène ;
R⁵ représente un alkyle en C₁₋₆ ou un halogénoalkyle en C₁₋₆ ;
R⁶ représente un alcoxy en C₁₋₆-alkyle en C₁₋₆ ;
R⁷ représente un morpholinyle, un (halogénoalkyle en C₁₋₆)pipérazinyle ou un alkyle en C₁₋₆-pipérazinyle ;
A¹ représente un thiazolylène ;
A² représente un alkylène en C₁₋₆ ;
à condition que R³ et R⁴ ne représentent pas H simultanément ;
ou un sel pharmaceutiquement acceptable de celui-ci.

2. Composé selon la revendication 1 de formule (Ia), 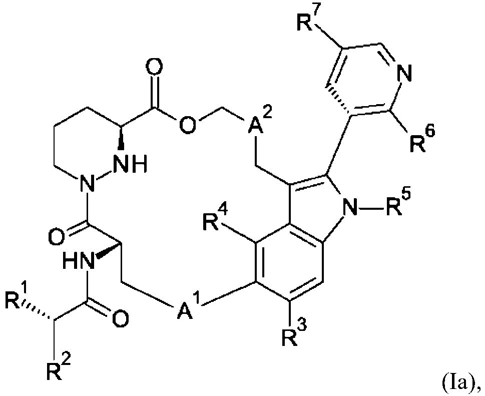 dans lequel
R¹ représente 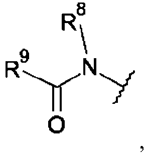 un 3-oxo-5,6,8,8a-tétrahydro-1H-imidazo[5,1-c][1,4]oxazinyle ou un (alkyle en C₁₋₆)oxoimidazolidinyle ;
dans lequel
R⁸ représente un alkyle en C₁₋₆ ;
R⁹ représente un ((alkyle en C₁₋₆)₂amino)azétidinyle, un alkyle en C₁₋₆-pipérazinyle, un halogénoazétidinyle, un halogénoalkyle en C₁₋₆-amino, un halogénoalkyle en C₁₋₆-aminoazétidinyle, un halogénoalkyle en C₁₋₆-pipérazinyle, un hydroxy(alkyle en C₁₋₆)pipéridinyle ou un morpholinyle ;
R² représente un alkyle en C₁₋₆ ;
R³ représente H ou un halogène ;
R⁴ représente H ou un halogène ;
R⁵ représente un alkyle en C₁₋₆ ou un halogénoalkyle en C₁₋₆ ;
R⁶ représente un alcoxy en C₁₋₆-alkyle en C₁₋₆ ;
R⁷ représente un morpholinyle, un (halogénoalkyle en C₁₋₆)pipérazinyle ou un alkyle en C₁₋₆-pipérazinyle ;
A¹ représente un thiazolylène ;
A² représente un alkylène en C₁₋₆ ;
à condition que R³ et R⁴ ne représentent pas H simultanément ;
ou un sel pharmaceutiquement acceptable de celui-ci.

3. Composé selon la revendication 1 ou 2, dans lequel R¹ représente 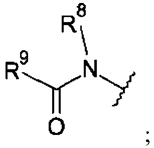 dans lequel R⁸ représente un alkyle en C₁₋₆ ; R⁹ représente un alkyle en C₁₋₆-pipérazinyle, un halogénoalkyle en C₁₋₆-pipérazinyle ou un morpholinyle.

4. Composé selon l'une quelconque des revendications 1 à 3, dans lequel R² représente un isopropyle.

5. Composé selon l'une quelconque des revendications 1 à 6, dans lequel R³ représente H ou un fluor.

6. Composé selon l'une quelconque des revendications 1 à 5, dans lequel R⁵ représente un halogénoalkyle en C₁₋₆.

7. Composé selon l'une quelconque des revendications 1 à 6, dans lequel R⁷ représente un (halogénoalkyle en C₁₋₆)pipérazinyle.

8. Composé selon l'une quelconque des revendications 1 à 7, dans lequel A¹ représente 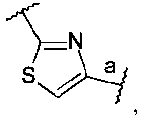 dans lequel la liaison « a » est reliée à un cycle indole.

9. Composé selon la revendication 1 ou 2, dans lequel
R¹ représente 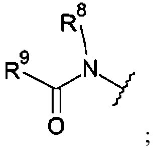 dans lequel R⁸ représente un alkyle en C₁₋₆; R⁹ représente un alkyle en C₁₋₆-pipérazinyle, un halogénoalkyle en C₁₋₆-pipérazinyle ou un morpholinyle ;
R² représente un alkyle en C₁₋₆ ;
R³ représente un halogène ;
R⁴ représente H ;
R⁵ représente un halogénoalkyle en C₁₋₆ ;
R⁶ représente un alcoxy en C₁₋₆-alkyle en C₁₋₆ ;
R⁷ représente un (halogénoalkyle en C₁₋₆)pipérazinyle ;
A¹ représente 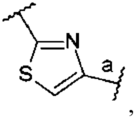 dans lequel la liaison « a » est reliée au cycle indole ;
A² représente un alkylène en C₁₋₆ ;
ou un sel pharmaceutiquement acceptable de celui-ci.

10. Composé selon la revendication 9, dans lequel
R¹ représente un méthyl-(4-méthylpipérazine-1-carbonyl)amino, un méthyl-[4-(2,2,2-trifluoroéthyl)pipérazine-1-carbonyl]amino ou un méthyl(morpholine-4-carbonyl)amino ;
R² représente un isopropyle ;
R³ représente un fluor ;
R⁴ représente H ;
R⁵ représente un 2,2,2-trifluoroéthyle ;
R⁶ représente un (1S)-1-méthoxyéthyle ;
R⁷ représente un 4-(2,2,2-trifluoroéthyl)pipérazin-1-yle ;
A¹ représente 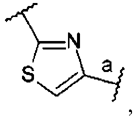 dans lequel la liaison « a » est reliée au cycle indole ;
A² représente un diméthylméthylène ;
ou un sel pharmaceutiquement acceptable de celui-ci.

11. Composé selon la revendication 1 choisi parmi :
le 3-(diméthylamino)-*N*-[(1*S*)-1-[[(7*S*,13*S*)-24-fluoro-(20*M*)-20-[2-[(1*S*)-1-méthoxyéthyl]-5-(4-méthylpipérazin-1-yl)-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-azétidine-1-carboxamide ;
le 3-(diméthylamino)-*N*-[(1*S*)-1-[[(7*S*,13*S*)-25-fluoro-(20*M*)-20-[2-[(1*S*)-1-méthoxyéthyl]-5-(4-méthylpipérazin-1-yl)-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-azétidine-1-carboxamide ;
le *N*-[(1*S*)-1-[[(7*S*,13*S*)-24-fluoro-(20*M*)-20-[2-[(1*S*)-1-méthoxyéthyl]-5-(4-méthylpipérazin-1-yl)-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le 3-(diméthylamino)-*N*-[(1*S*)-1-[[(7*S*,13*S*)-24-fluoro-(20*M*)-20-[2-[(1*S*)-1-méthoxyéthyl]-5-morpholino-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-azétidine-1-carboxamide ;
le *N*-[(1*S*)-1-[[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le *N*-[(1*S*)-1-[[(7*S,*13*S*)-24-fluoro-(20*M*)-20-[2-[(1*S*)-1-méthoxyéthyl]-5-[4-(2,2,2-trifluoroéthyl)pipérazin-1-yl]-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le *N*-[(1*S*)-1-[[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-3,3-difluoro-*N*-méthyl-azétidine-1-carboxamide ;
le (2*S*)-*N*-[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]-3-méthyl-2-(3-méthyl-2-oxo-imidazolidin-1-yl)butanamide ;
le *N*-[(1*S*)-1-[[(7*S,*13*S*)-21-éthyl-24-fluoro-(20*M*)-20-[2-[(1*S*)-1-méthoxyéthyl]-5-[4-(2,2,2-trifluoroéthyl)pipérazin-1-yl]-3-pyridyl]-17,17-diméthyl-8,14-dioxo-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le *N*-[(1*S*)-1-[[(7*S*,13*S*)-21-éthyl-24-fluoro-(20*M*)-20-[2-[(1*S*)-1-méthoxyéthyl]-5-(4-méthylpipérazin-1-yl)-3-pyridyl]-17,17-diméthyl-8,14-dioxo-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le *N*-[(1*S*)-1-[[(7*S*,13*S*)-24-fluoro-20-(20*M*)-[2-[(1*S*)-1-méthoxyéthyl]-5-morpholino-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le 3-(diméthylamino)-*N*-[(1*S*)-1-[[(7*S*,13*S*)-24-fluoro-(20*M*)-20-[2-[(1*S*)-1-méthoxyéthyl]-5-[4-(2,2,2-trifluoroéthyl)pipérazin-1-yl]-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]carbamoyl]-2-méthyl-propyl]-*N*-méthyl-azétidine-1-carboxamide ;
le (2*S*)-*N*-[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,5}.1,^{9,13}.0^{22,26}]octacosa-1(25),2,5(28),19,22(26),23-hexaén-7-yl]-3-méthyl-2-[méthyl(2,2,2-trifluoroéthylcarbamoyl)amino]butanamide ;
le *N*-[(1*S*)-1-[[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]carbamoyl]-2-méthyl-propyl]-*N*-méthyl-3-(trifluorométhyl)azétidine-1-carboxamide ;
le (2*S*)-*N*-[(7*S*,13*S*)-21-éthyl-24-fluoro-(20*M*)-20-[2-[(1*S*)-1-méthoxyéthyl]-5-morpholino-3-pyridyl]-17,17-diméthyl-8,14-dioxo-15-oxa-4-thia-9,21,27,28-tétrazapentacyclo[17.5.2.1^{2,1}.1^{9,13}.0^{22,26}]octacosa-1(25),2,5(28),19,22(26),23-hexaén-7-yl]-3-méthyl-2-(3-oxo-5,6,8,8a-tétrahydro-1*H*-imidazo[5,1-c][1,4]oxazin-2-yl)butanamide ;
le (3*R*)-*N*-[(1*S*)-1-[[(7*S*,13*S*)-24-fluoro-(20*M*)-20-[2-[(1*S*)-1-méthoxyéthyl]-5-morpholino-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-3-hydroxy-*N*,3-diméthyl-pipéridine-1-carboxamide ;
le *N*-[(1*S*)-1-[[(7*S,*13*S*)-25-fluoro-(20*M*-20-[2-[(1*S*)-1-méthoxyéthyl]-5-[4-(2,2,2-trifluoroéthyl)pipérazin-1-yl]-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-morpholine-4-carboxamide ;
le *N*-[(1*S*)-1-[[(7*S,*13*S*)-24-fluoro-(20*M*)-20-[2-[(1*S*)-1-méthoxyéthyl]-5*-*[4-(2,2,2-trifluoroéthyl)pipérazin-1-yl]-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.o^{22,26}]octacosa-1(25),2,5(28),19,22(26),23-hexaén-7-yl]carbamoyl]-2-méthyl-propyl]-*N*,4-diméthyl-pipérazine-1-carboxamide ; et
le *N*-[(1*S*)-1-[[(7*S*,13*S*)-24-fluoro-(20*M*)-20-[2-[(1*S*)-1-méthoxyéthyl]-5-[4-(2,2,2-trifluoroéthyl)pipérazin-1-yl]-3-pyridyl]-17,17-diméthyl-8,14-dioxo-21-(2,2,2-trifluoroéthyl)-15-oxa-4-thia-9,21,27,28-tétrazapentacyclo[17.5.2.1^{2,5}.1^{9,13}.0^{22,26}]octacosa-1(25),2,5(28),19,22(26),23-hexaén-7-yl]carbamoyl]-2-méthyl-propyl]-*N*-méthyl-4-(2,2,2-trifluoroéthyl)pipérazine-1-carboxamide ;
ou un sel pharmaceutiquement acceptable de ceux-ci.

12. Procédé de préparation d'un composé selon l'une quelconque des revendications 1 à 11 comprenant l'étape suivante :
a) réaction de couplage entre le composé de formule (II), 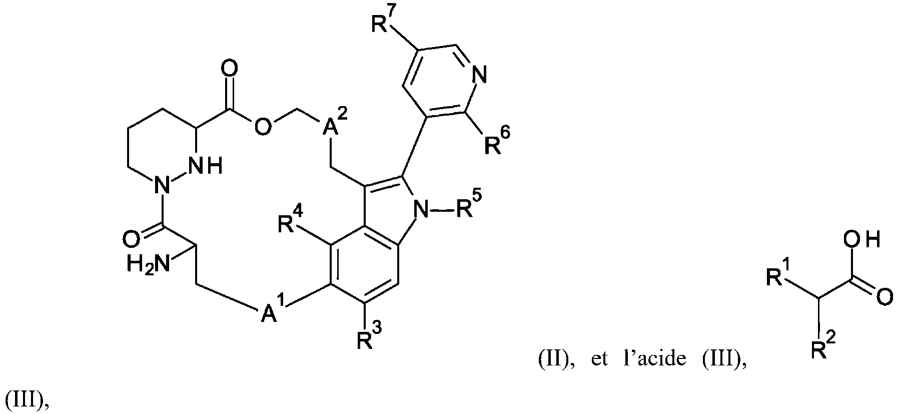 en présence d'un réactif de couplage et d'une base pour former le composé de formule (I) ;
dans lequel R¹, R², R³, R⁴, R⁵, R⁶, R⁷, A¹ et A² sont tels que définis dans l'une quelconque des revendications 1 à 10 ; le réactif de couplage est T₃P, HATU, PyBOP ou EDCI/HOBt ; la base est TEA, DIEPA ou DMAP.

13. Composé ou sel pharmaceutiquement acceptable selon l'une quelconque des revendications 1 à 11 pour une utilisation comme substance thérapeutiquement active.

14. Composition pharmaceutique comprenant un composé selon l'une quelconque des revendications 1 à 11 et un excipient pharmaceutiquement acceptable.

15. Composé ou sel pharmaceutiquement acceptable selon l'une quelconque des revendications 1 à 11 pour le traitement ou la prophylaxie de cancers entraînés par la mutation KRAS, dans lequel le cancer est choisi parmi l'adénocarcinome pancréatique, le cancer colorectal et le cancer du poumon non à petites cellules.
